# EUROPEAN PATENT SPECIFICATION

(11) **EP 4 157 186 B1**
(45) Date of publication and mention of the grant of the patent: **28.10.2026**
(21) Application number: 21812021.0
(22) Date of filing: 25.05.2021
(51) Int. Cl.: A61G 7/07, A61B 6/04, A61G 13/12, A61F 5/055

(54) **HEAD STABILIZATION SYSTEMS**
KOPFSTABILISIERUNGSSYSTEME
SYSTÈMES DE STABILISATION DE LA TÊTE

(30) Priority: 26.05.2020 US 202063029781 P; 22.02.2021 US 202163151926 P
(43) Date of publication of application: 05.04.2023
(73) Proprietor: Deinde Medical Corp., Miramar, FL 33025 (US)
(72) Inventor: FARNAN, Robert, C., Fort Lauderdale, FL 33308 (US); ARP, Scott, Miami, FL 33165 (US); TOMLIN, Damian, Coral Springs, FL 33065 (US); GAMEZ, Victor, Fort Lauderdale, FL 33305 (US); KRATSCH, Peter, Leander, TX 78641 (US)
(74) Representative: Reddie & Grose LLP
(86) International application number: PCT/US2021/034009
(87) International publication number: WO 2021/242731

(56) References cited:
- WO-A1-2012/095542
- US-A- 5 511 854
- US-A- 5 569 175
- US-A- 5 609 566
- US-A- 6 003 174
- US-A1- 2002 112 287
- US-A1- 2007 032 795
- US-A1- 2010 242 177
- US-A1- 2012 124 747
- US-A1- 2014 076 331
- US-A1- 2015 202 073
- US-B1- 6 260 220

## Description

This application is related to and claims the priority of U.S. Provisional Patent Application Serial Nos. 63/029,781 filed May 26, 2020 and 63/151,926 filed February 22, 2021.

### Technical Field

The present disclosure generally relates to patient positioning and stabilization systems and, more particularly, to head positioning and stabilization systems, such as for minimally invasive neurointerventional procedures, and related methods.

### Background

The present disclosure contemplates that some medical procedures, such as some surgical procedures and some imaging procedures, require precise positioning and/or stabilization of certain portions of a patient's anatomy. For example, some minimally invasive (e.g., catheter or endoscopically based) neurointerventional procedures may involve precise imaging of a patient's cerebral anatomy using several different imaging techniques. Multiple pieces of imaging equipment may be used (e.g., computerized tomography scan (CT), fluoroscope) that allow images to be merged for enhanced viewing of the target treatment location. To obtain the enhanced images and accurately merge and segment them, the patient's head is stabilized as the images are collected. The present disclosure contemplates that supportive materials, such as foam pads, towels, and drapes, may be used to stabilize the patient's head during imaging. But these methods may allow sufficient movement of the patient's head to reduce image quality or require repeated imaging. Also, the present disclosure contemplates that some head fixation devices normally used for neurosurgical procedures are capable of securely positioning a patient's head; however, these devices are typically complex, include materials that may interfere with imaging, and often involve potentially traumatic components (e.g., skull pins). Exemplary head stabilization systems according to the state of the art are disclosed in US 5 569 175 A, US 2015/202073 A1, US 2007/032795 A1 and US 2012/124747 A1.

Accordingly, and in spite of the various advances already made in this field, there is a need for further improvements related to patient head positioning and stabilization devices and, more particularly, to patient head stabilization devices for minimally invasive neurointerventional procedures, and related methods.

### Summary

The presently claimed invention is set out in independent claim 1. Preferred embodiments of the presently claimed invention are set out in the dependent claims. Any subject matter contained herein that does not fall within the scope of the appended claims is considered as being useful for understanding the invention.

Generally, a head stabilization system is provided and includes a head harness configured to engage a head of a patient and a head restraint mechanism configured to be operatively disposed on a patient support device, the head harness being releasably attachable to and at least partially supported by the head restraint mechanism. The head restraint mechanism is configured to stabilize the head harness relative to the patient support device and is configured to selectively allow lateral flexion of a neck of the patient, lateral rotation of the head of the patient, and extension and flexion of the neck of the patient. The head restraint mechanism may be configured to selectively provide about 60 degrees of lateral flexion of the neck of the patient, left and right, for a total of about 120 degrees of flexion of the neck of the patient. The head restraint mechanism may be configured to selectively provide about 120 degrees of lateral rotation of the head of the patient, left and right, for a total of about 240 degrees of rotation of the head of the patient. The head restraint mechanism may be configured to selectively provide about 50 degrees of neck extension and about 50 degrees of neck flexion, for a total of about 100 degrees of extension and flexion of the neck of the patient.

The head stabilization system may be used with multiple pieces of imaging equipment (e.g., computerized tomography scan (CT), magnetic resonance imaging (MRI), fluoroscope). The head harness, the head restraint mechanism, and the other components of the head stabilization system may be constructed substantially from materials that generally do not interfere with medical imaging modalities. The head harness and the head restraint mechanism may be constructed substantially from non-metallic materials. In some embodiments, the head harness and the head restraint mechanism may be configured to stabilize the head of the patient with the patient in a generally supine position.

The head harness of the head stabilization system may be configured to securely engage the head of the patient in a non-traumatic, non-penetrating manner. The head harness may be configured to engage a chin/jaw region, a forehead region, and a rear region of the head of the patient. The head harness may comprise a halo portion configured to engage the forehead region. The halo portion may comprise a right halo strap configured to extend front-to-back along the head of the patient, a left halo strap configured to extend front-to back along the head of the patient, and a halo adjustment mechanism coupled to the right halo strap and the left halo strap. The halo adjustment mechanism may be configured to allow adjustment of the halo portion to fit the head of the patient. The halo adjustment mechanism may further comprise a right halo hinge coupled to the right halo strap and a left halo hinge coupled to the left halo strap. The head harness may comprise a chin strap portion configured to engage the chin/jaw region. The head harness may further comprise a right halo stabilizer connecting the chin strap portion to the right halo strap and a left halo stabilizer connecting the chin strap portion to the left halo strap. The right halo stabilizer may comprise a right halo stabilizer halo strap portion connected to the right halo strap, and a right halo stabilizer chin strap portion connected to the chin strap. The right halo stabilizer halo strap portion may be releasably secured to the right halo stabilizer chin strap portion by a releasable right halo strap catch operable by a right halo strap actuator. The right halo strap actuator may comprise a right halo strap release button that, when pressed, releases the right halo stabilizer chin strap portion from the right halo stabilizer halo strap portion. The left halo stabilizer may comprise a left halo stabilizer halo strap portion connected to the left halo strap and a left halo stabilizer chin strap portion connected to the chin strap. The left halo stabilizer halo strap portion may be releasably secured to the left halo stabilizer chin strap portion by a releasable left halo strap catch operable by a left halo strap actuator. The left halo strap actuator may comprise a left halo strap release button that, when pressed, releases the left halo stabilizer chin strap portion from the left halo stabilizer halo strap portion.

The head harness may comprise a rear support portion configured to engage the rear region of a patient's head. The rear support portion may comprise a generally annular ring configured to be positioned centrally on the rear region of a patient's head. The rear support portion may be connected to the right halo strap and the left halo strap.

In alternative or additional aspects, the head harness may include one or more radiopaque markings. The radiopaque markings may facilitate alignment of imaging equipment, for example.

The head harness may comprise a head restraint portion connecting the rear support portion and the halo adjustment mechanism, the head restraint portion being configured to extend centrally front-to-back over the head of the patient. The head restraint portion may comprise a head restraint element disposed on a head restraint strap portion. The head restraint element may comprise a connecting element configured to releasably couple the head harness to the head restraint mechanism. The connecting element may comprise a harness connector housing. The harness connector housing may comprise a cavity and a slot configured to releasably engage the head restraint mechanism. The head restraint mechanism may comprise a harness connector assembly configured to releasably engage the head restraint element. The harness connector assembly may comprise a harness connector stop disposed on a harness connector shaft. The harness connector stop may be configured to be releasably received within the cavity of the harness connector housing. The harness connector shaft may be configured to be releasably received within the slot of the harness connector housing.

In some embodiments, the harness connector stop may comprise a generally spherical ball and the slot of the harness connector housing may comprise a generally circular opening. Positioning the harness connector stop within the cavity may comprise seating the generally spherical ball against the generally circular opening.

The connecting element may comprise a latch operative to selectively secure the connector stop in the cavity and the latch may be releasable by an actuator. The harness connector assembly and the connecting element may be selectively engageable at a plurality of discrete relative angular positions. The harness connector assembly may comprise a harness connector assembly toothed surface and the connecting element may include a connecting element toothed surface. The harness connector assembly toothed surface may be selectively engageable with the connecting element toothed surface at a plurality of discrete relative angular positions. The head restraint mechanism may comprise a generally vertically upstanding arcuate tilt guide and the head harness may be selectively repositionable generally vertically relative to the tilt guide. Repositioning the head harness generally vertically relative to the tilt guide may be operative to provide extension and flexion of the neck of the patient. The tilt guide may be disposed on a swivel arm. The swivel arm may be pivotably connected relative to the patient support device such that pivoting the swivel arm about a swivel axis may provide for lateral flexion of a neck of the patient. The swivel axis may be substantially perpendicular to an upper surface of the patient support device. The swivel arm may comprise a swivel lock assembly configured to selectively secure the swivel arm in an angular position. The tilt guide may be selectively repositionable longitudinally along the swivel arm.

The head harness may be releasably attachable to the head restraint mechanism by the connection of a connecting element of the head harness with a harness connector assembly of the head restraint mechanism. The harness connector assembly may be selectively slidably disposed in a generally vertically oriented tilt guide slot of the tilt guide. The harness connector assembly may comprise a harness connector knob operatively arranged to selectively secure the harness connector assembly in the tilt guide slot. The harness connector knob may be operative to secure the relative angular position of the head harness relative to the head restraint mechanism.

The head restraint mechanism may further comprise a locking pin and the tilt guide may comprise a plurality of laterally extending tilt guide through holes. The harness connector assembly may comprise a laterally extending harness connector assembly through hole. The pin may be configured to secure the harness connector assembly at a position on the tilt guide by extending the pin through one of the plurality of tilt guide through holes and the harness connector assembly through hole.

The head stabilization system may further comprise a shoulder restraint configured to engage a shoulder of the patient. The shoulder restraint may comprise a first shoulder restraint and a second shoulder restraint wherein the first shoulder restraint is configured to engage the right shoulder of a patient and the second shoulder restraint is configured to engage the left shoulder of a patient. The shoulder restraint may be selectively movable at least one of laterally and longitudinally relative to the head restraint mechanism. The shoulder restraint portion may be selectively movable laterally and longitudinally relative to the head restraint mechanism. The shoulder restraint may comprise a shoulder restraint connection element operatively interposing the shoulder restraint portion and the patient support device. The shoulder restraint portion may comprise a base and the base may be selectively slidably coupled to the shoulder restraint connection element. The shoulder restraint may further comprise a latch assembly configured to selectively secure the shoulder restraint portion relative to the shoulder restraint connection element. The base may comprise a longitudinal slot and the shoulder restraint connection element may comprise a lateral slot. At least a portion of the latch assembly may extend through the longitudinal slot and the lateral slot and, when in a locked configuration, the latch assembly may be operative to secure the shoulder restraint portion relative to the shoulder restraint connection element. In the locked configuration, the latch assembly may compress the base and the shoulder restraint connection element between a housing disposed against the base and the locking element disposed against the shoulder restraint connection element. In the locked configuration, the latch assembly may be in an over-center condition. The shoulder restraint may also include a shoulder pad disposed thereon. The shoulder restraint may be constructed substantially from materials that generally do not interfere with medical imaging modalities. The shoulder restraint may be constructed substantially from non-metallic materials.

The head stabilization system may further comprise a patient support plate assembly and the patient support plate may be configured to be secured to the patient support device. The patient support device may comprise an angiography table. The head stabilization system may further comprise at least one support plate attachment element configured to secure the patient support plate assembly to the patient support device. The at least one support plate attachment element may comprise at least one of a strap and a clamp. The patient support plate assembly may comprise at least one support plate assembly attachment element configured to engage the support plate attachment element. The at least one support plate assembly attachment element may comprise at least one fastening slot. The head restraint mechanism may be mounted to the patient support plate assembly. The head restraint mechanism may be selectively pivotably coupled to the patient support plate assembly by a head restraint mechanism connector. The head restraint mechanism connector may comprise a connector base affixed to the patient support plate assembly and a connector cap that extends through a bore through the head restraint mechanism connector. The head restraint mechanism connector may be connectable by rotating the connector cap with respect to the connector base in a first direction. The head restraint mechanism connector may be releasable by rotating the connector cap with respect to the connector base in a second direction, the second direction being opposite of the first direction. The head restraint mechanism may comprise a swivel arm, the swivel arm being pivotably coupled to the patient support plate assembly by the head restraint mechanism connector. One or more interfacing surfaces of at least one of the swivel arm and the head restraint mechanism connector may comprise a lubricious material for easier movement of the swivel arm by a user, for example. The swivel arm may comprise a swivel lock assembly configured to selectively secure the swivel arm in an angular position and the patient support plate assembly may comprise a swivel lock engagement element configured to operatively engage the swivel lock assembly. The swivel lock engagement element may comprise an edge of the patient support plate assembly. The edge of the patient support plate assembly may comprise a slot shaped substantially as a radius with respect to the head restraint mechanism connector and the slot may comprise a plurality of teeth. The swivel lock assembly may comprise an extendable tooth or teeth slidably disposed in the slot and the extendable tooth or teeth may be configured to selectively engage the plurality of teeth of the slot. One or more interfacing surfaces of at least one of the swivel lock assembly and the swivel lock engagement element may comprise a lubricious material.

The patient support plate assembly may comprise a patient support plate assembly shoulder restraint connection element configured to engage a shoulder restraint. The patient support plate assembly shoulder restraint connection element may comprise a retention element configured to releasably secure the shoulder restraint. The patient support plate assembly shoulder restraint connection element may comprise a laterally oriented slot configured to receive the shoulder restraint. The retention element may comprise an upwardly extending boss configured to releasably secure the shoulder restraint in the slot of the patient support plate assembly shoulder restraint connection. The patient support plate assembly shoulder restraint connection element may be formed of an elastomeric material. The patient support plate assembly may be constructed substantially from materials that generally do not interfere with medical imaging modalities. The patient support plate assembly may be constructed substantially from non-metallic materials.

In alternative or additional aspects, the head stabilization system may include a chest elevator configured to facilitate vertical positioning of an upper torso of the patient relative to the patient support device. The chest elevator may comprise an inflatable membrane configured to be disposed beneath the upper torso of the patient such that inflating the inflatable membrane raises the upper torso of the patient and deflating the inflatable membrane lowers the upper torso of the patient. The chest elevator may be operatively coupled to a pressurized air source.

In some embodiments, a head stabilization system may comprise a patient support plate assembly configured to be secured to a patient support device and to receive at least a portion of a patient thereon, a head harness configured to engage a head of the patient, a head restraint mechanism disposed on the patient support plate assembly, the head harness being releasably attachable to and at least partially supported by the head restraint mechanism. The head restraint mechanism may be configured to stabilize the head harness relative to the patient support plate assembly. The head restraint mechanism may be configured to selectively allow lateral flexion of a neck of the patient, lateral rotation of the head of the patient, and extension and flexion of the neck of the patient. The head restraint mechanism may be pivotably disposed on the patient support plate assembly.

In alternative or additional aspects, the head stabilization system may further comprise a first shoulder restraint and a second shoulder restraint disposed on the patient support plate. The first shoulder restraint may be configured to engage a first shoulder of the patient and the second shoulder restraint may be configured to engage a second shoulder of the patient. The first shoulder restraint and the second shoulder restraint may be laterally and longitudinally repositionable on the patient support plate assembly. The head stabilization system may further comprise a chest elevator disposed on the patient support plate and configured to position an upper torso of the patient. The patient support plate assembly, the head harness, the head restraint mechanism, and the chest elevator may be constructed substantially from materials that generally do not interfere with medical imaging modalities. The patient support plate assembly, the head harness, the head restraint mechanism, and the chest elevator may be constructed substantially from non-metallic materials.

In alternative embodiments, a head stabilization system may comprise a patient support plate assembly, a head harness, and a head restraint mechanism and may be configured to stabilize the head of the patient with the patient in a generally supine position. The head harness may be configured to engage a head of a patient. The head restraint mechanism may be configured to be operatively disposed on a patient support device. The head harness may be releasably attachable to and at least partially supported by the head restraint mechanism. The head restraint mechanism may be configured to stabilize the head harness relative to the patient support device. The head restraint mechanism may be configured to selectively allow vertical positioning of the head of the patient, rotation of the head of the patient, and extension and flexion of a neck of the patient. The head harness and the head restraint mechanism may be configured to stabilize the head of the patient with the patient in a generally lateral position. The head restraint mechanism may be configured to selectively provide about 30 degrees of lateral flexion of the neck of the patient, up and down, for a total of about 60 degrees of flexion of the neck of the patient. The head restraint mechanism may be configured to selectively provide about 60 degrees of rotation of the head of the patient, to the patient's right side and to the patient's left side, for a total of about 120 degrees of rotation of the head of the patient. The head restraint mechanism may be configured to selectively provide about 25 degrees of neck extension and about 25 degrees of neck flexion, for a total of about 50 degrees of extension and flexion of the neck of the patient.

In some embodiments, the head harness of the head stabilization system may be configured to securely engage the head of the patient in a non-traumatic, non-penetrating manner. The head harness may be configured to engage a chin/jaw region, a forehead region, and a rear region of the head of the patient. The head harness may comprise a halo portion configured to engage the forehead region. The halo portion may comprise a right halo strap configured to extend front-to-back along the head of the patient, a left halo strap configured to extend front-to back along the head of the patient, and a halo adjustment mechanism coupled to the right halo strap and the left halo strap. The halo adjustment mechanism may be configured to allow adjustment of the halo portion to fit the head of the patient. The halo adjustment mechanism may further comprise a halo bracket coupled to the right halo strap and the left halo strap a halo clip coupled to the halo bracket and a halo clamp coupled to the halo clip.

The head harness may comprise a chin strap portion configured to engage the chin/jaw region of the head of the patient. The head harness may further comprise a right halo stabilizer connecting the chin strap portion to the right halo strap and a left halo stabilizer connecting the chin strap portion to the left halo strap. The right halo stabilizer may comprise a right halo stabilizer halo strap portion connected to the right halo strap, a right chin strap end connector connected to the right halo stabilizer chin strap portion, and a right adjustable buckle type connector connected to the right chin strap end connector and a right chin strap portion. The right chin strap end connector may be releasably secured to the right adjustable buckle type connector by a releasable catch operable by a catch release. The right adjustable buckle type connector may comprise a catch release that, when actuated, releases the right halo stabilizer chin strap portion from the right halo stabilizer chin strap portion. The catch release may be configured for one handed operation.

The left halo stabilizer may comprise a left halo stabilizer halo strap portion connected to the left halo strap, a left chin strap end connector connected to the left halo stabilizer chin strap portion, and a left adjustable buckle type connector connected to the left chin strap end connector and a left chin strap portion. The left chin strap end connector may be releasably secured to the left adjustable buckle type connector by a releasable catch operable by a catch release. The left adjustable buckle type connector may comprise a catch release that, when actuated, releases the left halo stabilizer chin strap portion from the left halo stabilizer chin strap portion. The catch release may be configured for one handed operation.

The head harness may further comprise a rear support portion configured to engage the rear region. The rear support portion may comprise a generally annular ring configured to be positioned centrally on the rear region of the patient's head. The rear support portion may be connected to the right halo strap and the left halo strap. The head harness may further comprise a head restraint portion connecting the rear support portion and the halo adjustment mechanism, the head restraint portion being configured to extend centrally front-to-back over the head of the patient. The head restraint portion may comprise a head restraint element disposed on a head restraint strap portion. The head restraint element may comprise a connecting element configured to releasably couple the head harness to the head restraint mechanism. The connecting element may comprise a harness connector housing. The harness connector housing may comprise a cavity and a slot configured to releasably engage the head restraint mechanism. The head restraint mechanism may comprise a harness connector assembly configured to releasably engage the head restraint element.

In alternative or additional aspects, the harness connector assembly may comprise a harness connector disposed on a harness connector shaft and the harness connector may be configured to be releasably received within the cavity of the harness connector housing. The connecting element may comprise a latch operative to selectively secure the harness connector in the cavity and the latch may be releasable by an actuator. The actuator may be configured for one handed operation.

The harness connector assembly and the connecting element may be selectively engageable at a plurality of discrete relative angular positions. The head restraint mechanism may comprise a generally horizontally oriented arcuate tilt guide. The head harness may be selectively repositionable generally horizontally relative to the tilt guide. Repositioning the head harness generally horizontally relative to the tilt guide may be operative to provide extension and flexion of the neck of the patient.

The head harness may be releasably attachable to the head restraint mechanism by connection of a connecting element of the head harness with a harness connector assembly of the head restraint mechanism. The harness connector assembly may be selectively slidably disposed in a generally horizontally oriented tilt guide slot of the tilt guide. The harness connector assembly may comprise a harness connector knob operatively arranged to selectively secure the harness connector assembly in the tilt guide slot. The harness connector knob may be operative to secure the relative angular position of the head harness relative to the head restraint mechanism. The tilt guide may be disposed on a vertical support, the tilt guide being adjustably connected relative to the patient support device such that positioning the tilt guide along a vertical axis provides for vertical positioning of the head of the patient. The vertical axis may be substantially perpendicular to an upper surface of the patient support device. The vertical support may comprise a connector assembly configured to selectively secure the tilt guide vertically in relation to the patient support device. The tilt guide may be selectively repositionable vertically along the vertical support.

In alternative or additional aspects, the head restraint mechanism may be coupled to the patient support plate assembly by a head restraint mechanism connector. The head restraint mechanism connector may comprise a head restraint connector base affixed to the patient support plate assembly, a vertical support mounting plate, and a plurality of lock devices. The vertical support may be coupled to the vertical support mounting plate. The head restraint mechanism may be selectively positioned horizontally in relation to the patient support plate assembly by positioning the vertical support mounting plate with respect to the head restraint connector base. The head restraint mechanism may be secured in relation to the patient support plate assembly by operating the lock devices. The head restraint mechanism connector may include an incremental adjustment mechanism. The vertical support mounting plate may comprise a plurality of teeth and the head restraint connector base may comprise a plurality of teeth configured to selectively engage the teeth of the vertical support mounting plate.

A head stabilization system may further comprise a torso pad configured to facilitate vertical positioning of an upper torso of the patient relative to the patient support device. The torso pad may comprise an inflatable membrane configured to be disposed beneath the upper torso of the patient such that inflating the inflatable membrane raises the upper torso of the patient and deflating the inflatable membrane lowers the upper torso of the patient. The torso pad may be operatively coupled to a pressurized air source.

A head stabilization system may further comprise a neck pad configured to facilitate vertical positioning a neck of the patient relative to the patient support device. The neck pad may comprise an inflatable membrane configured to be disposed beneath the neck of the patient such that inflating the inflatable membrane supports the neck of the patient. The neck pad may be operatively coupled to a pressurized air source.

Generally, a method of stabilizing a head of a patient is contemplated and may comprise attaching a head harness to a head of a patient, attaching the head harness to a head restraint mechanism, positioning the head of the patient, and securing the head restraint mechanism. The method of stabilizing the head of the patient may comprise attaching the head harness to the head of the patient while the head harness is disconnected from the head restraint mechanism. Attaching the head harness to the head restraint mechanism may be performed with the head harness attached to the head of the patient.

Alternatively, a method of stabilizing a head of a patient may comprise attaching a head harness to a head restraint mechanism, attaching the head harness to the head of the patient, positioning the head of the patient and securing the head restraint mechanism. The method of stabilizing the head of the patient may comprise attaching the head harness to the head of the patient after attaching the head harness to the head restraint mechanism. Attaching the head harness to the head restraint mechanism may be performed before attaching the head harness to the head of the patient.

In some embodiments, a method of stabilizing a head of a patient is contemplated wherein the head restraint mechanism may be configured to stabilize the head harness relative to a patient support device. The method may comprise placing the patient on the patient support device. The method may comprise placing the patient on the patient support device in a generally supine position. The head harness may be attached to the head of the patient before placing the patient on the patient support device. Alternatively, the head harness may be attached to the head of the patient after placing the patient on the patient support device.

Alternatively, a method of stabilizing a head of a patient is contemplated wherein the head restraint mechanism is configured to be disposed on a patient support plate assembly and the method may further comprise attaching the patient support plate assembly to the patient support device. The method may further comprise installing the head restraint mechanism on the patient support plate assembly. The method may further comprise installing a first shoulder restraint and a second shoulder restraint on the patient support plate assembly. The method may further comprise installing a chest elevator on the patient support plate assembly. In alternative or additional aspects, the method may further comprise installing at least one pad.

In some embodiments, a method of stabilizing a head of a patient is contemplated wherein attaching the head harness to the head of the patient may comprise positioning a halo portion and a rear support portion to substantially circumscribe the head of the patient. The method may further comprise adjusting the halo portion using a halo adjustment knob. The method may further comprise positioning a chin strap portion on a chin/jaw region of the head of the patient. The method may further comprise attaching the head harness to the head of the patient. The head harness may comprise at least one halo stabilizer. The at least one halo stabilizer may comprise a halo stabilizer chin strap portion and a halo stabilizer halo strap portion. The method of attaching the head harness to the head of the patient may include coupling a halo stabilizer chin strap portion with a halo stabilizer halo strap portion. The method of attaching the head harness to the head of the patient may further comprise securing a head restraint portion extending centrally front-to-back over a top of the head of the patient.

In some embodiments, a method of stabilizing a head of a patient may further comprise adjusting a first shoulder restraint and a second shoulder restraint. Adjusting the first shoulder restraint and the second shoulder restraint may comprise repositioning at least one of the first shoulder restraint and the second should restraint at least one of laterally and longitudinally. Adjusting the first shoulder restraint and the second shoulder restraint may include at least one of unlocking a latch assembly and locking the latch assembly, the latch assembly being configured to selectively secure at least one of the first shoulder restraint and the second shoulder restraint. Adjusting the first shoulder restraint and the second shoulder restraint may include applying traction to a neck of the patient by applying a force on shoulders of the patient in a generally inferior direction.

In alternative or additional aspects, a method of attaching the head harness to the head restraint mechanism may comprise coupling a harness connector housing of the head harness to a harness connector assembly of the head restraint mechanism. The harness connector housing may comprise a cavity and a slot and the harness connector assembly may comprise a harness connector stop disposed on a harness connector shaft. The method of attaching the head harness to the head restraint mechanism may comprise positioning the harness connector stop within the cavity and the harness connector shaft within the slot. The harness connector stop may comprise a generally spherical ball and the slot may comprise a generally circular opening. Positioning the harness connector stop within the cavity may comprise seating the generally spherical ball against the generally circular opening.

In some embodiments, a method of stabilizing a head of a patient is contemplated and positioning the head of the patient may comprise laterally flexing a neck of the patient. The method may include pivoting the head restraint mechanism about an axis that is generally orthogonal to an upper surface of the patient support device.

In alternative or additional aspects, a method of stabilizing a head of a patient is contemplated and positioning the head of the patient may comprise rotating the head of the patient. The method may include rotating the head of the patient about a harness connector shaft connecting the head harness to the head restraint mechanism.

In another embodiment, a method of stabilizing a head of a patient is contemplated and positioning the head of the patient may comprise at least one of extending a neck of the patient and flexing the neck of the patient. The method may comprise vertically repositioning a harness connector assembly along an arcuate tilt guide. The method may comprise repositioning the harness connector assembly by slidably repositioning the harness connector assembly along the tilt guide slot. The method may further comprise securing the harness connector assembly on the tilt guide by installing a locking pin through a laterally extending tilt guide through hole and a laterally extending harness connector assembly through hole.

In some embodiments, a method of stabilizing a head of a patient may comprise securing the head restraint mechanism with the head of the patient positioned at a desired lateral flexion of the neck of the patient, at a desired lateral rotation of the head of the patient, and one of a desired neck extension and a desired neck flexion. The method may further comprise tightening a swivel lock assembly. Tightening the swivel lock assembly may include engaging an extendable tooth or teeth associated with the swivel lock assembly with a plurality of teeth associated with a swivel lock engagement element. The method may further comprise engaging a radially toothed surface associated with the head harness with a radially toothed surface associated with the head restraint mechanism. The method may further comprise tightening a harness connector assembly operatively coupling the head harness to the head restraint mechanism.

In alternative or additional aspects, a method of stabilizing a head of a patient may comprise raising an upper torso of the patient using a chest elevator. The method of raising the upper torso of the patient may further comprise at least partially inflating an inflatable membrane of the chest elevator. The method may further comprise lowering an upper torso of the patient using a chest elevator. The method of lowering the upper torso of the patient may further comprise at least partially deflating an inflatable membrane of the chest elevator.

A method of stabilizing a head of a patient may further comprise, after securing the head restraint mechanism, performing a procedure on the patient. The method may further comprise, removing the head harness from the head of the patient. The method may further comprise detaching the head harness from the head restraint mechanism. The method may further comprise loosening the head restraint mechanism.

In alternative or additional aspects, attaching the head harness to the head of the patient may comprise securely engaging the head of the patient with the head harness in a non-traumatic, non-penetrating manner.

A method of stabilizing a head of a patient is contemplated and the method may comprise attaching a head harness to a head of a patient, attaching the head harness to a head restraint mechanism, positioning the head of the patient, and securing the head restraint mechanism. The method of attaching the head harness to the head of the patient may be performed while the head harness is disconnected from the head restraint mechanism. The method of attaching the head harness to the head restraint mechanism may be performed with the head harness attached to the head of the patient.

In some embodiments, a method of stabilizing a head of a patient is contemplated and the head restraint mechanism may be configured to stabilize the head harness relative to a patient support device. The method may further comprise placing the patient on the patient support device. The method may include placing the patient on the patient support device in a generally lateral position. The head harness may be attached to the head of the patient before placing the patient on the patient support device. Alternatively, the head harness may be attached to the head of the patient after placing the patient on the patient support device.

In some embodiments, a method of attaching a head harness to a head of a patient may comprise positioning a halo portion and a rear support portion to substantially circumscribe the head of the patient. The method may further comprise adjusting the halo portion using a halo adjustment mechanism. The method may further comprise positioning a chin strap portion on a chin/jaw region of the head of the patient. The method may further comprise coupling a halo stabilizer chin strap portion with a chin strap portion. The method may further comprise securing a head restraint portion extending centrally front-to-back over a top of the head of the patient.

In alternative or additional aspects, a method of attaching a head harness to a head restraint mechanism may comprise coupling a harness connector housing of the head harness to a harness connector assembly of the head restraint mechanism. The harness connector housing may comprise a cavity and a slot and the harness connector assembly may comprise a harness connector stop disposed on a harness connector shaft. The method of attaching the head harness to the head restraint mechanism may comprise positioning the harness connector stop within the cavity and the harness connector shaft within the slot.

In alternative or additional aspects, a method of positioning the head of a patient may comprise vertically positioning the head of the patient. The method may further comprise moving a head restraint mechanism along an axis that is generally orthogonal to an upper surface of the patient support device.

In some embodiments, a method of positioning the head of a patient may comprise at least one of extending the neck of the patient and flexing the neck of the patient. The method may comprise horizontally repositioning a harness connector assembly along an arcuate tilt guide. The method may further comprise slidably repositioning the harness connector assembly along a tilt guide slot.

In some embodiments, a method of stabilizing a head of a patient may comprise positioning the upper torso of the patient using a torso pad. The method of positioning the upper torso of the patient may further comprise at least partially inflating an inflatable membrane of the torso pad.

In alternative or additional aspects, a method of stabilizing a head of a patient may comprise supporting the neck of the patient using a neck pad. The method of supporting the neck of the patient may further comprise at least partially inflating an inflatable membrane of the neck pad.

A method of manufacturing a head stabilization system may comprise installing a head restraint mechanism onto a patient support plate assembly. The method may further comprise installing at least one shoulder restraint. The method may further comprise installing the at least one shoulder restraint onto a patient support plate assembly. The method may further comprise installing a chest elevator. Installing the chest elevator may comprise installing the chest elevator onto a patient support plate assembly.

Alternatively, a method of manufacturing a head stabilization system may comprise installing a patient support plate assembly onto a patient support device. The method may further include installing a head restraint mechanism on the patient support plate assembly. The method may further comprise installing a torso pad on the patient support plate assembly. The method may further include installing a neck pad on the patient support plate assembly.

The invention is defined in the appended claims. Additional features, options or aspects of the invention will become more apparent through a review of a detailed description of various illustrative embodiments described in more detail herein, taken in conjunction with the accompanying drawings of these illustrative embodiments.

### Brief Description of Drawings

FIG. 1 is an isometric view of an illustrative head stabilization system in use on a patient, wherein the patient is positioned in a general supine position.
FIG. 2 is an isometric view of the illustrative head stabilization system shown in FIG. 1.
FIG. 3 is a plan view of the illustrative head stabilization system shown in FIGS. 1 and 2 showing lateral positioning of the patient's head.
FIG. 4 is a cranial elevation view of an illustrative head stabilization system shown in FIGS. 1 and 2 showing rotational positioning of the patent's head.
FIG. 5 is a lateral elevation view of an illustrative head stabilization system shown in FIGS. 1 and 2 showing extension of the patient's neck.
FIG. 6 is a lateral elevation view of an illustrative head stabilization system shown in FIGS. 1 and 2 showing compression of the patient's neck.
FIG. 7 is an isometric view of an illustrative head harness disposed on a patient's head.
FIG. 8 is another isometric view of the illustrative head harness shown in FIG. 7 disposed on a patient's head.
FIG. 9 is an isometric view of an illustrative head restraint mechanism.
FIG. 10 is another isometric view of the illustrative head restraint mechanism shown in FIG. 9.
FIG. 11 is a perspective view of the illustrative head restraint mechanism shown in FIG. 9.
FIG. 12 is an isometric view of an illustrative shoulder restraint in a locked configuration.
FIG. 13 is an isometric partial cutaway view of the illustrative shoulder restraint shown in FIG. 12 in an unlocked configuration.
FIG. 14 is another isometric view of the illustrative shoulder restraint shown in FIG. 12 in a locked configuration.
FIG. 15 is an isometric view of an illustrative patient support plate assembly.
FIG. 16 is an isometric view of the illustrative head stabilization system shown in FIGS. 1 and 2 in use on a patient with the head harness disconnected from the head restraint mechanism.
FIG. 17 is an isometric view of an alternative illustrative head restraint mechanism.
FIG. 18 is an isometric view of an alternative illustrative head harness.
FIG. 19 is an isometric view of an alternative illustrative head restraint mechanism.
FIG. 20 is an isometric view of an alternate illustrative head stabilization system in use on a patient, wherein the patient is positioned in a generally lateral position.
FIG. 21 is an isometric view of the illustrative head stabilization system shown in FIG. 20.
FIG. 22 is a lateral elevation view of the illustrative head stabilization system shown in FIGS. 20 and 21 showing vertical positioning of the patient's head.
FIG. 23 is a cranial elevation view of the illustrative head stabilization system shown in FIGS. 20 and 21 showing rotational positioning of the patent's head.
FIG. 24 is a plan view of the illustrative head stabilization system shown in FIGS. 20 and 21 showing extension of the patient's neck.
FIG. 25 is a plan view of the illustrative head stabilization system shown in FIGS. 20 and 21 showing flexion of the patient's neck.
FIG. 26 is an isometric view of an alternate illustrative head harness disposed on a patient's head.
FIG. 26A is an exploded view of the illustrative head harness shown in FIG. 26.
FIG. 27 is an alternate isometric view of the illustrative head harness shown in FIG. 26 disposed on a patient's head.
FIG. 28 is an isometric view of an alternate illustrative head restraint mechanism.
FIG. 29 is an exploded view of the illustrative head restraint mechanism shown in FIG. 28.
FIG. 30 is an alternate isometric view of the illustrative head restraint mechanism shown in FIG. 28.
FIG. 31A is an isometric view of an alternate illustrative shoulder restraint.
FIG. 31B is an alternate isometric view of the illustrative shoulder restraint shown in FIG. 31A.
FIG. 32A is an isometric view of an illustrative shoulder pad shown in FIGS. 31A and 31B.
FIG. 32B is another isometric view of the illustrative shoulder pad shown in FIG. 32A.
FIG. 33 is an isometric view of the illustrative shoulder restraint bracket shown in FIGS. 31A and 31B.
FIG. 34 is an isometric view of an alternate illustrative shoulder restraint.
FIG. 35 is an isometric view of the illustrative shoulder restraint shoulder pad shown in FIG. 34.
FIG. 36 is an isometric view of an alternate illustrative head stabilization system in use on a patient, wherein the patient is positioned in a general supine position.
FIG. 37 is an isometric view of the illustrative head stabilization system shown in FIG. 36.
FIG. 38 is a plan view of the illustrative head stabilization system shown in FIGS. 36 and 37 showing lateral positioning of the patient's head.
FIG. 39 is a cranial elevation view of the illustrative head stabilization system shown in FIGS. 36 and 37 showing rotational positioning of the patent's head.
FIG. 40 is a lateral elevation view of the illustrative head stabilization system shown in FIGS. 36 and 37 showing extension of the patient's neck.
FIG. 41 is a lateral elevation view of the illustrative head stabilization system shown in FIGS. 36 and 37 showing compression of the patient's neck.
FIG. 42 is an isometric view of an alternate illustrative head harness disposed on a patient's head.
FIG. 43 is an alternate isometric view of the illustrative head harness shown in FIG. 42 disposed on a patient's head.
FIG. 44 is an isometric view of an alternate illustrative head restraint mechanism.
FIG. 45 is another isometric view of the illustrative head restraint mechanism shown in FIG. 44.
FIG. 46 is an exploded view of the illustrative head restraint mechanism shown in FIGS. 44 and 45.
FIG. 47 is an isometric view of an alternate illustrative patient support plate assembly.
FIG. 48 is an alternate isometric view of the illustrative patient support plate assembly shown in FIG. 47.
FIG. 49 is an isometric view of an alternate illustrative head stabilization system in use on a patient, wherein the patient is positioned in a generally lateral position.
FIG. 50 is a lateral elevation view of the illustrative head stabilization system shown in FIG. 49 showing vertical positioning of the patient's head.
FIG. 51 is a cranial elevation view of the illustrative head stabilization system shown in FIG. 49 showing rotational positioning of the patent's head.
FIG. 52 is a plan view of the illustrative head stabilization system shown in FIG. 49 showing extension and flexion of the patient's neck.
FIG. 53 is an isometric view of an alternate illustrative head harness disposed on a patient's head.
FIG. 54 is an alternate isometric view of the illustrative head harness shown in FIG. 53 disposed on a patient's head.
FIG. 55 is an isometric view of an illustrative head harness disposed on a patient's head.
FIG. 56 is an alternate isometric view of the illustrative head harness shown in FIG. 53 disposed on a patient's head.
FIG. 57 is an isometric view of an illustrative head harness disposed on a patient's head.
FIG. 58 is an alternate isometric view of the illustrative head harness shown in FIG. 53 disposed on a patient's head.

### Detailed Description

Illustrative embodiments according to at least some aspects of the present disclosure are described and illustrated below and include devices and methods relating to medical procedures. Of course, it will be apparent to those of ordinary skill in the art that the embodiments discussed below are examples and may be reconfigured without departing from the scope of the present disclosure. It is also to be understood that variations of the exemplary embodiments contemplated by one of ordinary skill in the art shall concurrently comprise part of the instant disclosure. However, for clarity and precision, the illustrative embodiments as discussed below may include optional steps, methods, and features that one of ordinary skill should recognize as not being a requisite to fall within the scope of the present disclosure.

The present disclosure includes, among other things, patient head stabilization systems. Some illustrative embodiments according to at least some aspects of the present disclosure may be used as patient head positioning and stabilization systems for minimally invasive neurointerventional procedures. While the present detailed description of illustrative embodiments focuses on minimally invasive neurointerventional procedures, it will be appreciated that various embodiments according to at least some aspects of the present disclosure may be utilized in connection with other procedures.

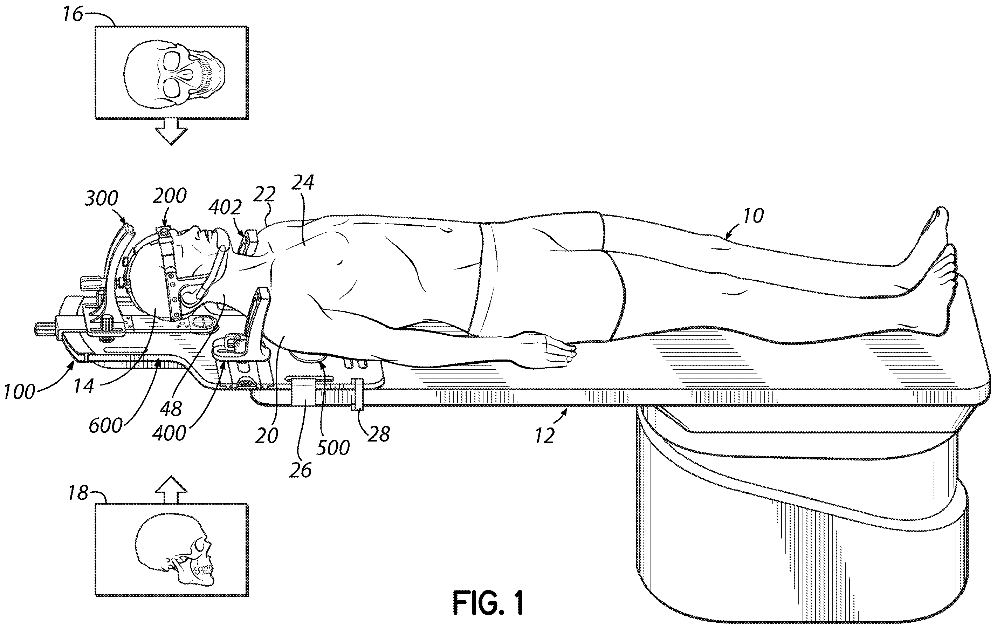

FIG. 1 is an isometric view of an illustrative head stabilization system 100 in use on a patient 10, according to at least some aspects of the present disclosure. The head stabilization system 100 is disposed on a patient support device, such as generally at the head end of an angiography table 12. The head stabilization system 100 is configured to facilitate various procedures, such as imaging of the patient's head 14 by one or more imaging devices 16, 18, which may be selectively positioned with respect to the table 12. Some illustrative embodiments may be used in connection with procedures involving accessing the cerebrospinal fluid space via a C1-C2 puncture site 48, for example.

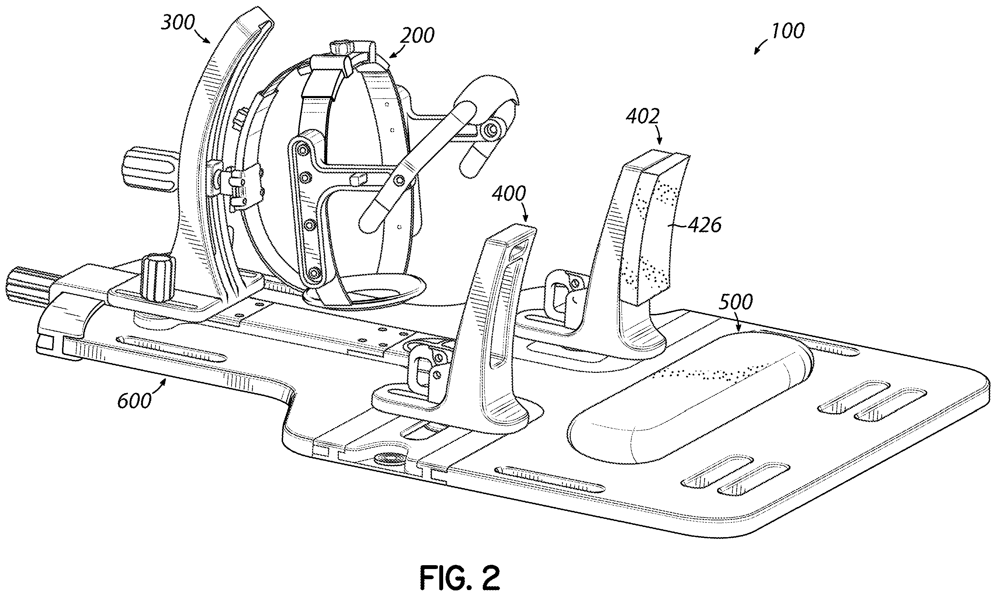

FIG. 2 is an isometric view of the illustrative head stabilization system 100, according to at least some aspects of the present disclosure. Referring to FIGS. 1 and 2, the illustrative head stabilization system 100 is generally configured to facilitate the desired positioning of the patient's head 14 and/or to support and stabilize the patient's head 14 (e.g., hold the patient's head 14 stationary), such as during a procedure. The illustrative head stabilization system 100 includes a head harness 200 configured to engage the patient's head 14 and a head restraint mechanism 300 to which the head harness 200 is releasably attachable. The head restraint mechanism 300 is configured to facilitate positioning and stabilization of the patient's head 14 with respect to the table 12 using the head harness 200. The illustrative head stabilization system 100 includes one or more shoulder restraints 400, 402 configured to engage the patient's shoulders 20, 22 and a chest elevator 500 configured to adjust the vertical position of the patient's upper torso 24. The head restraint mechanism 300, shoulder restraints 400, 402, and the chest elevator 500 are disposed on a support plate assembly 600. The support plate assembly 600 is secured to the table 12, such as by one or more support plate attachment elements, such as one or more straps 26 and/or clamps 28. Generally, the head stabilization system 100 allows the patient's head 14 to be positioned and/or oriented within a three-dimensional space, relative to the imaging devices 16, 18 and the table 12 and stabilizes the patient's head 14 in the desired position.

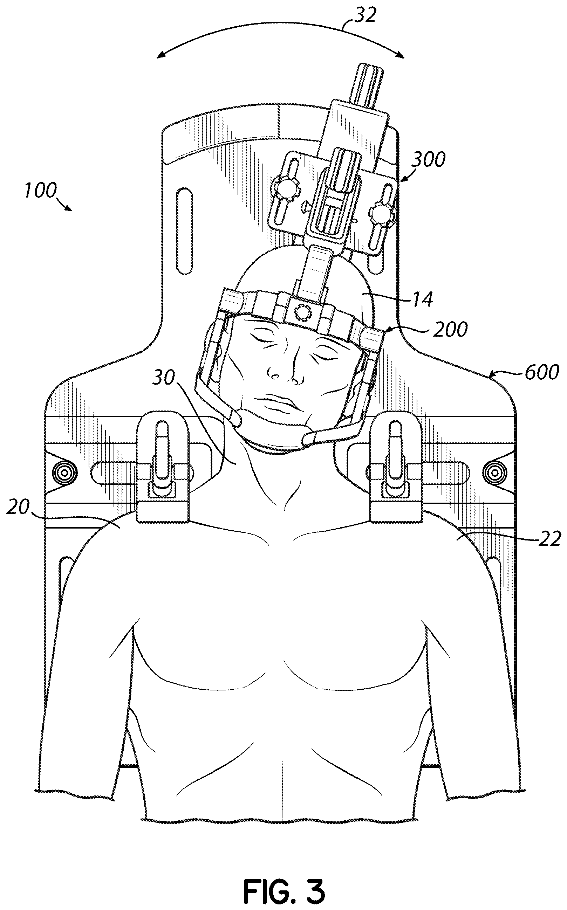

Referring to FIGS. 3-6, a summary overview of the potential positioning of the patient's head 14 facilitated by the head stabilization system 100 is provided in order to give context for the descriptions below. Generally, the illustrative head stabilization system 100 may be positioned and/or secured at any position and/or orientation within any combination of the ranges of motion described with reference to FIGS. 3-6 to position and/or stabilize the patient's head 14 as desired, such as for a procedure.

FIG. 3 is a plan view of the illustrative head stabilization system 100 showing lateral positioning of the patient's head 14, according to at least some aspects of the present disclosure. Generally, this articulation of the head restraint mechanism 300 provides lateral flexion of the patient's neck 30, left or right, as shown by arrow 32, in a plane generally parallel to the upper surface of the support plate assembly 600. This lateral flexion motion is similar to that which would occur if the patient tilted his or her head to the right or left to touch the ear to the shoulder 20, 22 on the same side, without rotating the head 14. This illustrative head stabilization system 100 facilitates about 30° of lateral flexion left of center and 30° of lateral flexion right of center, for a total range of lateral flexion of about 60° from farthest left to farthest right.

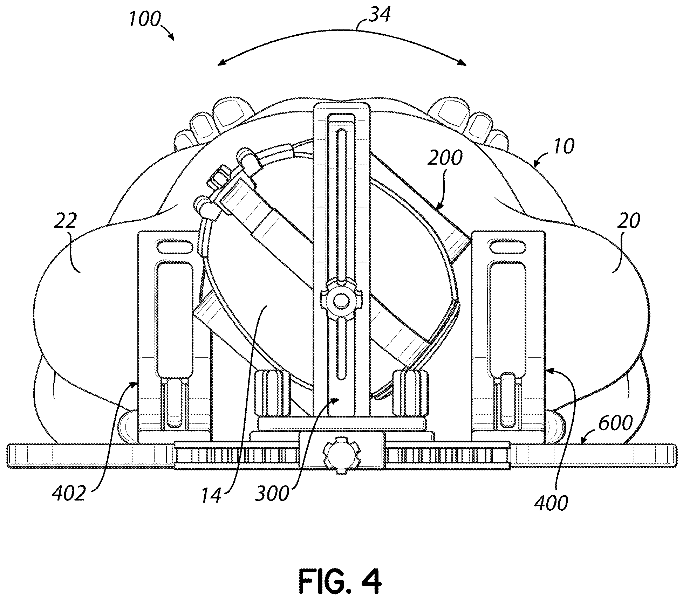

FIG. 4 is a cranial elevation view of the illustrative head stabilization system 100 showing rotational positioning of the patent's head 14, according to at least some aspects of the present disclosure. Generally, this articulation of the head harness 200 relative to the head restraint mechanism 300 provides lateral rotation of the patient's head 14, left or right, as shown by arrow 34, in a plane generally transverse to the upper surface of the support plate assembly 600. As used herein, "transverse" may refer to relative angular orientations that are non-parallel (e.g., perpendicular or oblique). This motion is generally similar to that which would occur if the patient attempted to shake his or her head 14 side to side as to say "no." This illustrative head stabilization system 100 facilitates about 60° of lateral rotation to the left and about 60° right, for a total range of lateral rotation of about 120°.

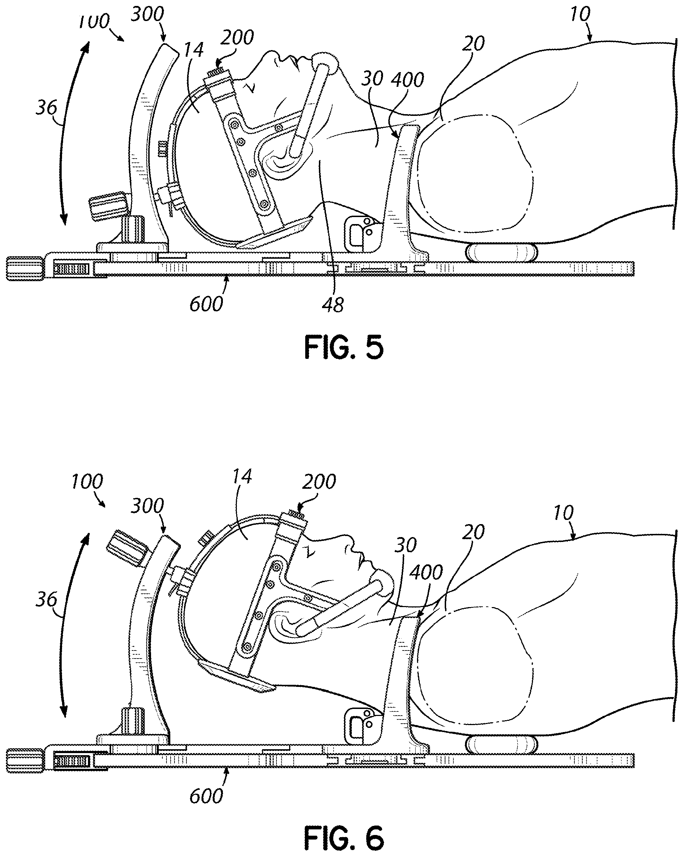

FIGS. 5 and 6 are lateral elevation views of the illustrative head stabilization system 100 showing extension and compression, respectively, of the patient's neck 30, according to at least some aspects of the present disclosure. Generally, this articulation of the head restraint mechanism 300 provides extension and flexion of the patient's neck 30, backward (i.e., extension) and forward (i.e., flexion), as shown by arrow 36, in a plane generally perpendicular to the upper surface of the support plate assembly 600. These motions are generally similar to those which would occur if the patient attempted to nod his or her head 14 up and down as to say "yes". This illustrative head stabilization system 100 facilitates about 25° of neck extension upward (from neutral) and about 25° of neck flexion downward (from neutral), for a total range of extension and flexion of about 50°. The position shown in FIG. 5 may improve access to the cerebrospinal fluid space via the C1-C2 puncture site 48, facilitating lateral, posterior needle introduction. The position shown in FIG. 6 may improve access to the anterior anatomy of the brain. In some circumstances, it may be advantageous to apply traction to the patient's neck 30. For example, the head restraint mechanism 300 (via the head harness 200) may apply a force on the patient's head 14 in a generally superior/cranial direction and/or the shoulder restraints 400, 402 may apply a force on the patient's shoulders 20, 22 generally in an inferior/caudal direction. Accordingly, the patient's neck 30 may be placed in tension, generally longitudinally.

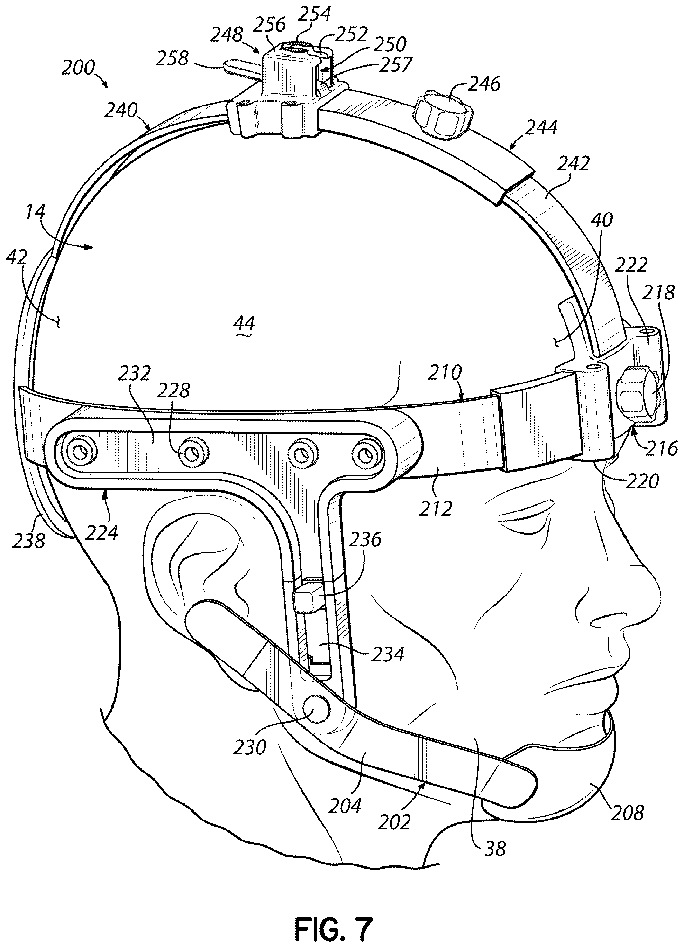

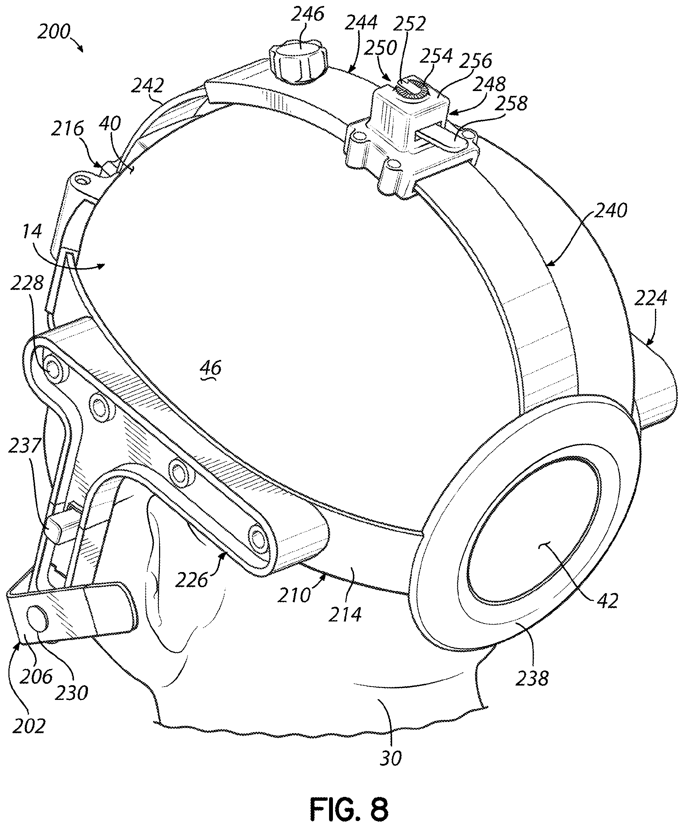

FIGS. 7 and 8 are isometric views of an illustrative head harness 200 disposed on a patient's head 14, according to at least some aspects of the present disclosure. The head harness 200 is configured to be secured to the patient's head 14 and to be coupled (e.g., releasably) to the head restraint mechanism 300 (FIG. 2). Generally, the illustrative head harness 200 is configured to engage the patient's head 14 at a chin/jaw region 38, a forehead region 40, and rear region 42. In some exemplary embodiments, the head harness 200 may be configured to securely engage the patient's head in a non-traumatic, non-penetrating manner (e.g., no component of the head harness 200 may penetrate the patient's skin).

The illustrative head harness 200 includes a chin strap portion 202 configured to engage the patient's chin/jaw region 38. The chin strap portion 202 facilitates control of the position of the patient's chin relative to the patient's neck 30. The chin strap portion 202 includes a right chin strap portion 204 and a left chin strap portion 206 that extend forward and downward generally along the patient's mandible to hold a chin pad 208 against the patient's chin/jaw region 38. The chin strap portions 204, 206 may be adjustable, such as by adjustable buckle type connectors (not shown).

The illustrative head harness 200 includes a halo portion 210 configured to engage the patient's forehead 40 and the lateral side portions 44, 46 of the patient's head 14. The halo portion 210 includes a right halo strap portion 212 and a left halo strap portion 214. The halo strap portions 212, 214 extend front-to-back along the patient's head 14. At the front, the halo strap portions 212, 214 are coupled to a halo adjustment mechanism 216, which includes a halo adjustment knob 218, a right halo adjustment hinge 220, and a left halo adjustment hinge 222 configured to allow adjustment of the halo portion 210 to securely fit an individual patient's head 14.

In the illustrative head harness 200, the halo portion 210 is connected to the chin strap portion 202 by a right halo stabilizer 224 and a left halo stabilizer 226. Each halo stabilizer 224, 226 is generally T-shaped, with the generally horizontal, upper portion affixed to the respective halo strap portion 212, 214 and the generally vertical, lower portion connecting to the respective chin strap portion 204, 206. In this illustrative embodiment, the T-shaped stabilizers 224, 226 are substantially rigid and each halo strap portion 212, 214 is connected to the respective stabilizer 224, 226, such as by one or more halo fasteners 228. The chin strap portions 204, 206 are pivotably attached at the lower ends of the vertical portions of the halo stabilizers 224, 226, such as by chin strap fasteners 230, which allow rotational adjustment to securely fit an individual patient's head 14.

Referring to FIG. 7, the right halo stabilizer 224 includes two releasably connected portions: the halo strap portion 232 and the chin strap portion 234. The halo strap portion 232 and the chin strap portion 234 are secured together by a releasable catch operated by an actuator, such as a release button 236. When actuated by a user, the release button 236 releases the chin strap portion 234 from the halo strap portion 232. In some embodiments, the button 236 may serve as an emergency release to allow the patient 10 to be quickly disconnected from the head stabilization system 100. For example, if the patient's airway becomes compromised, it may be desirable to quickly access the patient's mouth by detaching the chin strap portion 202 (e.g., by actuating the button 236 as a chin strap emergency release). In some embodiments, the button 236 may be configured for one-hand operation. The left halo stabilizer 226 is constructed in a substantially similar manner and includes a similar button 237 providing a chin strap emergency release.

The illustrative head harness 200 includes a rear support portion 238, which may be in the form of a generally annular ring that is configured to be positioned centrally on the rear region 42 of the patient's head 14. The rear support portion 238 is coupled to each of the halo strap portions 212, 214, so that the patient's head 14 is substantially circumscribed by the left halo strap portion 214, the halo adjustment mechanism 216, the right halo strap portion 212, and the rear support portion 238. The rear support portion 238 and the halo adjustment mechanism 216 are also connected by a head restraint portion 240 that extends centrally over the top of the patient's head 14 from front to back.

The head restraint portion 240 includes a head restraint strap portion 242 and a head restraint element 244 disposed thereon. The head restraint element 244 includes a head restraint strap adjustment knob 246 configured to allow adjustment of the head restraint strap portion 242 to securely fit an individual patient's head 14.

The illustrative head harness 200 includes a connecting element, such as a harness connector housing 248, which is configured to releasably couple the head harness 200 to the head restraint mechanism 300. The harness connector housing 248 is provided on the restraint element 244 and includes a cavity 250, a slot 252, and a toothed surface 254 configured to releasably engage corresponding components of the head restraint mechanism 300, as described below. The slot 252 extends through a wall 256, which at least partially defines the cavity 250. Additionally, the harness connector housing 248 includes a latch 257 releasable by an actuator, such as a finger tab 258, which is operatively coupled to selectively secure the head harness 200 (e.g., the harness connector housing 248) to the head restraint mechanism 300. The finger tab 258 may serve as an emergency release, allowing rapid disconnection of the head harness 200 from the head restraint mechanism when desired.

In this illustrative embodiment, the chin strap portions 204, 206, the halo strap portions 212, 214, and the head restraint strap 242 portion are constructed from flexible but substantially non-elastic materials. The halo stabilizers 224, 226 and the rear support portion 238 are constructed from generally rigid materials, such as rigid polymeric or composite materials. In some exemplary embodiments, padding may be provided on various patient contact surfaces of the head harness 200.

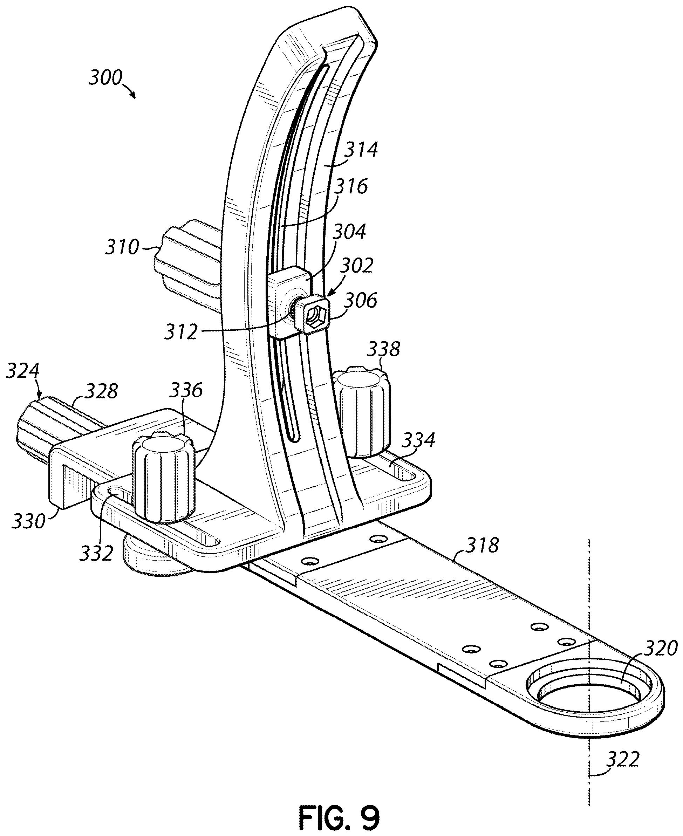

FIGS. 9-11 are isometric views of an illustrative head restraint mechanism 300, according to at least some aspects of the present disclosure. Generally, the head restraint mechanism 300 is configured to provide three-dimensional positioning of the patient's head. The head restraint mechanism 300 includes a selectively securable harness connector assembly 302. The illustrative harness connector assembly 302 includes a harness connector body 304 and an opposed harness connector stop 306 disposed on a harness connector shaft 308. A harness connector knob 310 is disposed on the harness connector shaft 308 and is operative to axially draw together the harness connector knob 310 and the harness connector stop 306, such as by a threaded engagement between the harness connector knob 310 and the harness connector shaft 308. The harness connector body 304 includes an axially oriented toothed surface 312 facing the harness connector stop 306.

Referring to FIGS. 7-11, the harness connector assembly 302 of the head restraint mechanism 300 is configured to releasably engage the harness connector housing 248 of the head harness 200. The harness connector stop 306 is sized and shaped to be received within the cavity 250, with the slot 252 receiving the harness connector shaft 308. The toothed surface 254 of the harness connector housing 248 engages the corresponding toothed surface 312 of the harness connector body 304. The toothed surfaces 254, 312 are engageable at a plurality of discrete relative angular positions across the range of lateral rotation as shown by arrow 34 in FIG. 4. Generally, the teeth of the toothed surfaces 254, 312 are radially oriented with respect to the harness connector shaft 308.

Referring to FIGS. 9-11, the head restraint mechanism 300 includes a generally vertically upstanding, arcuate tilt guide 314 having a generally vertically oriented tilt guide slot 316 therethrough. The harness connector shaft 308 of the harness connector assembly 302 extends through the tilt guide slot 316, between the harness connector knob 310 and the harness connector body 304. The harness connector knob 310 and the harness connector body 304 are wider than the tilt guide slot 316, so the harness connector assembly 302 is retained on the tilt guide 314. The harness connector assembly 302 is slidable generally vertically upward and downward along the length of the tilt guide slot 316 to provide the neck flexion and extension range of motion indicated by arrow 36 and as described above with reference to FIGS. 5 and 6.

With the stop 306 of the harness connector assembly 302 within the cavity 250 of harness connector housing 248 of the head harness 200, tightening the harness connector knob 310 is operative to secure the head harness 200 with respect to the tilt guide 314. More specifically, tightening the harness connector knob 310 secures the connector body 304 with respect to the tilt guide slot 316 and/or secures the relative angular positions of the toothed surfaces 254, 312. Loosening the harness connector knob 310 is operative to allow movement of the harness connector assembly 302 along the tilt guide slot 316 and/or to allow relative angular movement between the tooth surfaces 254, 312.

Referring to FIGS. 1-3 and 9-11, the illustrative head restraint mechanism 300 includes a swivel arm 318 operatively coupling the tilt guide 314 and the support plate assembly 600. The swivel arm 318 includes a rotatable connector element, such as a swivel arm bore 320, at which the swivel arm is pivotably connected to the support plate assembly 600. The swivel arm 318 is pivotable about a swivel axis 322 that is substantially perpendicular to the upper surface of the support plate assembly 600 (and the upper surface of the table 12) to provide the range of motion of lateral flexion of the patient's neck 30 as shown by arrow 32. In this illustrative embodiment, the swivel arm bore 320 is disposed proximate one end of the swivel arm 318, and an opposite end of the swivel arm 318 comprises a swivel lock assembly 324 configured to selectively secure the swivel arm 318 in a desired angular position.

The swivel lock assembly 324 includes a lock element, such as an extendable tooth 326, which is operatively connected to a swivel adjustment knob 328 for selective engagement between the swivel arm 318 and a corresponding component of the support plate assembly 600. In this illustrative embodiment, the swivel lock assembly 324 is disposed on a flange 330 that extends downwardly proximate the edge of the support plate assembly 600 so that the swivel lock assembly 324 engages the edge of the support plate assembly 600.

Referring to FIGS. 9-11, in this illustrative embodiment, the tilt guide 314 is selectively repositionable on the swivel arm 318. For example, the tilt guide 314 is longitudinally slidable and selectively securable with respect to a long axis of the swivel arm 318. The tilt guide 314 includes one or more tilt guide adjustment slots 332, 334 which receive threaded fasteners extending therethrough from the swivel arm 318. Respective tilt guide adjustment knobs 336, 338 are operatively connected to the threaded fasteners to selectively secure the tilt guide 314 in a desired longitudinal position relative to the swivel arm 318.

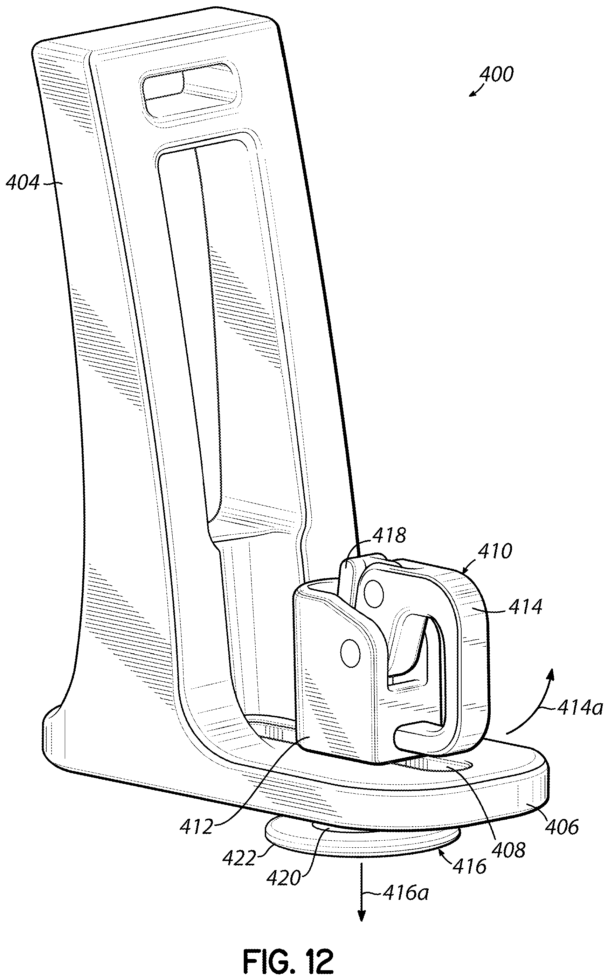

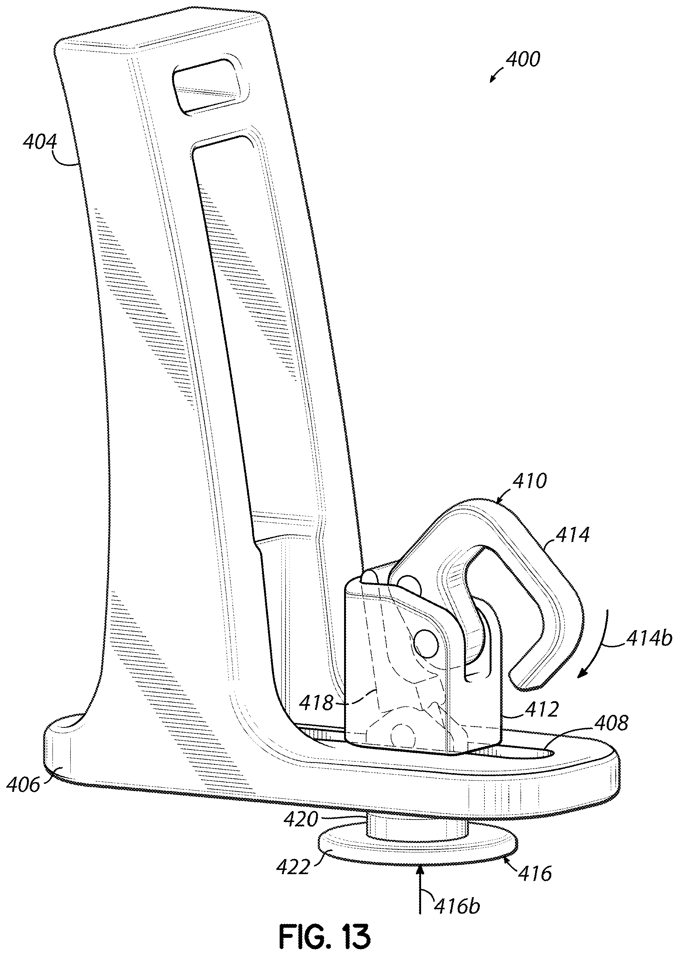

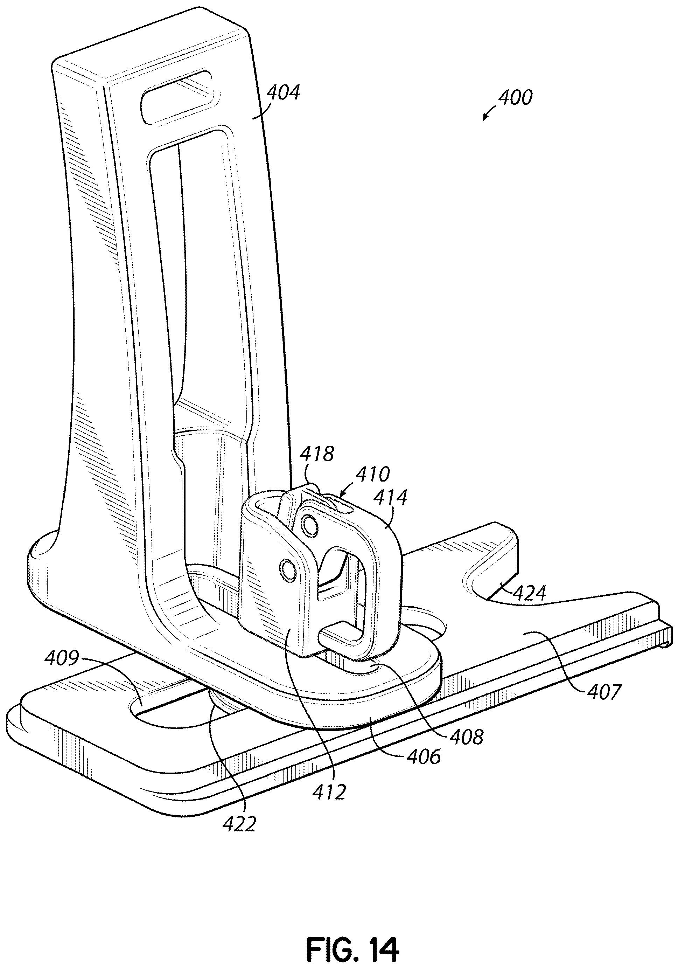

FIG. 12 is an isometric view of an illustrative shoulder restraint 400 in a locked configuration, FIG. 13 is an isometric partial cutaway view of the shoulder restraint 400 in an unlocked configuration, and FIG. 14 is an isometric view of the shoulder restraint in a locked configuration, all according to at least some aspects of the present disclosure. The following description focuses on the shoulder restraint 400; however, it also generally applies to the substantially similar shoulder restraint 402.

Referring to FIGS. 1, 2, and 12-14, the shoulder restraints 400, 402, in connection with the chest elevator 500, are generally configured to facilitate positioning and/or stabilizing the patient's upper torso 24 in connection with stabilizing the patient's head 14. The illustrative shoulder restraint 400 comprises a generally upstanding shoulder restraint portion 404 configured to engage the patient's shoulder 20, such as on the upper surface of the patient's shoulder 20. The shoulder restraint portion 404 comprises a base 406, which is selectively slidably coupled to a shoulder restraint connection element 407, which is attached to the support plate assembly 600 as described below. In this illustrative embodiment, the base 406 includes a longitudinal slot 408 and the connection element 407 includes a lateral slot 409, both of which slidably receive a latch assembly 410 therethrough. The connection element 407 includes a support plate retention element, such as a surface 424.

The latch assembly 410 is configured to selectively secure the shoulder restraint 400 with respect to the connection element 407 and the patient support plate assembly 600. The latch assembly 410 comprises a housing 412, a handle 414 pivotably disposed with respect to the housing 412, a locking element 416, and a linkage 418 pivotably connected to each of the handle 414 and the locking element 416. The housing 412 and the handle 414 are disposed above the base 406. The linkage 418 and/or the locking element 416 extend downward through the housing to below the base 406 and the connection element 407. The locking element 416 includes a boss 420 and a flange 422. The boss 420 extends downward through the lateral slot 409 of the connection element 407, and the flange 422 extends wider than the lateral slot 409 beneath the slot 409.

The illustrative latch assembly 410 is operated by pivoting the handle 414 with respect to the housing 412 between the locked configuration (FIGS. 12 and 14) and the unlocked configuration (FIG. 13). Pivoting the handle 414 from the locked configuration to the unlocked configuration (arrow 414a) causes the linkage 418 to move generally downward, which moves the locking element 416 downward (arrow 416a). This downward movement increases the distance between the flange 422 and the housing 412. In the unlocked configuration, linkage 418 is slidable within the longitudinal slot 408 and the boss 420 is slidable within the lateral slot 409. Accordingly, in the unlocked configuration, the shoulder restraint portion 404 is longitudinally and laterally repositionable with respect to the patient support plate assembly 600 and the table 12.

Similarly, pivoting the handle 414 from the unlocked configuration (FIG. 13) to the locked configuration (FIG. 14) (arrow 414b) causes the linkage 418 to move generally upward, which moves the locking element 416 upward (arrow 416b). This upward movement reduces the distance between the flange 422 and the housing 412. In the locked configuration, the base 406 and the connection element 407 are secured (e.g., compressed) between the housing 412 and the flange 422, thereby securing the shoulder restraint portion 404 with respect to the patient support assembly 600 and the table 12. The handle 414, linkage 418, and housing 412 may be configured such that the handle 414 is in an over-center condition in the locked configuration, which may prevent inadvertent movement of the latch assembly 410 from the locked configuration to the unlocked configuration.

Some exemplary embodiments may include padded patient contact surfaces. For example, referring to FIG. 2, the shoulder restraint 402 is shown with a shoulder pad 426. The shoulder pad 426 may be permanently or releasably secured to the shoulder restraint 402. For example, the pad 426 may be attached using hook and loop fasteners, screws, clamps, clips, and/or adhesives. In some exemplary embodiments, the pad 426 may be disposable, and in other embodiments the pad 426 may be reusable (e.g., readily cleanable between patients). It will be appreciated that other patient contact surfaces of the head stabilization system 100, such as the shoulder restraint 400, may include similar pads.

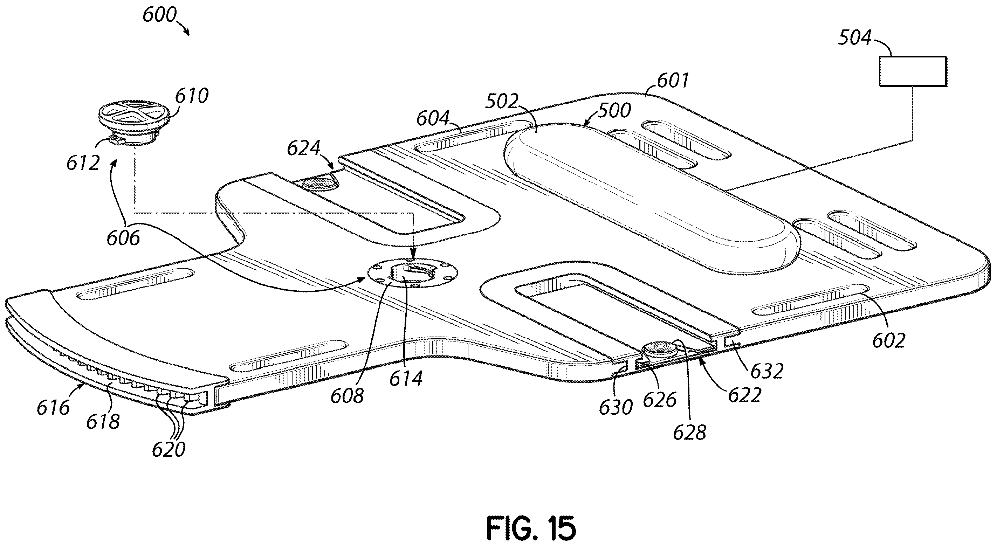

FIG. 15 is an isometric view of an illustrative patient support plate assembly 600, according to at least some aspects of the present disclosure. Referring to FIGS. 1, 2, and 15, the patient support plate assembly 600 is generally configured to secure the head stabilization system 100 to the table 12 and to support the various other components of the head stabilization system 100. The illustrative patient support plate assembly 600 comprises a generally planar, generally rigid support plate 601, to which other components of the support plate assembly 600 and the head stabilization system 100 are mounted. The support plate assembly 600 includes one or more attachment elements, such as support plate fastening slots 602, 604, which are configured to secure the support plate assembly 600 to the patient support device (e.g., the angiography table 12). For example, the support plate fastening slots 602, 604 may engage the support plate attachment elements (e.g., straps 26 and/or clamps 28) securing the patient support plate assembly 600 to the table 12. Other attachment elements on the support plate assembly 600 may be used in connection with other types of support plate attachment elements.

The illustrative support plate assembly 600 includes a head restraint mechanism connector 606, which is configured to releasably and pivotably couple the head restraint mechanism 300 to the support plate assembly 600. The head restraint mechanism connector 606 includes a connector base 608 affixed to the support plate 601 and a detachable connector cap 610. In this illustrative embodiment, the connector cap 610 is configured to extend through the swivel arm bore 320 (FIGS. 9-11) of the head restraint mechanism 300 and to releasably couple with the connector base 608. For example, the connector cap 610 may include one or more radially extending tabs 612, which may be configured to engaged corresponding slots 614 in the connector base 608. Rotation of the connector cap 610 by about a quarter turn (e.g., clockwise) may be operative to secure the connector cap 610 to the connector base 608, thereby securing the head restraint mechanism 300 to the support plate assembly 600. Similarly, rotation of the connector cap 610 by about a quarter turn in the opposite direction (e.g., counterclockwise) may be operative to release the connector cap 610 from the connector base 608, thereby releasing the head restraint mechanism 300. In the illustrative embodiment, the head restraint mechanism 300 is held generally against the upper surface of the support plate assembly 600 by the connector cap 610, but the head restraint mechanism 300 remains pivotable about the head restraint mechanism connector 606 to provide lateral flexion of the patient's neck 30 as shown in FIG. 3. One or more of the interfacing surfaces of the head restraint mechanism connector 606 and/or the head restraint mechanism 300 may include a bearing element, such as a lubricious material (e.g., a low-friction polytetrafluoroethylene or polyoxymethylene plastic) or a rolling element bearing, for ease of adjustment of the head restraint mechanism 300, for example.

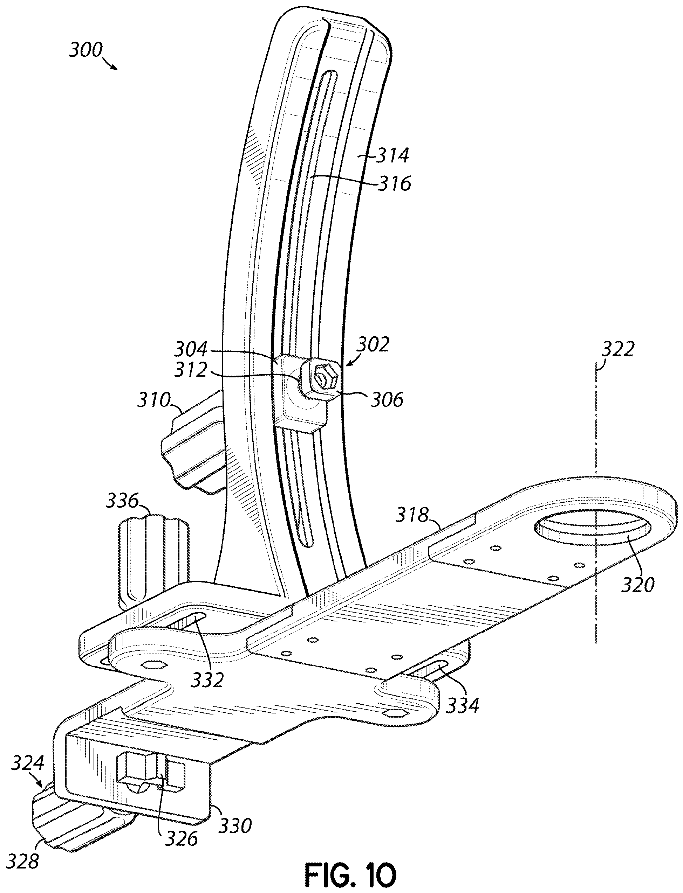

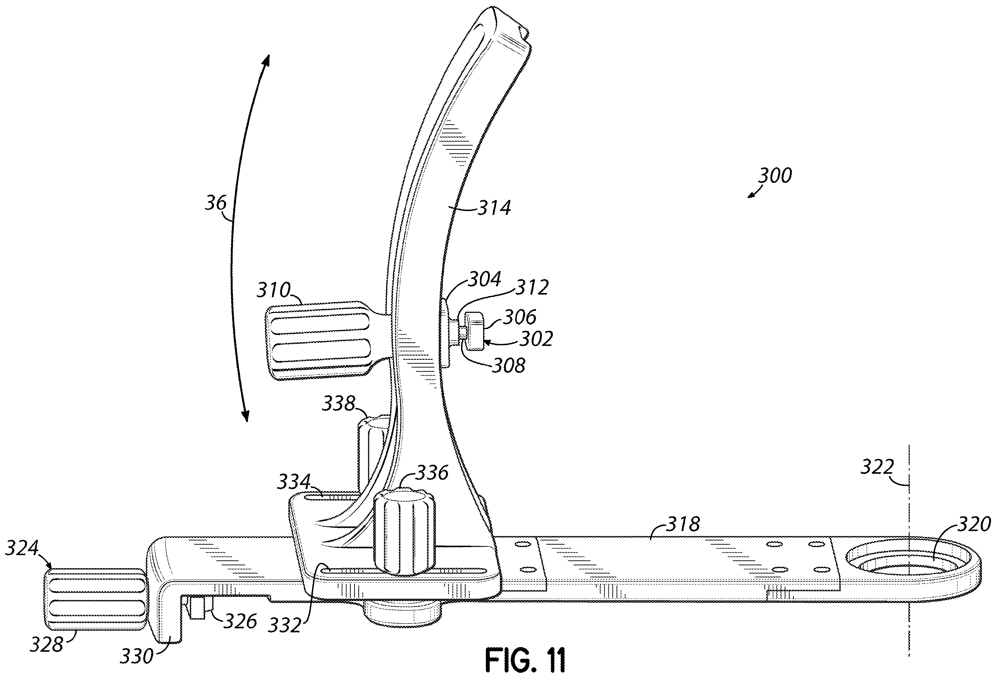

The illustrative support plate assembly 600 includes a swivel lock engagement element 616 configured to operatively engage the swivel lock assembly 324 (FIGS. 9-11). In this illustrative embodiment, the swivel lock engagement element 616 comprises an edge of the support plate assembly 600 that includes a slot 618 shaped substantially as a radius with respect to the head restraint mechanism connector 606. The slot 618 includes a plurality of teeth 620 configured to releasably engage the extendable tooth 326 of the swivel lock assembly 324 (FIGS. 10 and 11) to selectively fix the lateral flexion of the patient's head. One or more of the interfacing surfaces of the swivel lock engagement element 616 and/or the head restraint mechanism 300 may include a bearing element, such as a lubricious material (e.g., a low-friction polytetrafluoroethylene or polyoxymethylene plastic), for ease of adjustment of the head restraint mechanism 300, for example.

The illustrative support plate assembly 600 includes shoulder restraint connection elements 622, 624 for mounting the shoulder restraints 400, 402 to the support plate 601. The following description focuses on the shoulder restraint connection element 622 associated with the shoulder restraint 400; however, it also generally applies to the substantially similar shoulder restraint connection element 624 associated with the shoulder restraint 402.

Referring to FIGS. 12-15, the illustrative shoulder restraint connection element 622 includes a laterally oriented slot 626 configured to slidably receive the shoulder restraint's 400 connection element 407 therein. The shoulder restraint connection element 622 includes a retention element, such as an upwardly extending boss 628. The boss 628 is configured to releasably engage the support plate retention element (e.g., surface 424) of the shoulder restraint 400 to secure the shoulder restraint 400 onto the support plate assembly 600.

In this illustrative embodiment, the shoulder restraint connection element 622 is formed of an elastomeric material and is releasably connectable to the support plate 601. For example, the shoulder restraint connection element 622 is formed with a peripheral groove 630 which receives a corresponding flange 632 of the support plate 601 therein.

Referring to FIGS. 1, 2, 5, 6, and 15, the chest elevator 500, which is configured to facilitate vertical positioning of the patient's upper torso 24, is disposed on the support plate 601. The chest elevator 500 may be utilized to lift the patient's upper torso 24, such as to improve access to the C1-C2 puncture site 48. The illustrative chest elevator 500 comprises an inflatable membrane 502 positioned between the support plate 601 and the patient's upper torso 24 (e.g., on top of the support plate 601 and beneath the patient's upper torso 24). Inflating the inflatable membrane 502 generally raises the patient's upper torso 24 with respect to the support plate 601 and deflating the inflatable membrane 502 generally lowers the patient's upper torso 24 towards the support plate 601. The inflatable membrane 502 is operatively coupled to a pressurized air source 504, such as by appropriate tubing, valves, and connectors. In some exemplary embodiments, the pressurized air source 504 may comprise an installed pressurized air supply system of a medical facility. In other exemplary embodiments, the pressurized air source 504 may comprise a portable air source (e.g., portable compressor or portable compressed air tank).

Various components of exemplary head stabilization systems 100 according to at least some aspects of the present disclosure may be constructed from materials that generally do not interfere (or minimally interfere) with various imaging modalities. For example, all or nearly all of the components comprising an illustrative head stabilization system 100 may be constructed from suitable materials that are non-metallic and substantially do not interfere with the imaging of the patient's cranial anatomy. For example, various known polymers and/or composites may be utilized for components of head stabilization systems 100.

Exemplary methods of using an illustrative head stabilization system 100 according to at least some aspects of the present disclosure are described below and may include optional and/or alternative structures and/or operations. The head stabilization system 100 may be prepared for use. Referring to FIG. 1, the support plate assembly 600 is attached to the patient support device (e.g., angiography table 12). For example, one or more support plate attachment elements (e.g., straps 26 and/or clamps 28) is installed to secure the support plate assembly to the table 12. The head restraint mechanism 300, shoulder restraints 400, 402, and chest elevator 500 are installed, if they are not already in place. Referring to FIG. 2, one or more pads (e.g., shoulder pad 426) may be installed. Referring to FIG. 15, the chest elevator 500 is lowered, such as by deflating the inflatable membrane 502.

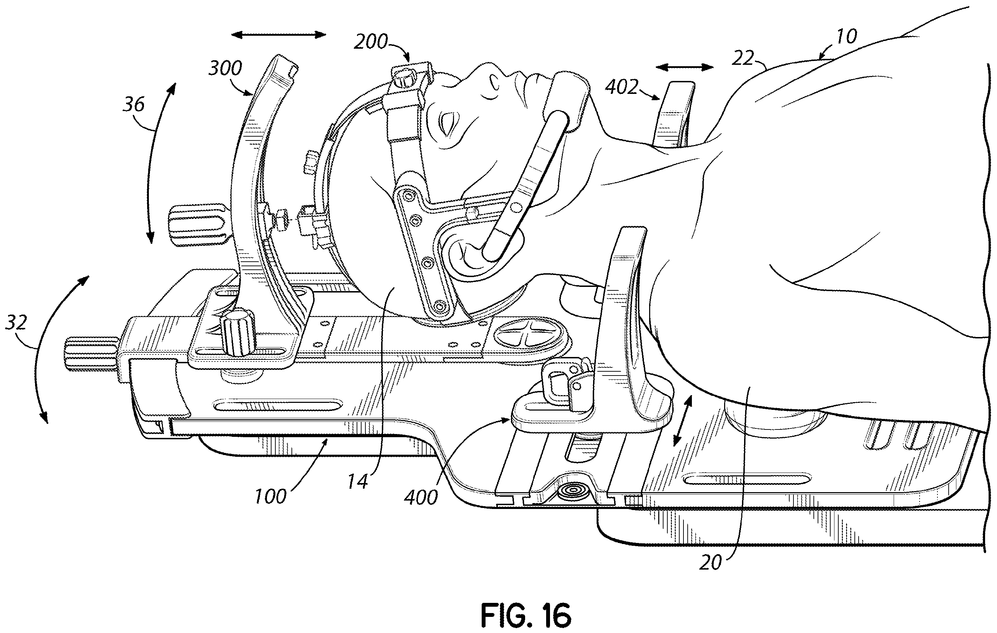

FIG. 16 is an isometric view of the illustrative head stabilization system 100 with the head harness 200 disconnected from the head restraint mechanism 300, according to at least some aspects of the present disclosure. Referring to FIGS. 1, 7, 8, and 16, the patient's head 14 may be secured in the head harness 200 while the head harness 200 is disconnected from the head restraint mechanism 300. In some circumstances, the head harness 200 may be secured onto the patient's head 14 before the patient 10 is placed on the patient support plate 600 and/or the table 12. Alternatively, the patient 10 may be placed on the patient support plate 600 and/or the table 12 before the head harness 200 is secured on the patient's head 14. In some circumstances, the patient's head may be secured in the head harness 200 while the head harness 200 is attached to the head restraint mechanism 300.

Referring to FIGS. 7 and 8, the head harness 200 is applied and fitted to the patient's head 14. The halo portion 210 and the rear support portion 238 are positioned to substantially circumscribe the patient's head 14. The halo adjustment knob 218 is utilized to facilitate fitting and securing the halo portion 210 and the rear support portion 238. The head restraint strap portion 242 is fitted and secured using the head restraint strap adjustment knob 246. The chin strap portion 202 is positioned on the chin/jaw region 38 of the patient's head 14 and, if not already attached, the chin strap portions 234 of the halo stabilizers 224, 226 are connected to the halo strap portions 232.

Referring to FIGS. 1 and 16, the patient is placed on the patient support plate 600 and the table 12. For example, the patient 10 may be positioned in a generally supine position (e.g., generally lying horizontally on the back with the face and front of the torso facing up). The shoulder restraints 400, 402 may be adjusted laterally and/or longitudinally as necessary, such as to engage the patient's shoulders 20, 22 and/or flex the patient's shoulders 20, 22 generally towards the patient's feet. For example, the latch assemblies 410 of the shoulder restraints 400, 402 may be unlocked and/or locked as necessary, leaving them in the locked configuration when adjustment is complete.

Referring to FIGS. 1, 7-11, and 16, the head restraint mechanism 300 may be repositioned as necessary to engage the harness connector housing 248 of the head harness 200 with the harness connector assembly 302 of the head restraint mechanism 300 (if they are not already connected). For example, the harness connector assembly 302 may be loosened (e.g., the harness connector knob 310 may be loosed) to allow adjustment of the harness connector assembly 302 along the tilt guide slot 316. In some exemplary embodiments, the harness connector knob 310 may remain loose while the harness connector housing 248 is engaged with the harness connector assembly. The tilt guide 314 may be loosened with respect to the swivel arm (e.g., the tilt guide adjustment knobs 336, 338 may be loosened) to allow longitudinal adjustment of the tilt guide 314 with respect to the swivel arm 318. The lock element of the swivel lock assembly 324 may be loosened (e.g., the swivel adjustment knob 328 may be loosened) to allow the swivel arm 318 to pivot about the head restraint mechanism connector 606.

After the harness connector housing 248 of the head harness 200 is coupled to the harness connector assembly 302 of the head restraint mechanism 300, the head stabilization system 100 may be adjusted to position and orient the patient's head 14 as desired. For example, the head stabilization system 100 may be adjusted (e.g., various components may be loosened, repositioned, and/or tightened) to facilitate any of the movements described above with respect to FIGS. 3-6 and/or to stabilize the patient's head 14 in a desired position and/or orientation.

Referring to FIGS. 9-11, the tilt guide 314 may be positioned longitudinally as desired with respect to the swivel arm 318. The tilt guide adjustment knobs 336, 338 may be tightened to secure the tilt guide 314 with respect to the swivel arm 318. Referring to FIGS. 3, 9-11, and 15, the lateral flexion of the patient's neck 30 may be adjusted, and the lock element of the swivel lock assembly 324 may be tightened (e.g., the swivel adjustment knob 328 may be tightened) to secure the swivel arm 318 at the desired angular orientation about the head restraint mechanism connector 606.

Referring to FIGS. 4 and 7-11, the harness connector assembly 302 may be loosened (e.g., the harness connector knob 310 may be loosed) to allow lateral rotational positioning of the patient's head 14. That is, the head harness 200 may be rotated about the harness connector shaft 308. When the desired orientation is achieved, the harness connector knob 310 may be tightened to engage the toothed surfaces 254, 312, thereby securing the lateral rotational positioning of the patient's head 14. Referring to FIGS. 5, 6, and 9-11, the harness connector assembly 302 may be loosened (e.g., the harness connector knob 310 may be loosed) to allow adjustment of the extension or compression of the patient's neck 30. That is, the harness connector assembly 302 may be repositioned along the tilt guide slot 316. When the desired position is achieved, the harness connector knob 310 may be tightened to secure the harness connector assembly 302 with respect to the tilt guide slot 316. Some exemplary methods may involve loosening the harness connector assembly 302, adjusting both the lateral rotational positioning of the patient's head 14 and the extension or compression of the patient's neck 30, and tightening the harness connector assembly.

Referring to FIGS. 1 and 15, the chest elevator 500 may be operated (e.g., inflatable membrane 502 may be at least partially inflated) to raise the patient's upper torso 24 with respect to the support plate assembly 600.

An illustrative method of manufacturing a head stabilization system 100 may include installing a head restraint mechanism 300, such as installing the head restraint mechanism 300 onto a patient support plate assembly 600. The method may include installing at least one shoulder restraint 400, 402, such as installing the at least one shoulder restraint 400,402 onto the patient support plate assembly 600. The method may include installing a chest elevator 500, such as installing the chest elevator 500 onto the patient support plate assembly 600.

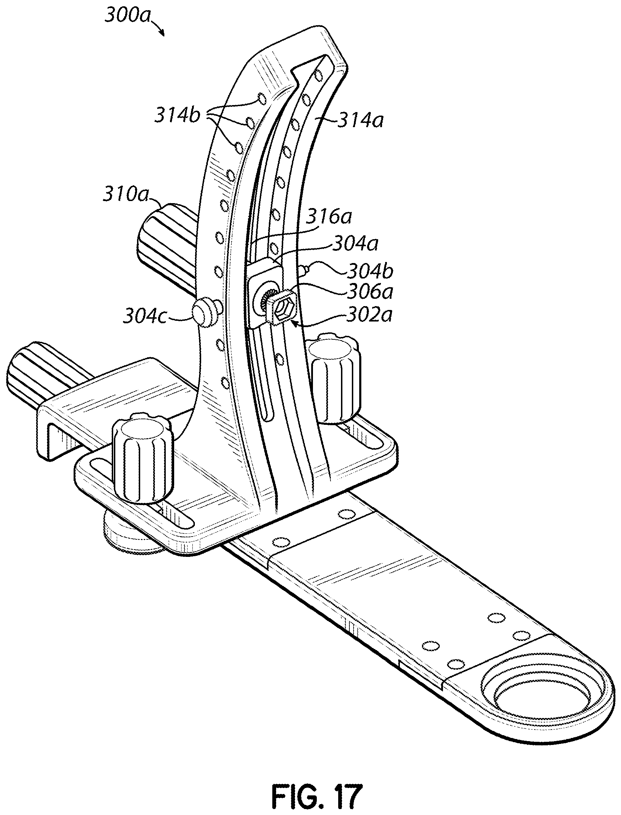

FIG. 17 is an isometric view of an alternative illustrative head restraint mechanism 300a, according to at least some aspects of the present disclosure. Generally, the head restraint mechanism 300a is similar in construction and operation to the head restraint mechanism 300 described above and the head restraint mechanism 300a may be substituted for other head restraint mechanisms, or any feature of the head restraint mechanism 300a may be used, in various other exemplary embodiments according to the present disclosure. Like reference numbers refer to like components. For brevity, the following description omits redundant description and focuses on the differences between the head restraint mechanism 300a and the head restraint mechanism 300.

The illustrative head restraint mechanism 300a is configured to provide three-dimensional positioning of the patient's head. The head restraint mechanism 300a includes a selectively securable harness connector assembly 302a. The illustrative harness connector assembly 302a includes a harness connector body 304a and an opposed harness connector stop 306a disposed on a harness connector shaft. A harness connector knob 310a is disposed on the harness connector shaft and is operative to axially draw together the harness connector knob 310a and the harness connector stop 306a, such as by a threaded engagement between the harness connector knob 310a and the harness connector shaft.

The head restraint mechanism 300a includes a generally vertically upstanding, arcuate tilt guide 314a having a generally vertically oriented tilt guide slot 316a therethrough. The harness connector shaft of the harness connector assembly 302a extends through the tilt guide slot 316a, between the harness connector knob 310a and the harness connector body 304a. The harness connector assembly 302a is slidable generally vertically upward and downward along the length of the tilt guide slot 316a. With the stop 306a of the harness connector assembly 302a within the cavity 250 of harness connector housing 248 of the head harness 200, tightening the harness connector knob 310a is operative to secure the head harness 200 with respect to the tilt guide 314a.

In this illustrative head restraint mechanism 300a, the tilt guide 314a includes a plurality of laterally extending through holes 314b. The holes 314b are positioned generally along the length of the tilt guide slot 316a in locations where it is desired to secure the harness connector assembly 302a. The holes 314b are configured to receive laterally therethrough a locking pin 304b. The pin comprises a head 304c, which may have a diameter greater than the diameter of the holes 314b. The harness connector body 304a of the harness connector assembly 302a includes a corresponding laterally extending through hole, which may be configured to receive the pin 304b therethrough. When it is desired to lock the harness connector assembly 302a in a particular position along the tilt guide slot 316a, the hole of the harness connector body 304a is aligned with one of the holes 314b of the tilt guide 314a. Then, the pin 304b is inserted through the hole of the harness connector body 304a and the selected hole 314b of the tilt guide 314a. With the pin 304b in place, the harness connector assembly 302a is secured in the selected position along the tilt guide slot 316a. The pin 304b may be used to secure the harness connector assembly 302a in addition to and/or in place of tightening the harness connector knob 310a as described above.

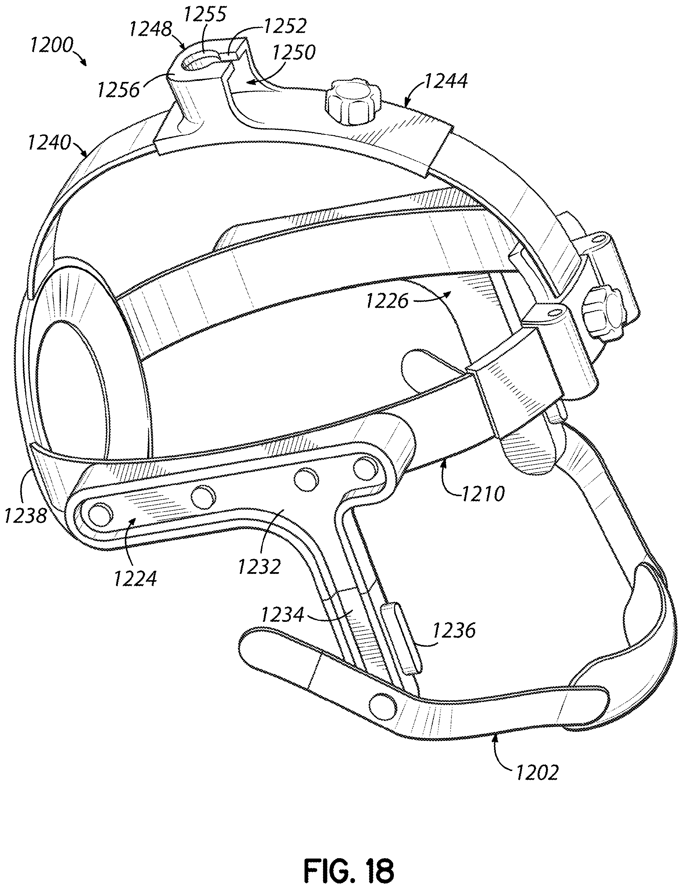

FIG. 18 is an isometric view of an alternative illustrative head harness 1200, according to at least some aspects of the present disclosure. Generally, the head harness 1200 is similar in construction and operation to the head harness 200 described above and the head harness 1200 may be substituted for other head harnesses, or any feature of the head harness 1200 may be used, in various other exemplary embodiments according to the present disclosure. Like reference numbers refer to like components. For brevity, the following description omits redundant description and focuses on the differences between the head harness 1200 and the head harness 200.

The illustrative head harness 1200 includes a chin strap portion 1202 and a halo portion 1210. The halo portion 1210 is connected to the chin strap portion 1202 by a right halo stabilizer 1224 and a left halo stabilizer 1226. The halo stabilizers 1224, 1226 are substantially similar, and for brevity only the right halo stabilizer 1224 is described in detail. The right halo stabilizer 1224 includes two releasably connected portions: the halo strap portion 1232 and the chin strap portion 1234. The halo strap portion 1232 and the chin strap portion 1234 are secured together by a releasable catch operated by an actuator, such as a release button 1236. When actuated by a user, the release button 1236 releases the chin strap portion 1234 from the halo strap portion 1232. In some embodiments, the button 1236 may serve as an emergency release to allow the patient to be quickly disconnected from the head stabilization system 100. The left halo stabilizer 1226 is constructed in a substantially similar manner and includes a similar button providing a chin strap emergency release.

The illustrative head harness 1200 includes a head restraint portion 1240 that extends centrally over the top of the patient's head from front to back. The head restraint portion 1240 includes a head restraint element 1244. The illustrative head harness 1200 includes a connecting element, such as a harness connector housing 1248, which is configured to releasably couple the head harness 1200 to the head restraint mechanism. The harness connector housing 1248 is provided on the restraint element 1244 and includes a cavity 1250 and a slot 1252 terminating in a generally circular opening 1255, which are configured to releasably engage corresponding components of the head restraint mechanism, as described below. The slot 1252 and the opening 1255 extend through a wall 1256, which at least partially defines the cavity 1250.

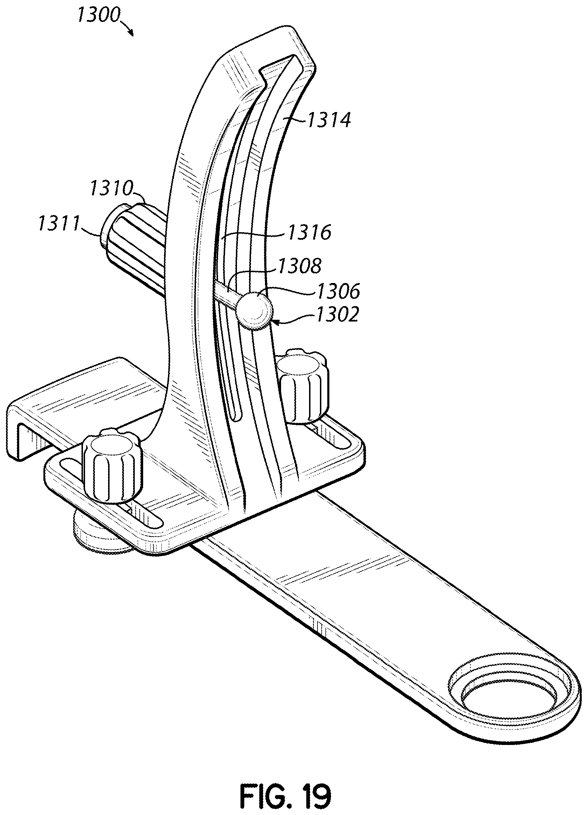

FIG. 19 is an isometric view of an alternative illustrative head restraint mechanism 1300, according to at least some aspects of the present disclosure. Generally, the head restraint mechanism 1300 is similar in construction and operation to the head restraint mechanisms 300, 300a described above and the head restraint mechanism 1300 may be substituted for other head restraint mechanisms, or any feature of the head restraint mechanism 1300 may be used, in various other exemplary embodiments according to the present disclosure. Like reference numbers refer to like components. For brevity, the following description omits redundant description and focuses on the differences between the head restraint mechanism 1300 and the head restraint mechanisms 300, 300a.

The illustrative head restraint mechanism 1300 is configured to provide three-dimensional positioning of the patient's head. The head restraint mechanism 1300 includes a selectively securable harness connector assembly 1302. The illustrative harness connector assembly 1302 includes a harness connector knob 1310 and an opposed harness connector stop 1306 disposed on a harness connector shaft 1308. The harness connector knob 1310 is operative to axially draw together the harness connector knob 1310 and the harness connector stop 1306, such as by a threaded engagement between the harness connector knob 1310 and the harness connector shaft 1308. The head restraint mechanism 1300 includes a generally vertically upstanding, arcuate tilt guide 1314 having a generally vertically oriented tilt guide slot 1316 therethrough. The harness connector shaft 1308 extends through the tilt guide slot 1316, between the harness connector knob 1310 and the harness connector stop 1306. The harness connector assembly 1302 is slidable generally vertically upward and downward along the length of the tilt guide slot 1316.

In this illustrative embodiment, the harness connector stop 1306 is in the form of a generally spherical ball configured to be received within the cavity 1250 of the harness connector housing 1248 shown in FIG. 18. The harness connector shaft 1308 is configured to be received through the slot 1252 and the opening 1255. With the stop 1306 of the harness connector assembly 1302 within the cavity 1250 of harness connector housing 1248 of the head harness 1200, tightening the harness connector knob 1310 is operative to secure the head harness 1200 with respect to the tilt guide 1314. For example, the stop 1306 may be drawn partially into and/or may seat against the opening 1255.

In some exemplary embodiments, the harness connector knob 1310 may include a release button 1311, which, when depressed generally axially along the harness connector shaft 1308, may be configured to facilitate rapid detachment of the head harness 1200 from the head restraint mechanism 1300.

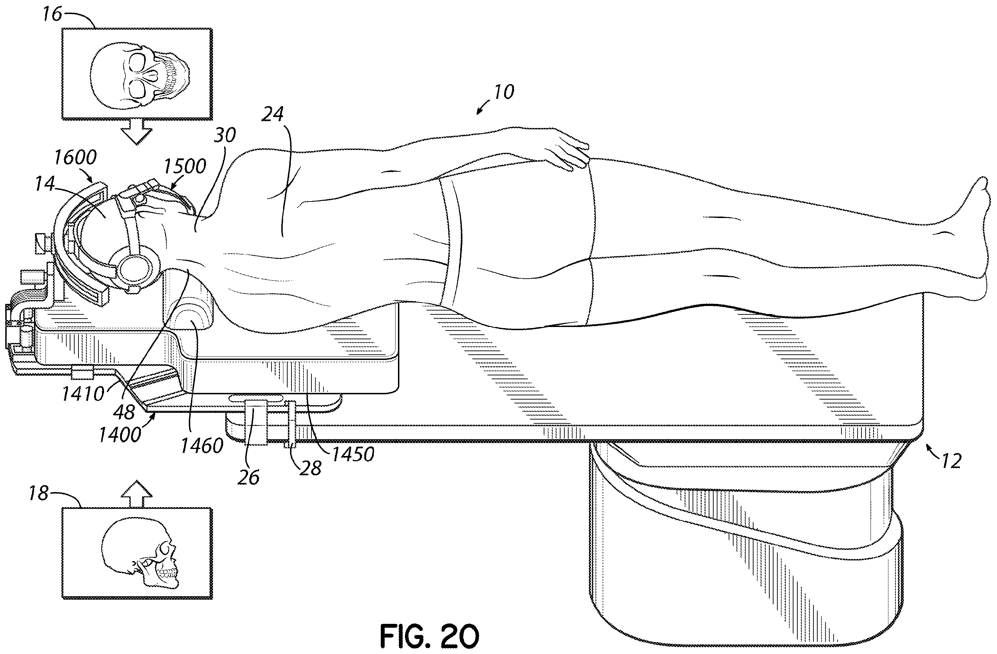

FIG. 20 is an isometric view of an alternate illustrative head stabilization system 1400 in use on a patient 10, wherein the patient 10 is positioned in a generally lateral position, according to at least some aspects of the present disclosure. While FIG. 20 illustrates patient 10 positioned on their left side, patient 10 may be positioned on either their left or right side. Generally, the head stabilization system 1400 is similar in construction and operation to the head stabilization system 100 described above and the head stabilization system 1400 may be substituted for other head stabilization systems, or any feature of the head stabilization system 1400 may be used, in various other exemplary embodiments according to the present disclosure. Like reference numbers refer to like components.

The head stabilization system 1400 is disposed on a patient support device, such as generally at the head end of an angiography table 12. The head stabilization system 1400 is configured to facilitate various procedures, such as imaging of the patient's head 14 by one or more imaging devices 16, 18, which may be selectively positioned with respect to the table 12. Some illustrative embodiments may be used in connection with procedures involving accessing the cerebrospinal fluid space via a C1-C2 puncture site 48, for example.

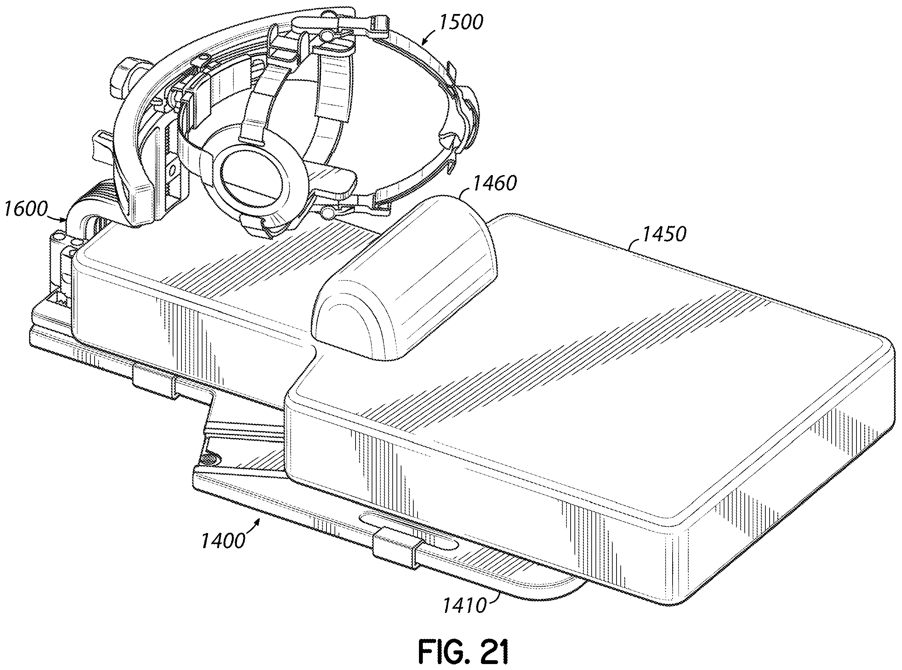

FIG. 21 is an isometric view of the illustrative head stabilization system 1400, according to at least some aspects of the present disclosure. Referring to FIGS. 20 and 21, the illustrative head stabilization system 1400 is generally configured to facilitate the desired positioning of the patient's head 14 and/or to support and stabilize the patient's head 14 (e.g., holding the patient's head 14 stationary), such as during a procedure.

The illustrative head stabilization system 1400 includes a head harness 1500 configured to engage the patient's head 14 and a head restraint mechanism 1600 to which the head harness 1500 is releasably attachable. The head restraint mechanism 1600 is configured to facilitate positioning and stabilization of the patient's head 14 with respect to the table 12 using the head harness 1500. The illustrative head stabilization system 1400 includes a torso pad 1450 configured to support and adjust the position of the patient's upper torso 24 and a neck pad 1460 configured to support and/or adjust the position of the patient's neck 30. The torso pad 1450 may be inflatable and/or adjustable to facilitate positioning the patient 10. The neck pad 1460 may be inflatable and/or adjustable to facilitate positioning of the patient's neck 30. The head restraint mechanism 1600, torso pad 1450, and neck pad 1460 are disposed on a support plate assembly 1410. The support plate assembly 1410 is secured to the table 12, such as by one or more support plate attachment elements, such as one or more straps 26 and/or clamps 28. Generally, the head stabilization system 1400 allows the patient's head 14 to be positioned and/or oriented within a three-dimensional space, relative to the imaging devices 16, 18 and the table 12 and stabilizes the patient's head 14 in the desired position.

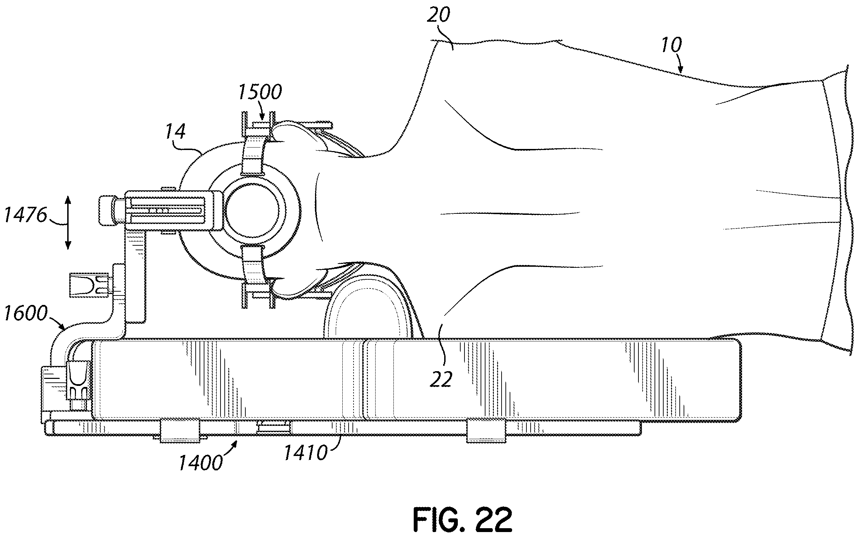

Referring to FIGS. 22-25, a summary overview of the potential positioning of the patient's head 14 facilitated by the head stabilization system 1400 is provided in order to give context for the descriptions below. Generally, the illustrative head stabilization system 1400 may be positioned and/or secured at any position and/or orientation within any combination of the ranges of motion described with reference to FIGS. 22-25 to position and/or stabilize the patient's head 14 as desired, such as for a procedure.

FIG. 22 is a lateral elevation view of the illustrative head stabilization system 1400 showing vertical positioning of the patient's head 14, according to at least some aspects of the present disclosure. Generally, this articulation of the head restraint mechanism 1600 provides vertical positioning of the patient's head 14, up or down, as shown by arrow 1476, in a plane generally perpendicular to the upper surface of the support plate assembly 1410. This vertical flexion motion is similar to that which would occur if the patient tilted his or her head to the right or left to touch the ear to the shoulder 20, 22 on the same side, without rotating the head 14. This illustrative head stabilization system 1400 facilitates about 30° of vertical flexion below center and 30° of vertical flexion above center, for a total range of lateral flexion of about 60°, from farthest down to farthest up.

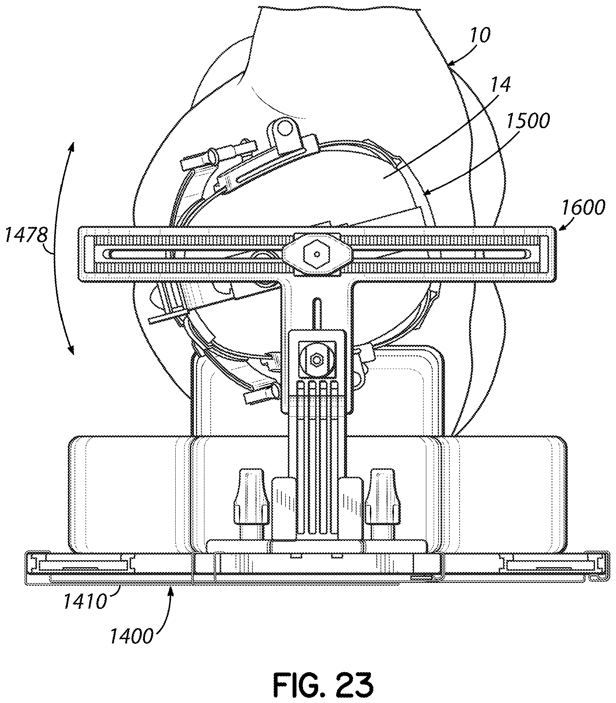

FIG. 23 is a cranial elevation view of the illustrative head stabilization system 1400 showing rotational positioning of the patent's head 14, according to at least some aspects of the present disclosure. Generally, this articulation of the head harness 1500 relative to the head restraint mechanism 1600 provides rotation of the patient's head 14, to the patient's right or to the patient's left, as shown by arrow 1478, in a plane generally perpendicular to the upper surface of the support plate assembly 1410. This motion is generally similar to that which would occur if the patient attempted to rotate his or her head 14 side to side as if to say "no". This illustrative head stabilization system 1400 facilitates about 60° of right rotation and about 60° of left rotation, for a total range of rotation of about 120°

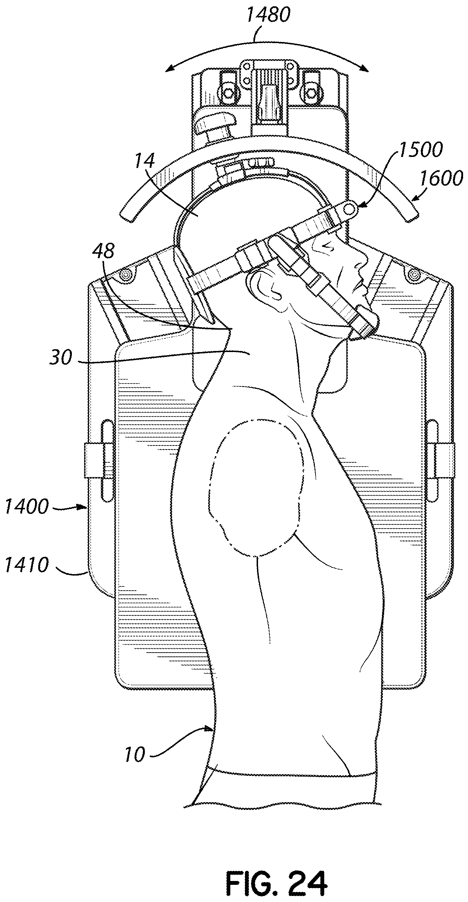

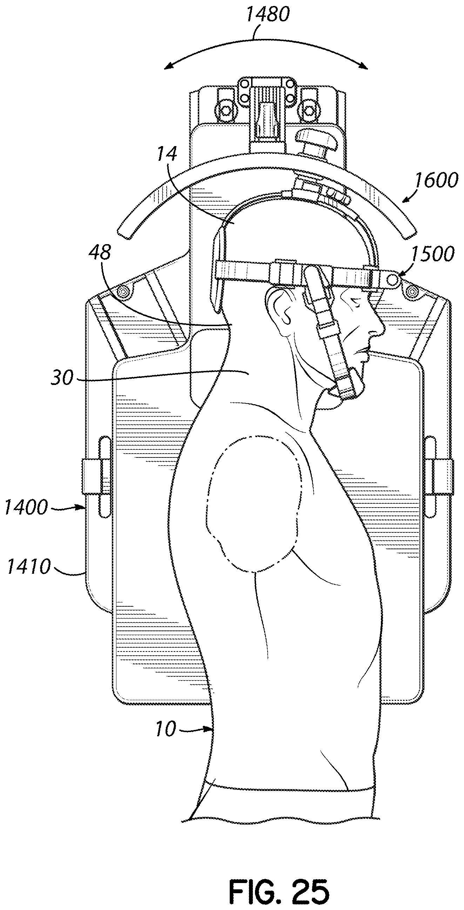

FIGS. 24 and 25 are plan views of the illustrative head stabilization system 1400 showing extension and flexion, respectively, of the patient's neck 30, according to at least some aspects of the present disclosure. Generally, this articulation of the head restraint mechanism 1600 provides extension and flexion of the patient's neck 30, backward (i.e., extension) and forward (i.e., flexion), as shown by arrow 1480, in a plane generally transverse to the upper surface of the support plate assembly 1410. These motions are generally similar to those which would occur if the patient attempted to nod his or her head 14 up and down as if to say "yes". This illustrative head stabilization system 1400 facilitates about 25° of neck extension backward (from neutral) and about 25° of neck flexion forward (from neutral), for a total range of extension and flexion of about 50°. The position shown in FIG. 25 may improve access to the cerebrospinal fluid space via the C1-C2 puncture site 48, facilitating lateral, posterior needle introduction. The position shown in FIG. 24 may improve access to the anterior anatomy of the brain. In some circumstances, it may be advantageous to apply traction to the patient's neck 30. For example, the head restraint mechanism 1600 (via the head harness 1500) may apply a force on the patient's head 14 in a generally superior/cranial direction.

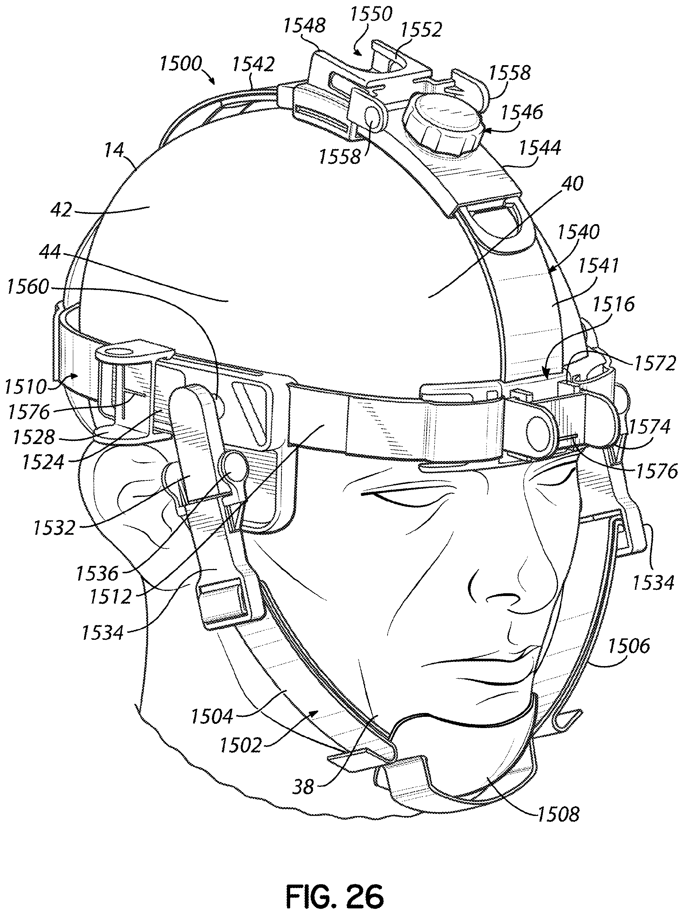

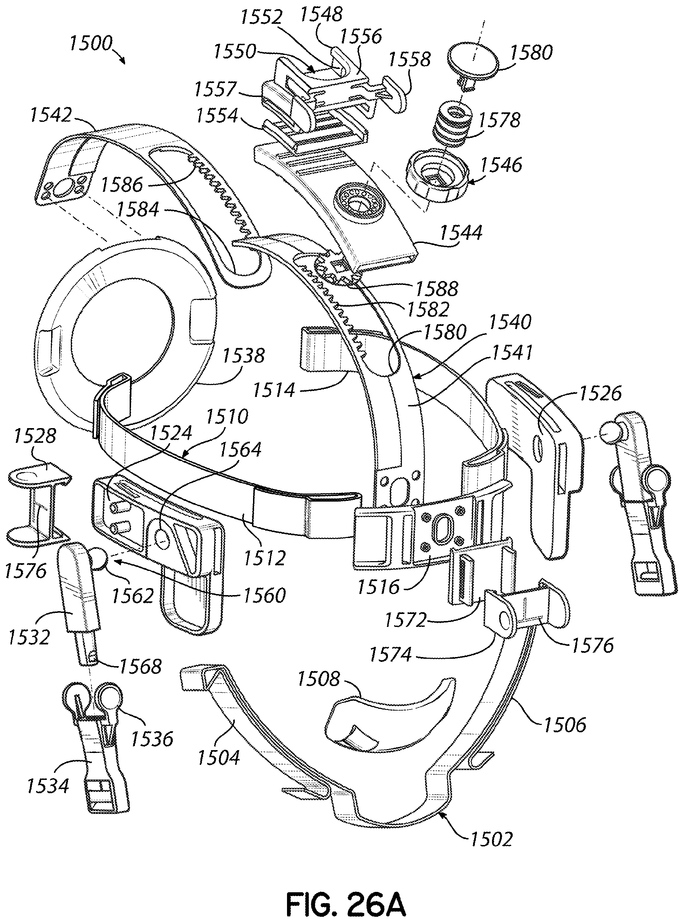

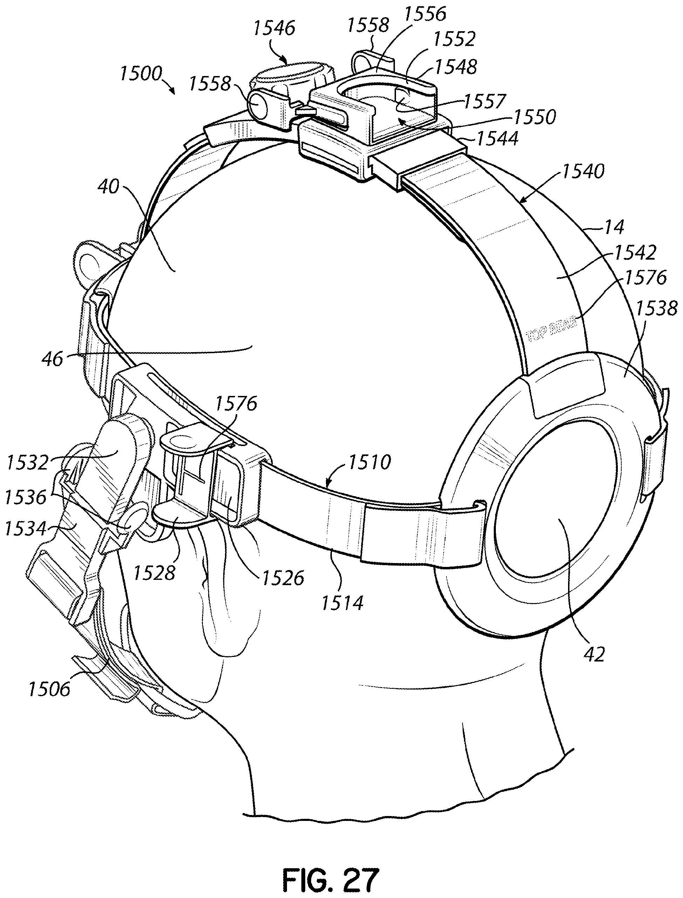

FIGS. 26, 26A, and 27 are isometric views of an alternate illustrative head harness 1500 disposed on a patient's head 14, according to at least some aspects of the present disclosure. Generally, the head harness 1500 is similar in construction and operation to the head harnesses 200, 1200 described above and the head harness 1500 may be substituted for other head harnesses, or any feature of the head harness 1500 may be used, in various other exemplary embodiments according to the present disclosure. Like reference numbers refer to like components.

The head harness 1500 is configured to be secured to the patient's head 14 and to be coupled (e.g., releasably) to the head restraint mechanism 1600 (FIG. 21). Generally, the illustrative head harness 1500 is configured to engage the patient's head 14 at a chin/jaw region 38, a forehead region 40, and rear region 42. In some exemplary embodiments, the head harness 1500 may be configured to securely engage the patient's head in a non-traumatic, non-penetrating manner (e.g., no component of the head harness 1500 may penetrate the patient's skin).

The illustrative head harness 1500 includes a chin strap portion 1502 configured to engage the patient's chin/jaw region 38. The chin strap portion 1502 facilitates control of the position of the patient's chin relative to the patient's neck 30. The chin strap portion 1502 includes a right chin strap portion 1504 and a left chin strap portion 1506 that extend forward and downward generally along the patient's mandible to hold a chin pad 1508 against the patient's chin/jaw region 38. The chin strap portions 1504, 1506 may be adjustable, such as by adjustable buckle type connectors 1534.

The illustrative head harness 1500 includes one or more chin strap end connectors 1532. The right chin strap portion 1504 is connected to the chin strap end connector 1532 by an adjustable buckle type connector 1534 with an integral releasable catch 1568. The chin strap end connector 1532 and the adjustable buckle type connector 1534 are secured together by a releasable catch 1568 operated by an actuator, such as one or more catch release tabs 1536. When actuated by a user, the catch release tabs 1536 release the adjustable buckle type connector 1534 from the chin strap end connector 1532. In some embodiments, the catch release tabs 1536 may serve as an emergency release to allow the patient 10 to be quickly disconnected from the head stabilization system 1400. For example, if the patient's airway becomes compromised, it may be desirable to quickly access the patient's mouth by detaching the chin strap portion 1502 (e.g., by actuating the catch release tabs 1536 as a chin strap emergency release). In some embodiments, the catch release tabs 1536 may be configured for one-hand operation. The left chin strap portion 1506 is constructed in a substantially similar manner providing for chin strap emergency release.

The illustrative head harness 1500 includes a halo portion 1510 configured to engage the patient's forehead 40 and the lateral side portions 44, 46 of the patient's head 14. The halo portion 1510 includes a right halo strap portion 1512 and a left halo strap portion 1514. The halo strap portions 1512, 1514 extend back-to-front along the patient's head 14. At the front, the halo strap portions 1512, 1514 are coupled to a halo adjustment mechanism 1516 configured to allow adjustment of the halo portion 1510 to securely fit an individual patient's head 14. The halo adjustment mechanism 1516 may include one or more tabs 1572 for an alignment clip1574. The alignment clip 1574 may include radiopaque markings 1576 which may be useful for imaging equipment calibration, for example.

In the illustrative head harness 1500, the halo portion 1510 is connected to the chin strap portion 1502 by a right halo stabilizer 1524 and a left halo stabilizer 1526. Each halo stabilizer 1524, 1526 is generally L-shaped, with the generally horizontal, upper portion affixed to the respective halo strap portion 1512, 1514 and the portion closer to the patient's forehead 40 connecting to the respective chin strap portion 1504, 1506. In this illustrative embodiment, the halo stabilizers 1524, 1526 are substantially rigid and each halo strap portion 1512, 1514 passes through and is secured to the respective stabilizer 1524, 1526, such as by one or more alignment clips 1528. The alignment clips 1528 may include radiopaque markings 1576 which may be useful for imaging equipment calibration, for example. The chin strap portions 1504, 1506 are pivotably attached to the halo stabilizers 1524, 1526, such as by ball joint connections 1560, which allow rotational adjustment to securely fit an individual patient's head 14. Each ball joint connection 1560 includes a generally spherical bearing stud 1562 attached to the chin strap end connector 1532 and generally spherical socket 1564 which is integral to the respective halo stabilizer 1524, 1526. Each chin strap portion 1504, 1506 is connected to an adjustable buckle type connector 1534 which is connected to a chin strap end connector 1532 which is connected to the respective stabilizer 1524, 1526.

The illustrative head harness 1500 includes a rear support portion 1538, which may be in the form of a generally annular ring that is configured to be positioned centrally on the rear region 42 of the patient's head 14. The rear support portion 1538 is coupled to each of the halo strap portions 1512, 1514, so that the patient's head 14 is substantially circumscribed by the left halo strap portion 1514, the halo adjustment mechanism 1516, the right halo strap portion 1512, and the rear support portion 1538. The rear support portion 1538 and the halo adjustment mechanism 1516 are also connected by a head restraint portion 1540 that extends centrally over the top of the patient's head 14 from front to back.

In this illustrative embodiment, the head restraint portion 1540 includes a front head restraint strap portion 1541, a rear head restraint strap portion 1542, and a head restraint element 1544 disposed thereon. The head restraint portion 1540 may include radiopaque markings 1576 which may be useful for imaging equipment calibration, for example. The head restraint portion 1540 may be configured to allow adjustment to securely fit an individual patient's head 14. In this illustrative embodiment, the head restraint element 1544 includes a head restraint strap adjustment knob assembly 1546 configured to allow adjustment of the head restraint strap portions 1541, 1542 to securely fit the patient's head 14. The front head restraint strap portion 1541 includes a longitudinal slot 1580 such that a row of teeth 1582 are aligned along one side of the slot 1580. The rear head restraint strap portion 1542 includes a longitudinal slot 1584 such that a row of teeth 1586 are aligned along one side of the slot 1584 such that the teeth 1586 are aligned opposite the teeth 1582 of the front strap portion 1541 when the strap portions 1541, 1542 are in their installed positions. The adjustment knob assembly 1546 includes a radially oriented toothed wheel 1588. The radially oriented toothed wheel 1588 engages the teeth 1582, 1586 of the strap portions 1541, 1542 such that when the adjustment knob assembly 1546 is rotated the strap portions 1541, 1542 will move in relation to each other so as to lengthen or shorten the length of the head restraint portion 1540 to securely fit the patient's head 14.

The illustrative head harness 1500 includes a connecting element, such as a harness connector housing 1548, which is configured to releasably couple the head harness 1500 to the head restraint mechanism 1600. In this illustrative embodiment, the harness connector housing 1548 is provided on the head restraint element 1544 and includes a cavity 1550 and a slot 1552 configured to releasably engage corresponding components of the head restraint mechanism 1600, as described below. The slot 1552 extends through a wall 1556, which at least partially defines the cavity 1550. Additionally, the harness connector housing 1548 includes a latch 1557 releasable by an actuator, such as one or more finger tabs 1558, which are operatively coupled to selectively secure the head harness 1500 (e.g., the harness connector housing 1548) to the head restraint mechanism 1600. The finger tabs 1558 may serve as an emergency release, allowing rapid disconnection of the head harness 1500 from the head restraint mechanism 1600 when desired. In some embodiments, the finger tabs 1558 may be configured for one-hand operation.

In this illustrative embodiment, the chin strap portions 1504, 1506, the halo strap portions 1512, 1514, and the head restraint strap portions 1541, 1542 are constructed from flexible but substantially non-elastic materials. The halo stabilizers 1524, 1526 and the rear support portion 1538 are constructed from generally rigid materials, such as rigid polymeric or composite materials. In some exemplary embodiments, padding may be provided on various patient contact surfaces of the head harness 1500.

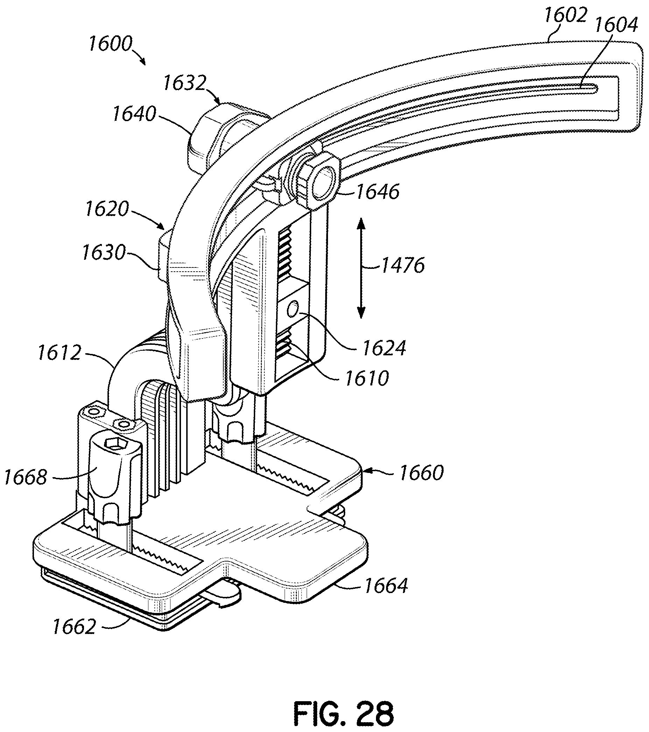

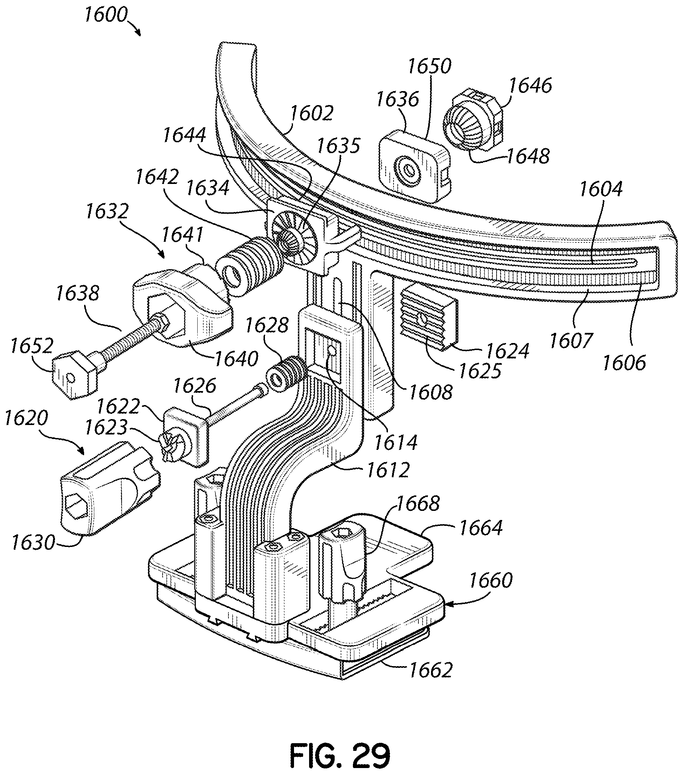

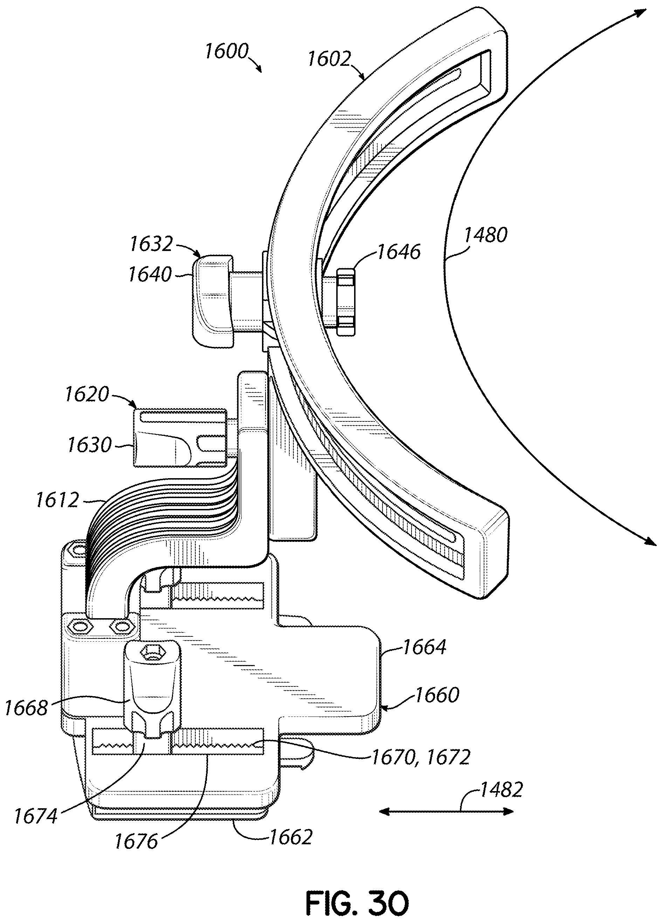

FIGS. 28-30 are isometric views of an alternate illustrative head restraint mechanism 1600, according to at least some aspects of the present disclosure. Generally, the head restraint mechanism 1600 is similar in construction and operation to the head restraint mechanisms 300, 300a, and 1300 described above and the head restraint mechanism 1600 may be substituted for other head restraint mechanisms, or any feature of the head restraint mechanism 1600 may be used, in various other exemplary embodiments according to the present disclosure. Like reference numbers refer to like components.

Generally, the head restraint mechanism 1600 is configured to provide three-dimensional positioning of the patient's head. In this illustrative embodiment the head restraint mechanism 1600 includes a tilt guide 1602 connected to a vertical support 1612 by a connector assembly 1620. The tilt guide 1602 is vertically adjustable on the vertical support 1612 in the direction shown by the arrow 1476 and the tilt guide 1602 may be secured to the vertical support 1612 in a plurality of positions. The illustrative connector assembly 1620 includes a connector knob retainer 1622 and an opposed connector stop 1624 disposed on a connector shaft 1626. A connector knob 1630 and compression spring 1628 is disposed on the connector shaft 1626 and is operative to axially draw together the connector knob 1630 the compression spring 1628 the connector knob retainer 1622 and the connector stop 1624, such as by a threaded engagement between the connector knob 1630 and the connector shaft 1626. The connector knob 1630 includes an axially oriented toothed surface (not shown) facing an axially oriented toothed surface 1623 on the connector knob retainer 1622. The toothed surfaces on the connector knob 1630 and the connector knob retainer 1622 may serve to secure the position of the connector knob 1630 in relation to the connector knob retainer 1622 when the connector assembly 1620 is draw together. The tilt guide 1602 includes an axially oriented toothed surface 1610 facing an axially oriented toothed surface 1625 on the connector stop 1624. The vertical support 1612 includes a through hole 1614 for the connector shaft 1626. The tilt guide 1602 includes a through slot 1608 for the connector shaft 1626. The tilt guide 1602 may be vertically positioned in a plurality of positions on the vertical support 1612 utilizing the engagement of the toothed surfaces 1610, 1625.

Referring to FIGS. 20-23 and 28-30, the illustrative head restraint mechanism 1600 includes a head restraint mechanism connector assembly 1660 operatively coupling the head restraint mechanism 1600 and the support plate assembly 1400. In this illustrative embodiment, the head restraint mechanism connector assembly 1660 allows the head restraint mechanism 1600 to be adjusted horizontally in a plane that is generally transvers to the upper surface of the support plate assembly 1400 (and the upper surface of the table 12) in the direction indicated by arrow 1482 and selectively secured at the desired position. In this illustrative embodiment, the head restraint mechanism connector assembly 1660 includes a head restraint connector base 1662 a vertical support mounting plate 1664 and one or more locking devices 1668. When installed the head restraint connector base 1662 is coupled to the support plate assembly 1400. The vertical support mounting plate 1664 is adjustable in relation to the head restraint connector base 1662 in the direction of arrow 1482. The locking devices 1668 are operative to draw the vertical support mounting plate 1664 and the head restraint connector base 1662 together. The head restraint mechanism connector assembly 1660 may include an incremental adjustment mechanism allowing the head restraint mechanism 1600 to be positioned in a plurality of positions relative to the support plate assembly 1410. In this illustrative embodiment, the head restraint connector base 1662 includes a plurality of parallel toothed surfaces 1670 and the vertical support mounting plate 1664 includes a matching opposed plurality of parallel toothed surfaces 1672. When the vertical support mounting plate 1664 is at the desired position on the head restraint connector base 1662 and the locking devices 1668 are operated the toothed surfaces 1670, 1672 are engaged securing the head restraint mechanism 1600 and preventing movement in the direction indicated by arrow 1482. Adjusting the vertical support mounting plate 1664 and thereby the head restraint mechanism 1600 in the direction of arrow 1482 may allow for the application of a force on the patient's head 14 in a generally superior/cranial direction. Accordingly, the patient's neck 30 may be placed in tension, generally longitudinally.

In this illustrative embodiment, the head restraint mechanism 1600 includes a selectively securable harness connector assembly 1632. The illustrative harness connector assembly 1632 includes a harness connector stop 1646 a harness connector body 1636 a connector knob retainer 1634 a harness connector spring 1642 a harness connector knob 1640 and a harness connector nut 1652 disposed on a harness connector shaft 1638. The harness connector knob 1640 is operative on the harness connector shaft 1638 to axially draw together the harness connector stop 1646 the harness connector body 1636 the connector knob retainer 1634 the harness connector spring 1642 the harness connector knob 1640 and the harness connector nut 1652, such as by a threaded engagement between the harness connector nut 1652 and the harness connector shaft 1638. The harness connector knob 1640 includes a radially oriented toothed surface 1641 facing a radially oriented toothed surface 1635 on the connector knob retainer 1634. The toothed surfaces 1635, 1641 may serve to secure the position of the connector knob 1640 in relation to the connector knob retainer 1634 when the connector assembly 1632 is draw together. The harness connector stop 1646 is rotatable about the harness connector shaft 1638 to provide the rotational range of motion indicated by arrow 1478 and as described above with reference to FIG. 23. The harness connector stop 1646 includes a generally half spherical and radially oriented toothed surface 1648 oriented to oppositely engage a generally half spherical cavity and radially toothed surface 1650 on the harness connector body 1636. Tightening the harness connector knob 1640 secures the harness connector stop 1646 with respect to the harness connector body 1636 and/or secures the relative angular positions of the toothed surfaces 1648, 1650. Loosening the harness connector knob 1640 is operative to allow movement of the harness connector stop 1646 relative to the harness connector body 1636 and allows relative angular movement between the tooth surfaces 1648, 1650.

Referring to FIGS. 24-30, the harness connector assembly 1632 of the head restraint mechanism 1600 is configured to releasably engage the tilt guide 1602 of the head restraint mechanism 1600. The tilt guide includes a cavity 1607 a tilt guide slot 1604 and a toothed surface 1606 axially oriented along the length of the tilt guide slot 1604. The connector knob retainer 1634 is sized and shaped to be received within the cavity 1607 with the tilt guide slot 1604 receiving the harness connector shaft 1638. The connector knob retainer 1634 includes an axially oriented toothed surface 1644 facing the axially oriented toothed surface 1606 on the tilt guide 1602. The axially toothed surface 1644 of the connector knob retainer 1634 may engage the corresponding toothed surface 1606 of the tilt guide 1602 in a plurality of positions along the tilt guide 1602. The toothed surfaces 1644, 1606 are engageable at a plurality of discrete relative angular positions across the range of extension and flexion as shown by arrow 1480 in FIGS. 24 and 25.

In this illustrative embodiment, the harness connector stop 1646 of the harness connector assembly 1632 has a plurality of equal length sides such that the harness connector body may engage within the cavity 1550 of the harness connector housing 1548 in a plurality of positions. With the harness connector stop 1646 of the harness connector assembly 1632 within the cavity 1550 of harness connector housing 1548, tightening the harness connector knob 1640 is operative to secure the head harness 1500 with respect to the tilt guide 1602. More specifically, tightening the harness connector knob 1640 secures the connector knob retainer 1634 with respect to the tilt guide slot 1604 and/or secures the relative angular positions of the toothed surfaces 1644, 1606. Loosening the harness connector knob 1640 is operative to allow movement of the harness connector assembly 1632 along the tilt guide slot 1604 and/or to allow relative angular movement between the tooth surfaces 1644, 1606.

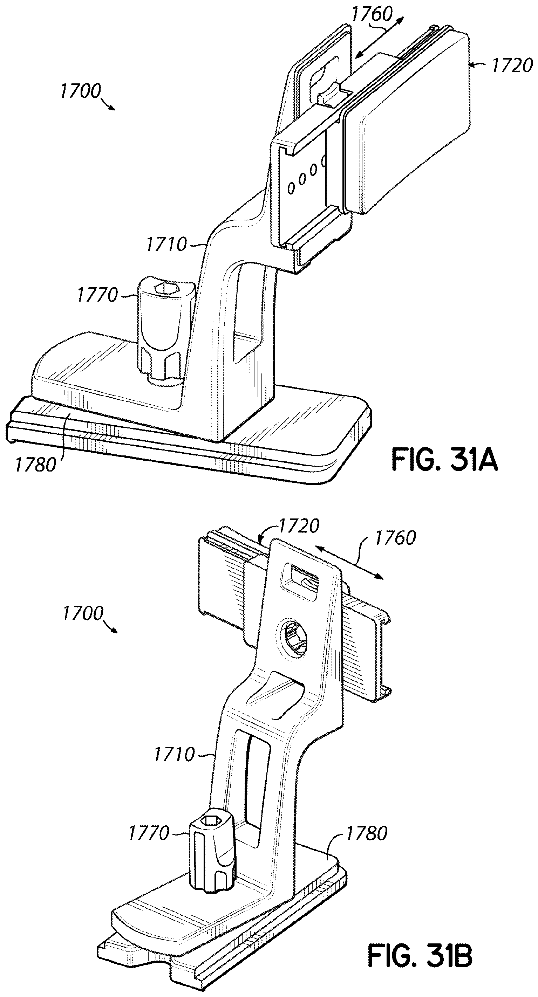

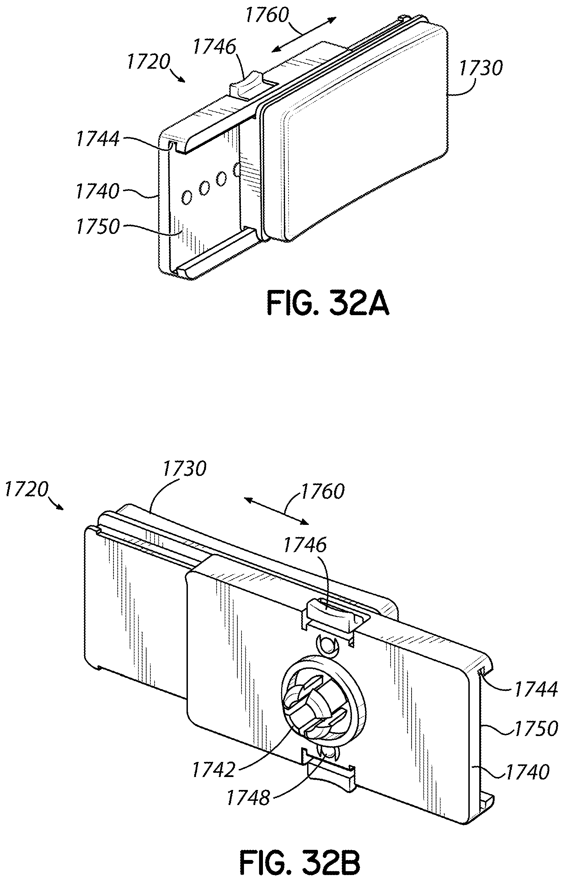

FIGS. 31A, 31B, 32A, 32B, and 33 are isometric views of an alternate illustrative shoulder restraint 1700 according to at least some aspects of the present disclosure. Generally, the shoulder restraint 1700 is similar in construction and operation to the shoulder restraints 400, 402 described above and the shoulder restraint 1700 may be substituted for other shoulder restraints, or any feature of the shoulder restraint 1700 may be used, in various other exemplary embodiments according to the present disclosure. Like reference numbers refer to like components.

Generally, the shoulder restraint 1700 is configured to position and secure the patient's shoulders. The illustrative shoulder restraint 1700 includes a shoulder restraint bracket 1710, a generally rectangular pad assembly 1720, a locking device 1770, and a shoulder restraint base 1780. The illustrative should restraint bracket 1710 includes a hole 1712 for mounting a pad assembly 1720, and a plurality of dimples 1714 generally arranged in opposing pairs radially around the hole 1712. The illustrative pad assembly 1720 includes a pad 1730 and mounting plate 1740. The illustrative mounting plate includes a plurality of integral mounting tabs 1742, pad grooves 1744, locking tabs 1746, locating pins 1748, and a cavity 1750. The mounting tabs 1742 are operative to secure the pad assembly 1720 to the shoulder restraint bracket 1710. The locating pins 1748 of the pad mounting plate 1740 engage a pair of the corresponding dimples 1714 of the shoulder restraint bracket 1710. The dimples 1714 and pins 1748 are engageable at a plurality of discrete relative angular positions. The engagement of the dimples 1714 and pins 1748 secure the angular position of the pad assembly 1720 on the shoulder restraint bracket 1710. The pad grooves 1744 are arranged opposite one another inside the cavity 1750 in such a way as to permit movement of the pad 1730 within the cavity 1750 in the direction shown by the arrow 1760. The locking tabs 1746 secure the position of the pad 1730 on the pad mounting plate 1740.

The locking device 1770 attaches the shoulder restraint bracket 1710 to the shoulder restraint base 1780. The shoulder restraint base 1780 attaches the shoulder restraint 1700 to a support plate assembly 600, for example. Turning the locking device 1770, clockwise for example, secures the shoulder restraint bracket 1710 to the shoulder restraint base 1780. Turning the locking device 1770, counterclockwise for example, allows the shoulder restraint bracket 1710 to be rotated in relation to the shoulder restraint base1780. Rotating the shoulder restraint bracket 1710 relative to the shoulder restraint base 1780 may allow for adjustment of the shoulder restraint 1700 to secure a patient's shoulder, for example.

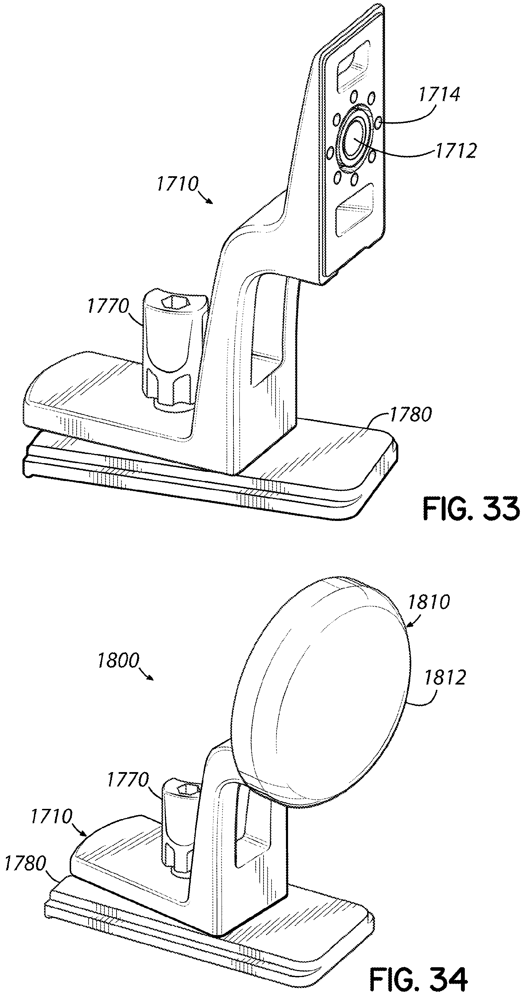

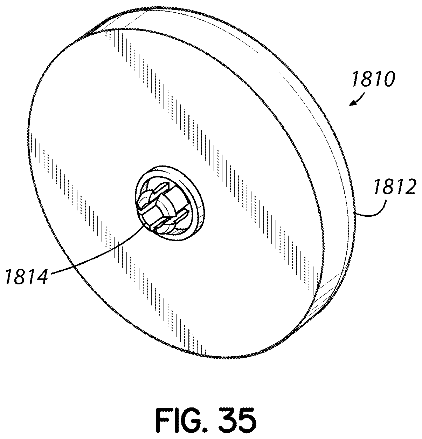

FIGS. 34 and 35 are isometric views of an alternate illustrative shoulder restraint 1800 according to at least some aspects of the present disclosure. Generally, the shoulder restraint 1800 is similar to the shoulder restraint 1700 with a generally round pad assembly 1810 replacing the generally rectangular pad assembly 1720. Generally, the shoulder restraint 1800 is similar in construction and operation to the shoulder restraints 400, 402, and 1700 described above and the shoulder restraint 1800 may be substituted for other shoulder restraints, or any feature of the shoulder restraint 1800 may be used, in various other exemplary embodiments according to the present disclosure. Like reference numbers refer to like components. The illustrative shoulder restraint 1800 includes a bracket 1710, a pad assembly 1810, a locking device 1770, and a shoulder restraint base 1780. The illustrative pad assembly 1810 includes a pad 1812, and a plurality of integral mounting tabs 1814. The mounting tabs 1814 are operative to secure the pad assembly 1810 to the bracket 1710.

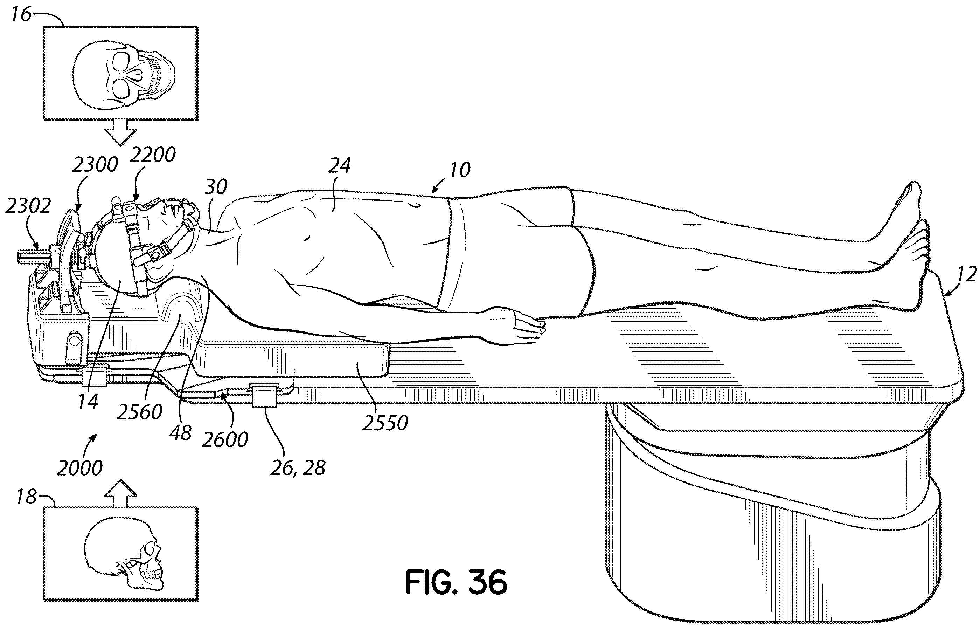

FIG. 36 is an isometric view of an alternate illustrative head stabilization system 2000 in use on a patient 10, according to at least some aspects of the present disclosure. Generally, the head stabilization system 2000 is similar in construction and operation to the head stabilization systems 100, 1400 described above and the head stabilization system 2000 may be substituted for other head stabilization systems, or any feature of the head stabilization system 2000 may be used, in various other exemplary embodiments according to the present disclosure. Like reference numbers refer to like components.

The head stabilization system 2000 is disposed on a patient support device, such as generally at the head end of an angiography table 12. The head stabilization system 2000 is configured to facilitate various procedures, such as imaging of the patient's head 14 by one or more imaging devices 16, 18, which may be selectively positioned with respect to the table 12. Some illustrative embodiments may be used in connection with procedures involving accessing the cerebrospinal fluid space via a C1-C2 puncture site 48, for example.

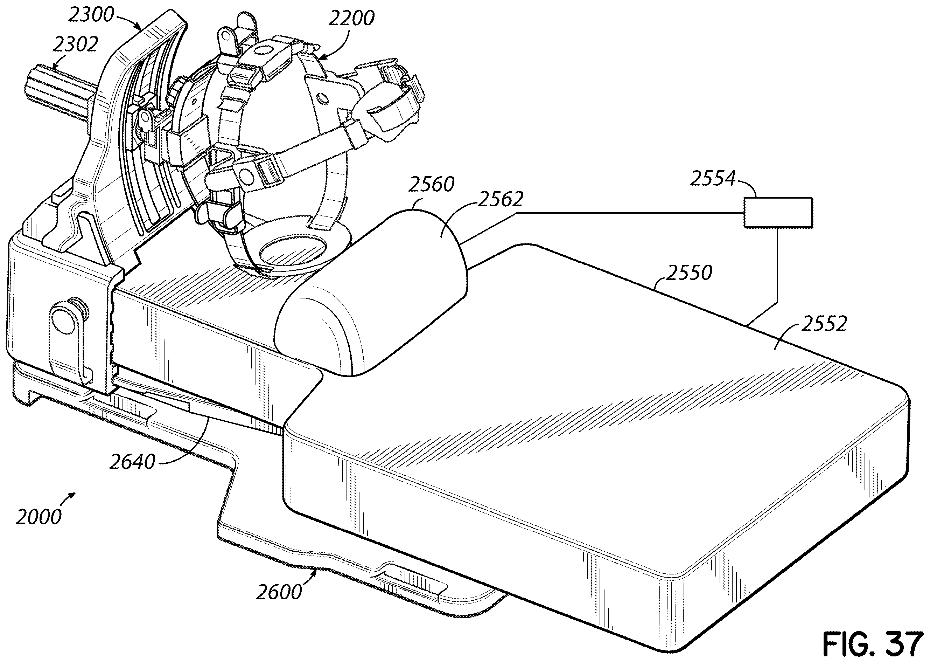

FIG. 37 is an isometric view of the illustrative head stabilization system 2000, according to at least some aspects of the present disclosure. Referring to FIGS. 36 and 37, the illustrative head stabilization system 2000 is generally configured to facilitate the desired positioning of the patient's head 14 and/or to support and stabilize the patient's head 14 (e.g., hold the patient's head 14 stationary), such as during a procedure. The illustrative head stabilization system 2000 includes a head harness 2200 configured to engage the patient's head 14 and a head restraint mechanism 2300 to which the head harness 2200 is releasably attachable. The head restraint mechanism 2300 is configured to facilitate positioning and stabilization of the patient's head 14 with respect to the table 12 using the head harness 2200. The illustrative head stabilization system 2000 includes a torso pad 2550 configured to adjust the vertical position of the patient's upper torso 24 and a neck pad 2560 configured to support and/or adjust the position of the patient's neck 30. The torso pad 2550 may be inflatable and/or adjustable to facilitate positioning the patient 10. The neck pad 2560 may be inflatable and/or adjustable to facilitate positioning of the patient's neck 30. The head restraint mechanism 2000, torso pad 2550, and neck pad 2560 are disposed on a support plate assembly 2600. The support plate assembly 2600 is secured to the table 12, such as by one or more support plate attachment elements, such as one or more straps 26 and/or clamps 28. Generally, the head stabilization system 2000 allows the patient's head 14 to be positioned and/or oriented within a three-dimensional space, relative to the imaging devices 16, 18 and the table 12 and stabilizes the patient's head 14 in the desired position.

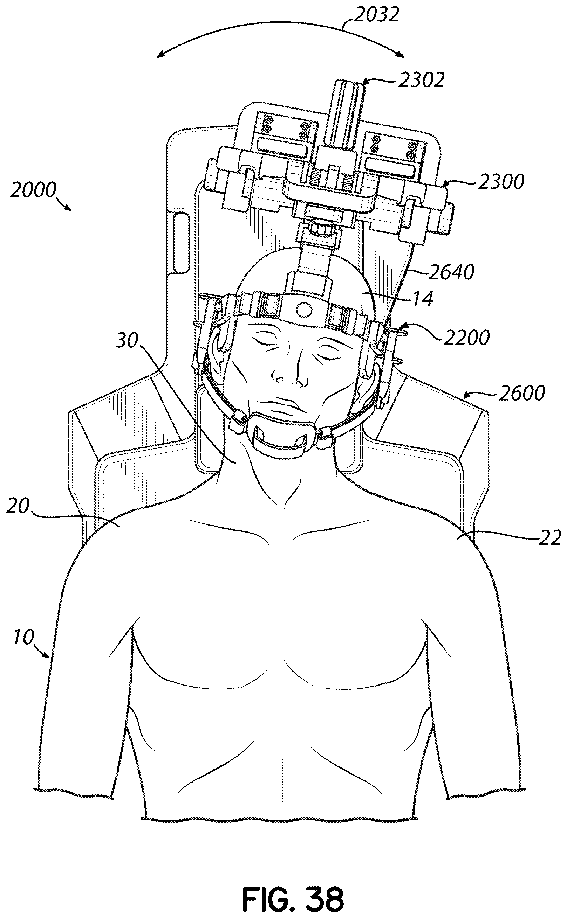

Referring to FIGS. 38-41, a summary overview of the potential positioning of the patient's head 14 facilitated by the head stabilization system 2000 is provided in order to give context for the descriptions below. Generally, the illustrative head stabilization system 2000 may be positioned and/or secured at any position and/or orientation within any combination of the ranges of motion described with reference to FIGS. 38-41 to position and/or stabilize the patient's head 14 as desired, such as for a procedure.

FIG. 38 is a plan view of the illustrative head stabilization system 2000 showing lateral positioning of the patient's head 14, according to at least some aspects of the present disclosure. Generally, this articulation of the head restraint mechanism 2300 provides lateral flexion of the patient's neck 30, left or right, as shown by arrow 2032, in a plane generally parallel to the upper surface of the support plate assembly 2600. This lateral flexion motion is similar to that which would occur if the patient tilted his or her head to the right or left to touch the ear to the shoulder 20, 22 on the same side, without rotating the head 14. This illustrative head stabilization system 2000 facilitates about 30° of lateral flexion left of center and 30° of lateral flexion right of center, for a total range of lateral flexion of about 60°, from farthest left to farthest right.

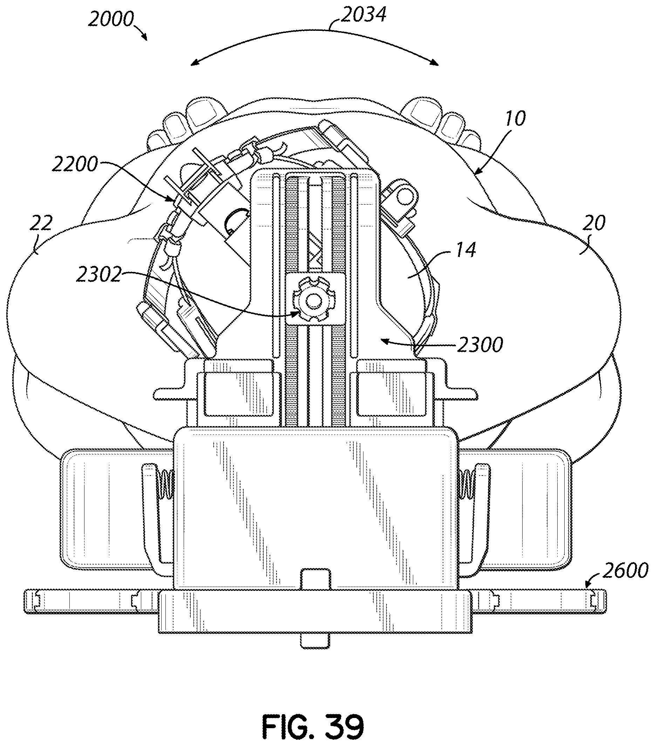

FIG. 39 is a cranial elevation view of the illustrative head stabilization system 2000 showing rotational positioning of the patent's head 14, according to at least some aspects of the present disclosure. Generally, this articulation of the head harness 2200 relative to the head restraint mechanism 2300 provides lateral rotation of the patient's head 14, left or right, as shown by arrow 2034, in a plane generally transverse to the upper surface of the support plate assembly 2600. As used herein, "transverse" may refer to relative angular orientations that are non-parallel (e.g., perpendicular or oblique). This motion is generally similar to that which would occur if the patient attempted to shake his or her head 14 side to side as to say "no". This illustrative head stabilization system 2000 facilitates about 60° of lateral rotation to the left and about 60° right, for a total range of lateral rotation of about 120°.

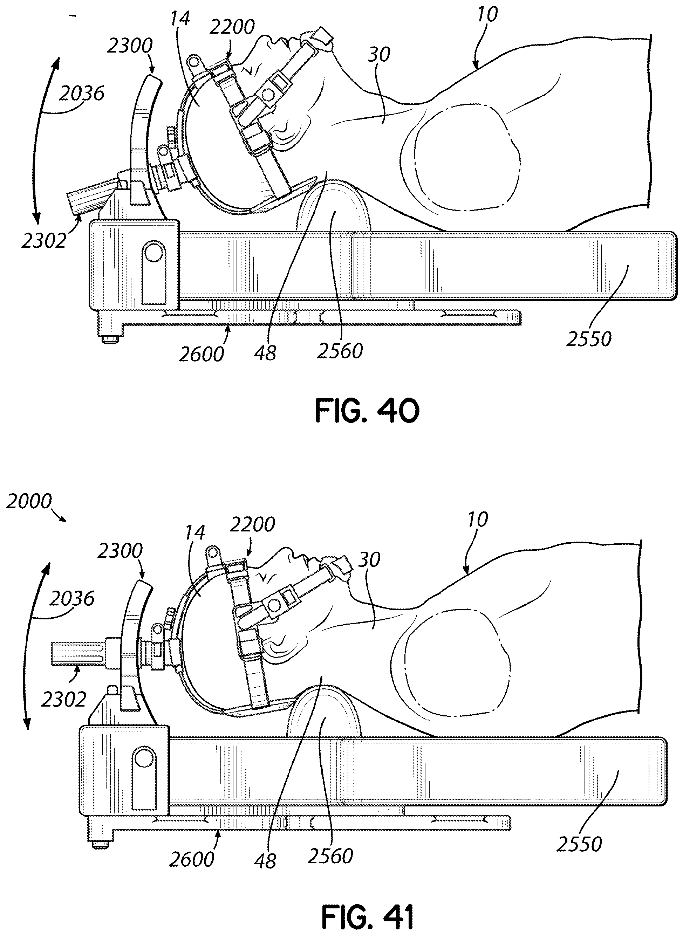

FIGS. 40 and 41 are lateral elevation views of the illustrative head stabilization system 2000 showing extension and compression, respectively, of the patient's neck 30, according to at least some aspects of the present disclosure. Generally, this articulation of the head restraint mechanism 2300 provides extension and flexion of the patient's neck 30, backward (i.e., extension) and forward (i.e., flexion), as shown by arrow 2036, in a plane generally perpendicular to the upper surface of the support plate assembly 2600. These motions are generally similar to those which would occur if the patient attempted to nod his or her head 14 up and down as to say "yes". This illustrative head stabilization system 2000 facilitates about 25° of neck extension upward (from neutral) and about 25° of neck flexion downward (from neutral), for a total range of extension and flexion of about 50°. The position shown in FIG. 40 may improve access to the cerebrospinal fluid space via the C1-C2 puncture site 48, facilitating lateral, posterior needle introduction. The position shown in FIG. 41 may improve access to the anterior anatomy of the brain. In some circumstances, it may be advantageous to apply traction to the patient's neck 30. For example, the head restraint mechanism 2300 (via the head harness 2200) may apply a force on the patient's head 14 in a generally superior/cranial direction. Accordingly, the patient's neck 30 may be placed in tension, generally longitudinally.

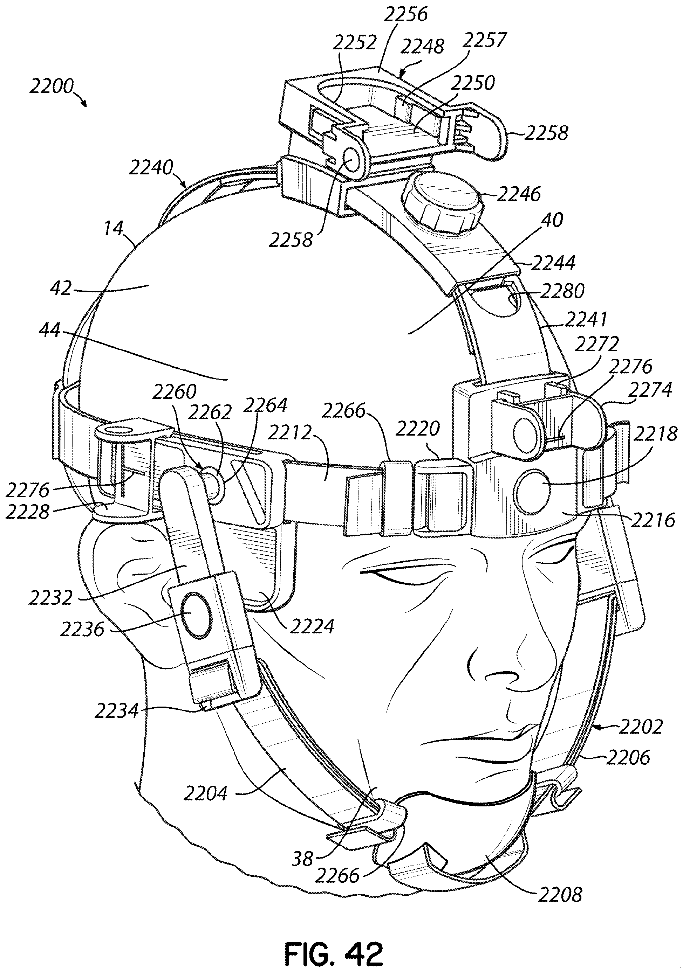

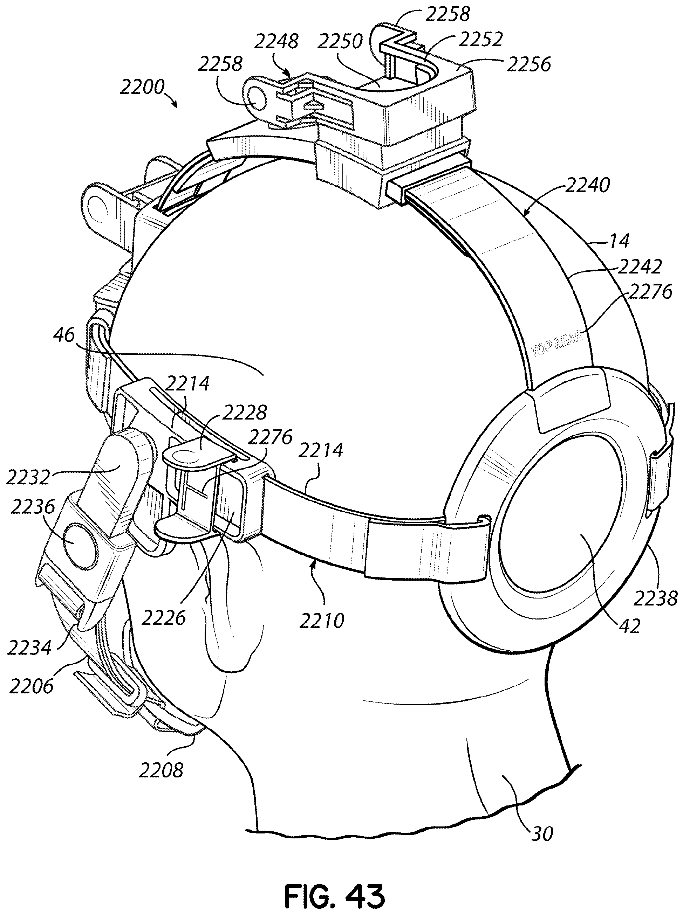

FIGS. 42 and 43 are isometric views of an illustrative head harness 2200 disposed on a patient's head 14, according to at least some aspects of the present disclosure. Generally, the head harness 2200 is similar in construction and operation to the head harnesses 200, 1200, and 1500 described above and the head harness 2200 may be substituted for other head harnesses, or any feature of the head harness 2200 may be used, in various other exemplary embodiments according to the present disclosure. Like reference numbers refer to like components.

The head harness 2200 is configured to be secured to the patient's head 14 and to be coupled (e.g., releasably) to the head restraint mechanism 2300 (FIGS. 36, 37). Generally, the illustrative head harness 2200 is configured to engage the patient's head 14 at a chin/jaw region 38, a forehead region 40, and rear region 42. In some exemplary embodiments, the head harness 2200 may be configured to securely engage the patient's head in a non-traumatic, non-penetrating manner (e.g., no component of the head harness 2200 may penetrate the patient's skin).

The illustrative head harness 2200 includes a chin strap portion 2202 configured to engage the patient's chin/jaw region 38. The chin strap portion 2202 facilitates control of the position of the patient's chin relative to the patient's neck 30. The chin strap portion 2202 includes a right chin strap portion 2204 and a left chin strap portion 2206 that extend forward and downward generally along the patient's mandible to hold a chin pad 2208 against the patient's chin/jaw region 38. The chin strap portions 2204, 2206 may be adjustable, such as by buckle type connectors 2234. The chin strap portions 2204, 2206 may include one or more strap retainers 2266 to secure the loose ends of the chin strap portions 2204, 2206.

The illustrative head harness 2200 includes one or more chin strap end connectors 2232. The right chin strap portion 2204 is connected to the chin strap end connector 2232 by a buckle type connector 2234 with an integral releasable catch (not shown). The chin strap end connector 2232 and the buckle type connector 2234 are secured together by the releasable catch and operated by an actuator, such as a release button 2236. When actuated by a user, the release button 2236 releases the buckle type connector 2234 from the chin strap end connector 2232. In some embodiments, the release button 2236 may serve as an emergency release to allow the patient 10 to be quickly disconnected from the head stabilization system 2000. For example, if the patient's airway becomes compromised, it may be desirable to quickly access the patient's mouth by detaching the chin strap portion 2202 (e.g., by actuating the release button 2236 as a chin strap emergency release). In some embodiments, the button release 2236 may be configured for one-hand operation. The left chin strap portion 2206 is constructed in a substantially similar manner providing for chin strap emergency release.

The illustrative head harness 2200 includes a halo portion 2210 configured to engage the patient's forehead 40 and the lateral side portions 44, 46 of the patient's head 14. The halo portion 2210 includes a right halo strap portion 2212 and a left halo strap portion 2214. The halo strap portions 2212, 2214 extend back-to-front along the patient's head 14. The halo strap portions 2212, 2214 may be adjustable, such as by adjustable buckle type connectors 2220 for adjustment of the halo strap portions 2212, 2214 to securely fit an individual patient's head 14. The halo strap portions 2212, 2214 may include one or more strap retainers 2266 to secure the loose ends of the halo strap portions 2212, 2214. At the front of the patient's head 14, the halo strap portions 2212, 2214 are coupled to a halo connector housing 2216 by the buckle type connectors 2220 with a releasable catch (not shown) operated by an actuator, such as a release button 2218. When actuated by a user, the release button 2218 releases the buckle type connectors 2220 from the halo connector housing 2216. In some embodiments, the release button 2218 may serve as an emergency release to allow the patient 10 to be quickly disconnected from the head stabilization system 2000. In some embodiments, the button release 2218 may be configured for one-hand operation. The halo connector housing 2216 may include one or more tabs 2272 for an alignment clip 2274. The alignment clip 2274 may include radiopaque markings 2276 which may be useful for imaging equipment calibration, for example.

In the illustrative head harness 2200, the halo portion 2210 is connected to the chin strap portion 2202 by a right halo stabilizer 2224 and a left halo stabilizer 2226. Each halo stabilizer 2224, 2226 is generally L-shaped, with the generally horizontal, upper portion affixed to the respective halo strap portion 2212, 2214 and the portion closer to the patient's forehead 40 connecting to the respective chin strap portion 2204, 2206. In this illustrative embodiment, the halo stabilizers 2224, 2226 are substantially rigid and each halo strap portion 2212, 2214 passes through and is secured to the respective stabilizer 2224, 2226, such as by one or more alignment clips 2228. The alignment clips 2228 may include radiopaque markings 2276 which may be useful for imaging equipment calibration, for example. The chin strap portions 2204, 2206 are pivotably attached to the halo stabilizers 2224, 2226, such as by ball joint connections 2260, which allow rotational adjustment to securely fit an individual patient's head 14. Each ball joint connection 2260 includes a generally spherical bearing stud 2262 attached to a chin strap end connector 2232 and a generally spherical socket 2264 which is integral to the respective halo stabilizer 2224, 2226. Each chin strap portion 2204, 2206 is connected to an adjustable buckle type connector 2234 which is connected to a chin strap end connector 2232 which is connected to the respective stabilizer 2224, 2226.

The illustrative head harness 2200 includes a rear support portion 2238, which may be in the form of a generally annular ring that is configured to be positioned centrally on the rear region 42 of the patient's head 14. The rear support portion 2238 is coupled to each of the halo strap portions 2212, 2214, so that the patient's head 14 is substantially circumscribed by the left halo strap portion 2214, the halo connector housing 2216, the right halo strap portion 2212, and the rear support portion 2238. The rear support portion 2238 and the halo connector housing 2216 are also connected by a head restraint portion 2240 that extends centrally over the top of the patient's head 14 from front to back.

In this illustrative embodiment, the head restraint portion 2240 includes a front head restraint strap portion 2241, a rear head restraint strap portion 2242, and a head restraint element 2244 disposed thereon. The head restraint portion 2240 may include radiopaque markings 2276 which may be useful for imaging equipment calibration, for example. The head restraint portion 2240 may be configured to allow adjustment to securely fit an individual patient's head 14. In this illustrative embodiment, the head restraint element 2244 includes a head restraint strap adjustment knob assembly 2246 configured to allow adjustment of the head restraint strap portions 2241, 2242 to securely fit the patient's head 14. The front head restraint strap portion 2241 contains a cavity 2280 such that a plurality of teeth (not shown) are aligned along one side of the cavity 2280. The rear head restraint strap portion 2242 contains a cavity 2284 such that a plurality of teeth (not shown) are aligned along one side of the cavity 2284 such that the teeth rear strap portion 2242 are aligned opposite the teeth of the front strap portion 2241 when the strap portions 2241, 2242 are in their installed positions. The adjustment knob assembly 2246 includes a radially oriented toothed wheel (not shown). The radially oriented toothed wheel of the adjustment knob assembly 2246 engages the teeth of the strap portions 2241, 2242 such that when the adjustment knob assembly 2246 is rotated the strap portions 2241, 2242 will move in relation to each other to lengthen or shorten the length of the head restraint portion 2240 to securely fit the patient's head 14.

The illustrative head harness 2200 includes a connecting element, such as a harness connector housing 2248, which is configured to releasably couple the head harness 2200 to the head restraint mechanism 2300. In this illustrative embodiment, the harness connector housing 2248 is provided on the head restraint element 2244 and includes a cavity 2250 and a slot 2252 configured to releasably engage corresponding components of the head restraint mechanism 2300, as described below. The slot 2252 extends through a wall 2256, which at least partially defines the cavity 2250. Additionally, the harness connector housing 2248 includes a latch 2257 releasable by an actuator, such as one or more finger tabs 2258, which are operatively coupled to selectively secure the head harness 2200 (e.g., the harness connector housing 2248) to the head restraint mechanism 2300. The finger tabs 2258 may serve as an emergency release, allowing rapid disconnection of the head harness 2200 from the head restraint mechanism 2300 when desired. In some embodiments, the finger tabs 2258 may be configured for one-hand operation.

In this illustrative embodiment, the chin strap portions 2204, 2206, the halo strap portions 2212, 2214, and the head restraint strap portions 2241, 2242 are constructed from flexible but substantially non-elastic materials. The halo stabilizers 2224, 2226 and the rear support portion 2238 are constructed from generally rigid materials, such as rigid polymeric or composite materials. In some exemplary embodiments, padding may be provided on various patient contact surfaces of the head harness 2200.

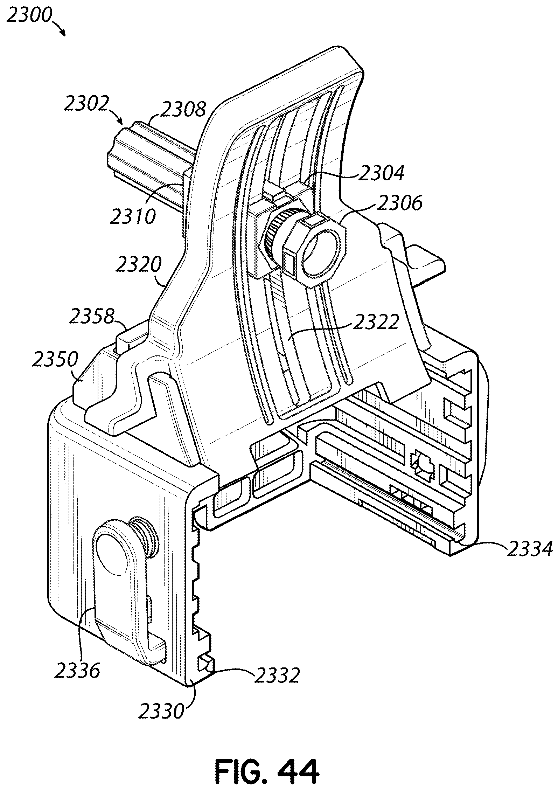

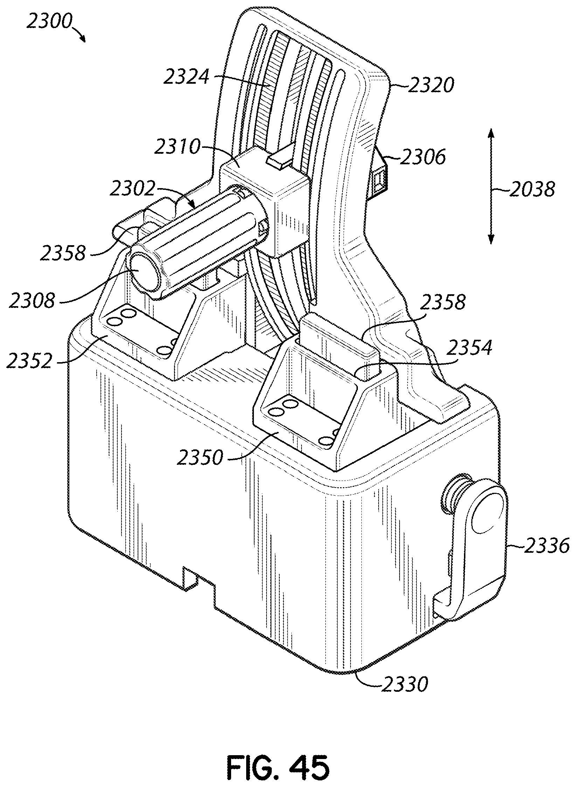

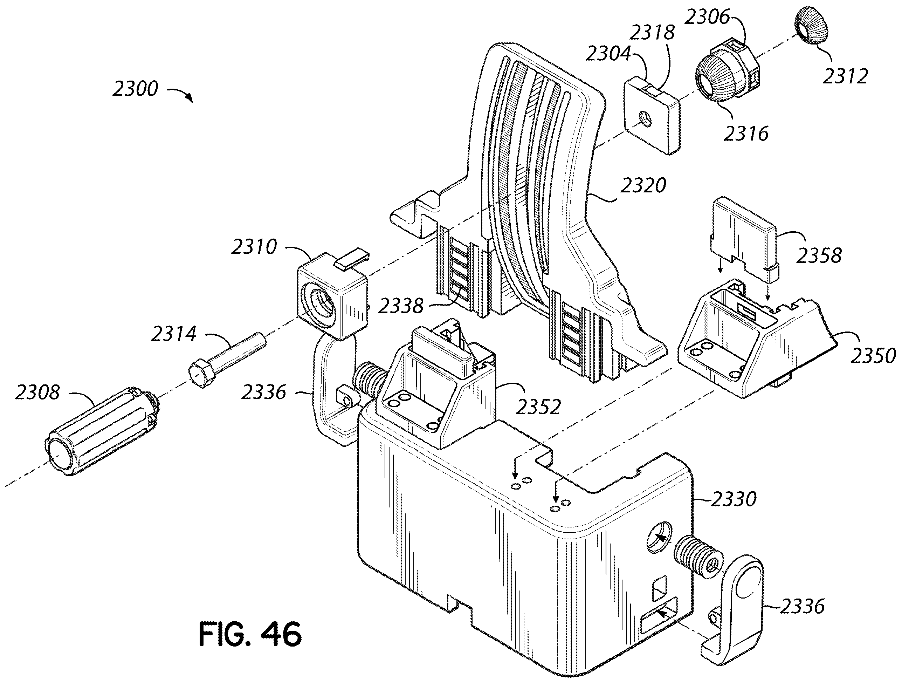

FIGS. 44-46 are isometric views of an illustrative head restraint mechanism 2300, according to at least some aspects of the present disclosure. Generally, the head restraint mechanism 2300 is similar in construction and operation to the head restraint mechanisms 300, 300a, 1300, and 1600 described above and the head restraint mechanism 2300 may be substituted for other head restraint mechanisms, or any feature of the head restraint mechanism 2300 may be used, in various other exemplary embodiments according to the present disclosure. Like reference numbers refer to like components.

Generally, the head restraint mechanism 2300 is configured to provide three-dimensional positioning of the patient's head. The head restraint mechanism 2300 includes a selectively securable harness connector assembly 2302. The illustrative harness connector assembly 2302 includes a harness connector stop 2306 a harness connector body 2304 a harness connector knob retainer 2310 a harness connector knob 2308 and a harness connector nut 2312 disposed on a harness connector shaft 2314. The harness connector knob 2308 is operative on the harness connector shaft 2314 to axially draw together the harness connector stop 2306 the harness connector body 2304 the harness connector knob retainer 2310 the harness connector knob 2308 and the connector nut 2312, such as by a threaded engagement between the harness connector nut 2312 and the harness connector shaft 2314. The harness connector stop 2306 is rotatable about the harness connector shaft 2314 to provide the rotational range of motion indicated by arrow 2034 and as described above with reference to FIG. 39.

In this illustrative embodiment, the harness connector stop 2306 includes a generally half spherical and axially oriented toothed surface 2316 facing the harness connector body 2304. The harness connector body 2304 includes a generally half spherical cavity and axially oriented toothed surface 2318 oriented to oppositely engage the toothed surface 2316 of the harness connector stop 2306. With the harness connector stop 2306 of the harness connector assembly 2302 within the cavity 2250 of harness connector housing 2248 of the head harness 2200, tightening the harness connector knob 2308 is operative to selectively secure the head harness 2200 with respect to the tilt guide 2320. More specifically, tightening the harness connector knob 2308 secures the harness connector stop 2306 with respect to the harness connector body 2304 and/or secures the relative angular positions of the toothed surfaces 2316, 2318. Loosening the harness connector knob 2308 is operative to allow rotation of the harness connector stop 2306 about the harness connector shaft 2314 and/or to allow relative angular movement between the tooth surfaces 2316, 2318.

Referring to FIGS. 44-46, the head restraint mechanism 2300 includes a generally vertically upstanding, arcuate tilt guide 2320 having a generally vertically oriented tilt guide slot 2322 therethrough. The harness connector shaft 2314 of the harness connector assembly 2302 extends through the tilt guide slot 2322, between the harness connector knob 2308 and the harness connector stop 2306. The connector knob retainer 2310 and the harness connector body 2304 are wider than the tilt guide slot 2322, so the harness connector assembly 2302 is retained on the tilt guide 2320. The harness connector assembly 2302 is slidable generally vertically upward and downward along the length of the tilt guide slot 2322 to provide the neck flexion and extension range of motion indicated by arrow 2036 and as described above with reference to FIGS. 40 and 41.

In this illustrative embodiment, the tilt guide 2320 includes one or more toothed surfaces 2324 axially oriented on one or both sides of the tilt guide slot 2322. In this illustrative embodiment, the connector knob retainer 2310 includes one or more toothed surfaces axially oriented to oppositely engage the toothed surfaces 2324 of the tilt guide 2320. With the harness connector knob retainer 2310 of the harness connector assembly 2302 positioned against the tilt guide 2320, tightening the harness connector knob 2308 is operative to selectively secure the harness connector assembly 2302 with respect to the tilt guide 2320. More specifically, tightening the harness connector knob 2308 secures the knob retainer 2310 with respect to the tilt guide 2320 and/or secures the relative angular positions of the toothed surfaces of the harness connector knob retainer 2310 and the tilt guide 2320. Loosening the harness connector knob 2308 is operative to allow movement of the harness connector assembly 2302 along the tilt guide slot 2322.

Referring to FIGS. 42-43 and 44-46, the harness connector assembly 2302 of the head restraint mechanism 2300 is configured to releasably engage the harness connector housing 2248 of the head harness 2200. The harness connector stop 2306 is sized and shaped to be received within the cavity 2250, with the slot 2252 receiving the harness connector shaft 2314. In this illustrative embodiment, the harness connector stop 2306 of the harness connector assembly 2302 has a plurality of equal length sides such that the harness connector stop 2306 may engage within the cavity 2250 of the harness connector housing 2248 in a plurality of positions. With the harness connector stop 2306 of the harness connector assembly 2302 within the cavity 2250 of harness connector housing 2248, tightening the harness connector knob 2308 is operative to selectively secure the head harness 2200 with respect to the tilt guide 2320. More specifically, tightening the harness connector knob 2308 secures the harness connector stop 2306 with respect to the tilt guide 2320. Loosening the harness connector knob 2308 is operative to allow movement of the harness connector assembly 2302 along the tilt guide slot 2322.

Referring to FIGS. 336, 37, and 44-46, the head restraint mechanism 2300 includes a head restraint housing 2330 and one or more tilt guide housings 2350, 2352. In this illustrative example, the tilt guide housings 2350, 2352 are coupled to the head restraint housing 2330. The tilt guide 2320 is operatively coupled to the tilt guide housings 2350, 2352. The tilt guide housings 2350, 2352 include push button slots 2354 for push buttons 2358 and push button springs 2360. The tilt guide 2320 includes a plurality of semi cylindrical cavities 2338 facing the push buttons 2358. The push buttons 2358 include a cavity facing the semi cylindrical cavities 2338 on the tilt guide 2320. The cavities 2338 on the tilt guide 2320 and push buttons 2358 are arranged to include a dowel pin 2364. The tilt guide 2320 is incrementally vertically adjustable relative to the head restraint housing 2330 and the tilt guide housings 2350, 2352 generally in the direction of indicated by arrow 2038 by selectively positioning the dowel pins 2364 in the cavities 2338. The head restraint mechanism housing 2330 includes one or more head restraint mechanism housing adjustment slots 2332, 2334 for securing the head restraint mechanism 2300 on the support plate assembly 2600. Respective head restraint housing push levers 2336 are operatively connected to the head restraint mechanism housing 2330 to selectively secure the head restraint mechanism housing 2330 in a desired longitudinal position relative to the support plate assembly 2600.

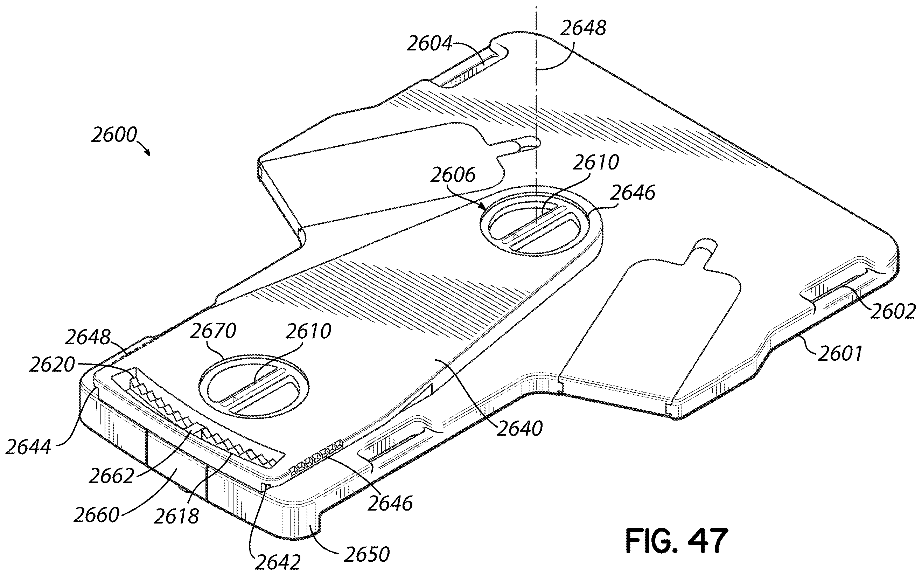

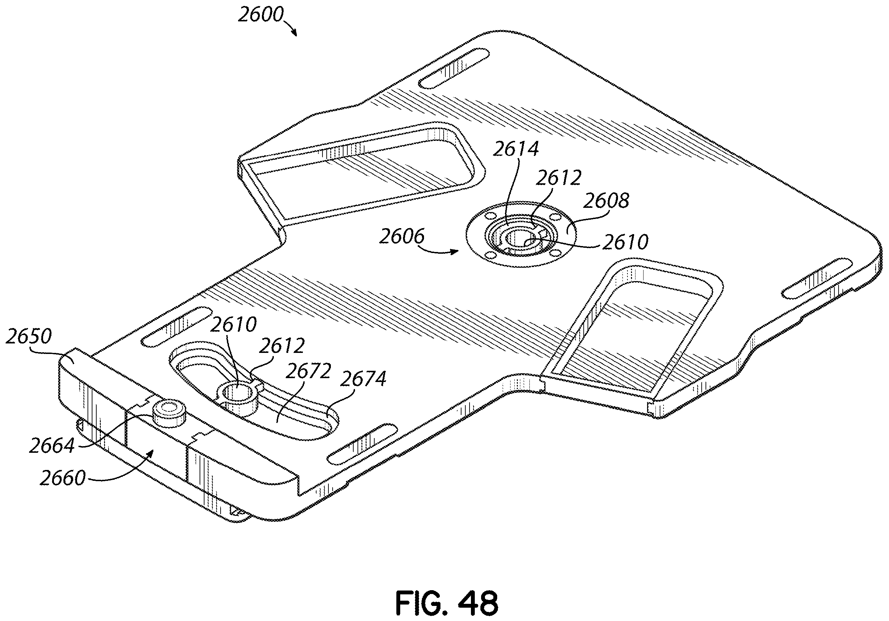

FIGS. 47 and 48 are isometric views of an illustrative patient support plate assembly 2600, according to at least some aspects of the present disclosure. Generally, the support plate assembly 2600 is similar in construction and operation to the support plate assemblies 600, 1410 described above and the patient support plate assembly 2600 may be substituted for other head restraint mechanisms, or any feature of the patient support plate assembly 2600 may be used, in various other exemplary embodiments according to the present disclosure. Like reference numbers refer to like components.

Referring to FIGS. 36, 37, 47, and 48, the patient support plate assembly 2600 is generally configured to secure the head stabilization system 2000 to the table 12 and to support the various other components of the head stabilization system 2000. The illustrative patient support plate assembly 2600 comprises a generally planar, generally rigid support plate 2601, to which other components of the support plate assembly 2600 and the head stabilization system 2000 are mounted. The support plate assembly 2600 includes one or more attachment elements, such as support plate fastening slots 2602, 2604, which are configured to secure the support plate assembly 2600 to the patient support device (e.g., the angiography table 12). For example, the support plate fastening slots 2602, 2604 may engage the support plate attachment elements (e.g., straps 26 and/or clamps 28) securing the patient support plate assembly 2600 to the table 12. Other attachment elements on the support plate assembly 2600 may be used in connection with other types of support plate attachment elements.

Referring to FIGS. 36, 37 and 44-48, the illustrative support plate assembly 2600 includes a support plate 2601 and a pivot plate 2640. The pivot plate 2640 operatively couples the tilt guide housing 2330 and the support plate 2601. The illustrative support plate 601 includes a pivot plate connector 2606, which is configured to releasably and pivotably couple the pivot plate 2640 to the support plate 2601. The pivot plate connector 2606 includes a connector base 2608 affixed to the support plate 2601 and a detachable connector cap 2610. The pivot plate 2640 includes a pivot plate bore 2646 which is disposed proximate one end of the pivot plate 2640. In this illustrative embodiment, the connector cap 2610 is configured to extend through the pivot plate bore 2646 of the pivot plate 2640 and to releasably couple with the connector base 2608. For example, the connector cap 2610 may include one or more radially extending tabs 2612, which may be configured to engaged corresponding slots 2614 in the connector base 2608. Rotation of the connector cap 2610 by about a quarter turn (e.g., clockwise) may be operative to secure the connector cap 2610 to the connector base 2608, thereby securing the pivot plate 2640 to the support plate 2601. Similarly, rotation of the connector cap 2610 by about a quarter turn in the opposite direction (e.g., counterclockwise) may be operative to release the connector cap 2610 from the connector base 2608, thereby releasing the pivot plate 2640. The pivot plate 2640 is pivotable about a pivot axis 2648 that is substantially perpendicular to the upper surface of the support plate assembly 2600 (and the upper surface of the table 12) to provide the range of motion of lateral flexion of the patient's neck 30 as shown by arrow 2032 on FIG. 38. One or more of the interfacing surfaces of the support plate assembly 2600 may include a bearing element, such as a lubricious material (e.g., a low-friction polytetrafluoroethylene or polyoxymethylene plastic) or a rolling element bearing, for ease of adjustment of the head restraint mechanism 2300, for example.

In this illustrative embodiment, the pivot plate 2640 includes a pivot lock slot 2618 shaped substantially as a radius with respect to the pivot plate bore 2646 and disposed near the opposite end of the pivot plate 2640 from the pivot plate bore 2646. The pivot lock slot 2618 includes a plurality of teeth 2620 axially oriented on one or both sides of the pivot lock slot 2618.

In this illustrative embodiment, the support plate 2601 includes a pivot lock assembly 2660 disposed on a flange 2650 that extends downwardly proximate the edge of the support plate 2601. The pivot lock assembly 2660 includes a lock element, such as a tooth or teeth 2662 which is operatively connected to a pivot lock button 2664. The tooth or teeth 2662 may selectively engage one or more of the teeth 2620 of the pivot plate 2640 thereby securing the angular position of the pivot plate 2640 relative to the support plate 2601. The depressing the pivot lock button 2664 disengages the tooth or teeth 2662 from the one or more teeth 2620 of the pivot plate 2640 allowing the pivot plate 2640 to rotate around the axis 2648. The tooth or teeth 2662 of the pivot lock assembly 2660 may be rotated approximately 90° allowing the tooth or teeth 2662 to pass through the pivot lock slot 2618 of pivot plate 2640, such as to allow the removal of the pivot plate 2640 from the support plate 2601.

In this illustrative embodiment, the pivot plate 2640 includes a pivot plate bore 2670 disposed proximate the pivot lock slot 2618 of pivot plate 2640. The support plate 2601 includes a pivot slot 2672 that includes a flange 2674 on one or both sides of the pivot slot 2672. The pivot slot 2672 is shaped substantially as a radius with respect to the pivot plate connector 2606. The illustrative support plate assembly 2600 includes a pivot plate connector cap 2610, which is configured to releasably and pivotably couple the pivot plate 2640 to the support plate 2601. In this illustrative embodiment, the connector cap 2610 is configured to extend through the pivot plate bore 2670 of the pivot plate 2640 and to releasably couple the pivot slot 2672 in the support plate 2601. For example, the connector cap 2610 may include one or more radially extending tabs 2612, which may be configured to engaged corresponding flange 2674 in the support plate 2601. Rotation of the connector cap 2610 by about a quarter turn (e.g., clockwise) may be operative to secure the connector cap 2610 to the support plate 2601, thereby securing the pivot plate 2640 to the support plate 2601. Similarly, rotation of the connector cap 2610 by about a quarter turn in the opposite direction (e.g., counterclockwise) may be operative to allow the connector cap 2610 from the support plate 2601, thereby releasing the pivot plate 2640. In the illustrative embodiment, the pivot plate 2640 is held generally against the upper surface of the support plate 2601 by the connector cap 2610, but the pivot plate 2640 remains pivotable about the pivot axis 2648 to provide lateral flexion of the patient's neck 30 as shown in FIG. 38.

Referring to FIGS. 44-48, the head restraint mechanism housing 2330 is releasably and selectively positionally coupled on the pivot plate 2640. For example, the head restraint mechanism housing 2330 is longitudinally slidable and selectively securable with respect to a long axis of the pivot plate 2640. In this illustrative example, the pivot plate 2640 includes channels 2642, 2644 for mounting the head restraint mechanism housing 2330. Each channel 2642, 2644 further comprises a series of indents 2646, 2648. The head restraint mechanism housing 2330 includes one or more head restraint mechanism housing adjustment slots 2332, 2334 which are disposed on the channels 2642, 2644 of the pivot plate 2640. The head restraint housing push levers 2336 engage with the indents 2646, 2648 to selectively secure the head restraint mechanism housing 2330 in a desired longitudinal position relative to the pivot plate 2640. Depressing the head restraint housing push levers 2336 disengages the head restraint housing push levers 2336 from the indents 2646, 2648 allowing a user to reposition the head restraint mechanism housing 2330 relative to the pivot plate 2640.

Referring to FIGS. 36-41 the torso pad 2550, which is configured to facilitate vertical positioning of the patient's upper torso 24, is disposed on the support plate assembly 2600. The torso pad 2550 may be utilized to lift the patient's upper torso 24. The illustrative torso pad 2550 comprises an inflatable membrane 2552 positioned between the support plate assembly 2600 and the patient's upper torso 24 (e.g., on top of the support plate assembly 2600 and beneath the patient's upper torso 24). Inflating the inflatable membrane 2552 generally raises the patient's upper torso 24 with respect to the support plate assembly 2600. Deflating the inflatable membrane 2552 generally lowers the patient's upper torso 24 towards the support plate assembly 2600. The inflatable membrane 2552 may be operatively coupled to a pressurized air source 2554, such as by appropriate tubing, valves, and connectors. In some exemplary embodiments, the pressurized air source 2554 may comprise an installed pressurized air supply system of a medical facility. In other exemplary embodiments, the pressurized air source 2554 may comprise a portable air source (e.g., portable compressor or portable compressed air tank).

The neck pad 2560, which is configured to facilitate vertical positioning of the patient's neck 30, is disposed on the support plate assembly 2600. The neck pad 2560 may be utilized to lift and/or support the patient's neck 30. The illustrative neck pad 2560 comprises an inflatable membrane 2562 positioned between the support plate assembly 2600 and the patient's neck 30 (e.g., on top of the support plate assembly 2600 and beneath the patient's neck 30). Inflating the inflatable membrane 2562 generally raises the patient's neck 30 with respect to the support plate assembly 2600. Deflating the inflatable membrane 2562 generally lowers the patient's neck 30 towards the support plate assembly 2600. The inflatable membrane 2562 may be operatively coupled to a pressurized air source 2554, such as by appropriate tubing, valves, and connectors.

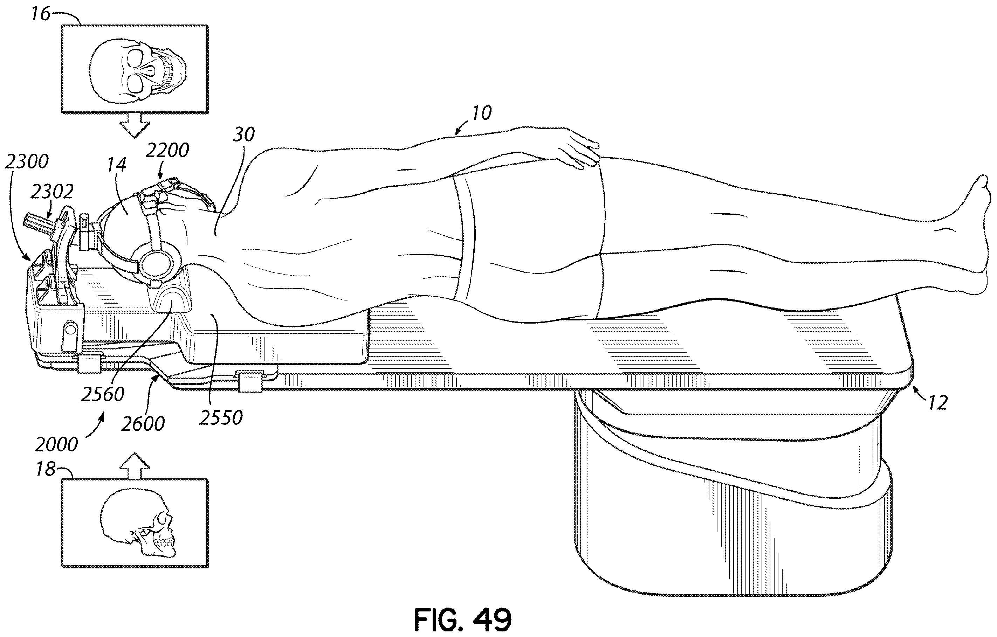

FIG. 49 is an isometric view of an alternate illustrative head stabilization system 2000 in use on a patient 10, wherein the patient 10 is positioned in a generally lateral position, according to at least some aspects of the present disclosure. While FIG. 49 illustrates patient 10 positioned on their left side, patient 10 may be positioned on either their left or right side.
reference to FIGS. 49-52, a summary overview of the potential positioning of the patient's head 14 facilitated by the head stabilization system 2000 is provided in order to give context for the descriptions below. Generally, the illustrative head stabilization system 2000 may be positioned and/or secured at any position and/or orientation within any combination of the ranges of motion described with reference to FIGS. 49-52 to position and/or stabilize the patient's head 14 as desired, such as for a procedure.

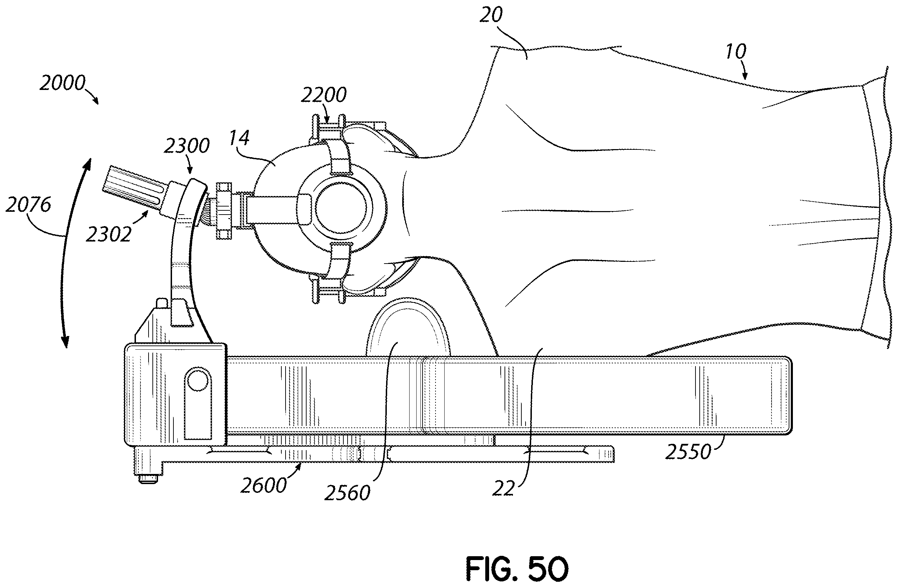

FIG. 50 is a lateral elevation view of the illustrative head stabilization system 2000 showing vertical positioning of the patient's head 14, according to at least some aspects of the present disclosure. Generally, this articulation of the head restraint mechanism 2000 provides vertical positioning of the patient's head 14, up or down, as shown by arrow 2076, in a plane generally perpendicular to the upper surface of the support plate assembly 2600. This vertical flexion motion is similar to that which would occur if the patient tilted his or her head to the right or left to touch the ear to the shoulder 20, 22 on the same side, without rotating the head 14. This illustrative head stabilization system 2000 facilitates about 30° of vertical flexion below center and 30° of vertical flexion above center, for a total range of lateral flexion of about 60°, from farthest down to farthest up.

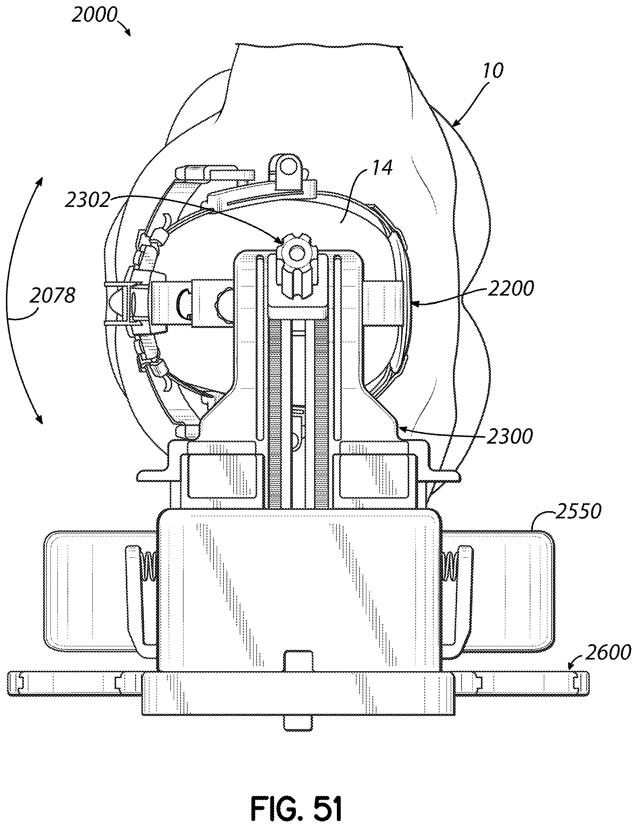

FIG. 51 is a cranial elevation view of the illustrative head stabilization system 2000 showing rotational positioning of the patent's head 14, according to at least some aspects of the present disclosure. Generally, this articulation of the head harness 2200 relative to the head restraint mechanism 2300 provides rotation of the patient's head 14, to the patient's right or to the patient's left, as shown by arrow 2078, in a plane generally perpendicular to the upper surface of the support plate assembly 2600. This motion is generally similar to that which would occur if the patient attempted to rotate his or her head 14 side to side as if to say "no". This illustrative head stabilization system 2000 facilitates about 60° of right rotation and about 60° of left rotation, for a total range of rotation of about 120°.

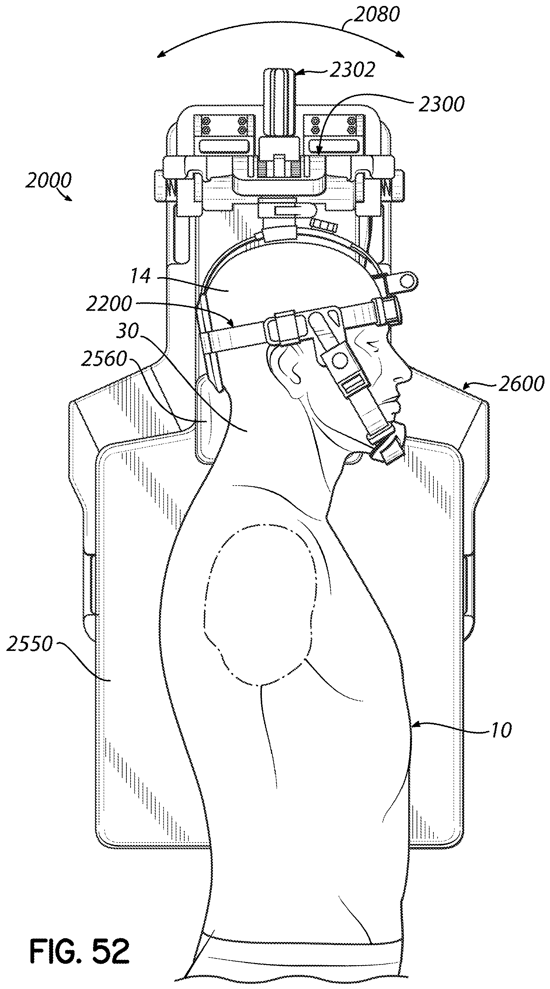

FIG. 52 is a plan view of the illustrative head stabilization system 2000 showing extension and flexion of the patient's neck 30, according to at least some aspects of the present disclosure. Generally, this articulation of the head restraint mechanism 2300 provides extension and flexion of the patient's neck 30, backward (i.e., extension) and forward (i.e., flexion), as shown by arrow 2080, in a plane generally transverse to the upper surface of the support plate assembly 2600. These motions are generally similar to those which would occur if the patient attempted to nod his or her head 14 up and down as if to say "yes". This illustrative head stabilization system 2000 facilitates about 25° of neck extension backward (from neutral) and about 25° of neck flexion forward (from neutral), for a total range of extension and flexion of about 50°. In some circumstances, it may be advantageous to apply traction to the patient's neck 30. For example, the head restraint mechanism 2300 (via the head harness 2200) may apply a force on the patient's head 14 in a generally superior/cranial direction. Accordingly, the patient's neck 30 may be placed in tension, generally longitudinally.

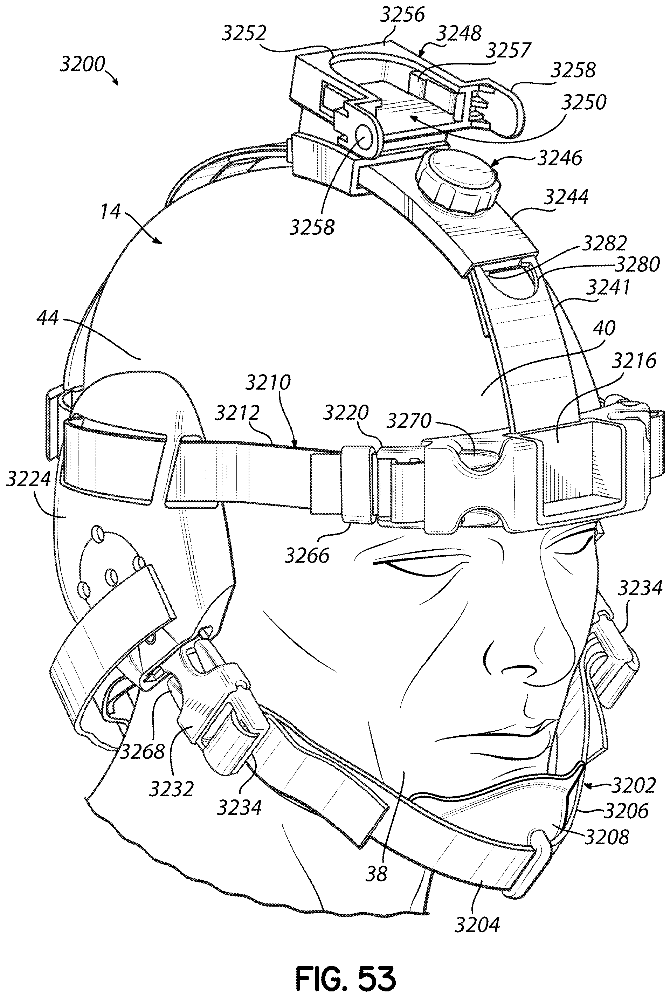

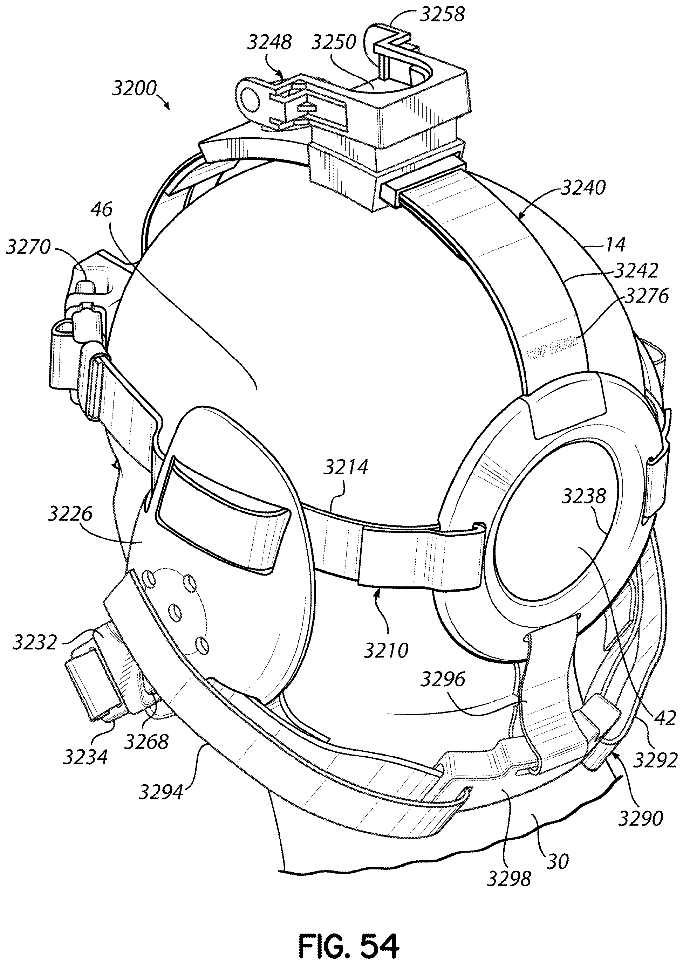

FIGS. 53 and 54 are isometric views of an illustrative head harness 3200 disposed on a patient's head 14, according to at least some aspects of the present disclosure. Generally, the head harnesses 3200 is similar in construction and operation to the head harnesses 200, 1200, 1500, and 2200 described above and the head harness 3200 may be substituted for other head harnesses, or any feature of the head harness 3200 may be used, in various other exemplary embodiments according to the present disclosure. Like reference numbers refer to like components.

The head harness 3200 is configured to be secured to the patient's head 14 and to be coupled (e.g., releasably) to a head restraint mechanism. Generally, the illustrative head harness 3200 is configured to engage the patient's head 14 at a chin/jaw region 38, a forehead region 40, and rear region 42. In some exemplary embodiments, the head harness 3200 may be configured to securely engage the patient's head in a non-traumatic, non-penetrating manner (e.g., no component of the head harness 3200 may penetrate the patient's skin).

The illustrative head harness 3200 includes a chin strap portion 3202 configured to engage the patient's chin/jaw region 38. The chin strap portion 3202 facilitates control of the position of the patient's chin relative to the patient's neck 30. The chin strap portion 3202 includes a right chin strap portion 3204 and a left chin strap portion 3206 that extend forward and downward generally along the patient's mandible to hold a chin pad 3208 against the patient's chin/jaw region 38. The chin strap portions 3204, 3206 may be adjustable, such as by buckle type connectors 3234.

The illustrative head harness 3200 includes one or more chin strap end connectors 3232. The right chin strap portion 3204 is connected to the chin strap end connector 3232 by a buckle type connector 3234 with an integral releasable catch 3268. The chin strap end connector 3232 and the buckle type connector 3234 are secured together by the releasable catch 3268. When actuated by a user, the releasable catch 3268 releases the buckle type connector 3234 from the chin strap end connector 3232. In some embodiments, the releasable catch 3268 may serve as an emergency release to allow the patient 10 to be quickly disconnected from the head harness 3200. For example, if the patient's airway becomes compromised, it may be desirable to quickly access the patient's mouth by detaching the chin strap portion 3202 (e.g., by actuating the releasable catch 3268 as a chin strap emergency release). In some embodiments, the releasable catch 3268 may be configured for one-hand operation. The left chin strap portion 3206 is constructed in a substantially similar manner providing for chin strap emergency release.

The illustrative head harness 3200 includes a halo portion 3210 configured to engage the patient's forehead 40 and the lateral side portions 44, 46 of the patient's head 14. The halo portion 3210 includes a right halo strap portion 3212 and a left halo strap portion 3214. The halo strap portions 3212, 3214 extend back-to-front along the patient's head 14. The halo strap portions 3212, 3214 may be adjustable, such as by adjustable buckle type connectors 3220 for adjustment of the halo strap portions 3212, 3214 to securely fit an individual patient's head 14. The halo strap portions 3212, 3214 may include one or more strap retainers 3266 to secure the loose ends of the halo strap portions 3212, 3214. At the front of the patient's head 14, the halo strap portions 3212, 3214 are coupled to a halo connector housing 3216 by the buckle type connectors 3220 with a releasable catch 3270. When actuated by a user, the releasable catch 3270 releases the buckle type connectors 3220 from the halo connector housing 3216. In some embodiments, the releasable catch 3270 may serve as an emergency release to allow the patient 10 to be quickly disconnected from the head harness 3200. In some embodiments, the releasable catch 3270 may be configured for one-hand operation.

In the illustrative head harness 3200, the halo portion 3210 is connected to the chin strap portion 3202 by a right ear cover 3224 and a left ear cover 3226. Each ear cover 3224, 3226 is generally shaped to cover the patient's ear, with the upper portion affixed to the respective halo strap portion 3212, 3214 and the lower portion connecting to the respective chin strap portion 3204, 3206. In this illustrative embodiment, the ear covers 3224, 3226 are substantially rigid and each halo strap portion 3212, 3214 passes through and is secured to the respective ear cover 3224, 3226. The chin strap portions 3204, 3206 are attached to the ear covers 3224, 3226. Each chin strap portion 3204, 3206 is connected to an adjustable buckle type connector 3234 which is connected to a chin strap end connector 3232 which is connected to the respective ear cover 3224, 3226.

The illustrative head harness 3200 includes a rear support portion 3238, which may be in the form of a generally annular ring that is configured to be positioned centrally on the rear region 42 of the patient's head 14. The rear support portion 3238 is coupled to each of the halo strap portions 3212, 3214, so that the patient's head 14 is substantially circumscribed by the left halo strap portion 3214, the halo connector housing 3216, the right halo strap portion 3212, and the rear support portion 3238. The rear support portion 3238 and the halo connector housing 3216 are also connected by a head restraint portion 3240 that extends centrally over the top of the patient's head 14 from front to back.

In this illustrative embodiment, the head restraint portion 3240 includes a front head restraint strap portion 3241, a rear head restraint strap portion 3242, and a head restraint element 3244 disposed thereon. The head restraint portion 3240 may include radiopaque markings 3276 which may be useful for imaging equipment calibration, for example. The head restraint portion 3240 may be configured to allow adjustment to securely fit an individual patient's head 14. In this illustrative embodiment, the head restraint element 3244 includes a head restraint strap adjustment knob assembly 3246 configured to allow adjustment of the head restraint strap portions 3241, 3242 to securely fit the patient's head 14. The front head restraint strap portion 3241 contains a cavity 3280 such that a plurality of teeth (not shown) are aligned along one side of the cavity 3280. The rear head restraint strap portion 3242 contains a cavity 3284 such that a plurality of teeth (not shown) are aligned along one side of the cavity 3284 such that the teeth rear strap portion 3242 are aligned opposite the teeth of the front strap portion 3241 when the strap portions 3241, 3242 are in their installed positions. The adjustment knob assembly 3246 includes a radially oriented toothed wheel (not shown). The radially oriented toothed wheel of the adjustment knob assembly 3246 engages the teeth of the strap portions 3241, 3242 such that when the adjustment knob assembly 3246 is rotated the strap portions 3241, 3242 will move in relation to each other to lengthen or shorten the length of the head restraint portion 3240 to securely fit the patient's head 14.

The illustrative head harness 3200 includes a connecting element, such as a harness connector housing 3248, which is configured to releasably couple the head harness 3200 to a head restraint mechanism. In this illustrative embodiment, the harness connector housing 3248 is provided on the head restraint element 3244 and includes a cavity 3250 and a slot 3252 configured to releasably engage corresponding components of a head restraint mechanism. The slot 3252 extends through a wall 3256, which at least partially defines the cavity 3250. Additionally, the harness connector housing 3248 includes a latch 3257 releasable by an actuator, such as one or more finger tabs 3258, which are operatively coupled to selectively secure the head harness 3200 (e.g., the harness connector housing 3248) to the head restraint mechanism 2300, for example. The finger tabs 3258 may serve as an emergency release, allowing rapid disconnection of the head harness 3200 from a head restraint mechanism when desired. In some embodiments, the finger tabs 3258 may be configured for one-hand operation.

The illustrative head harness 3200 includes a securing strap assembly 3290 located at the rear of the patient's head 14 and is intended to engage the base of the patient's skull to further secure the head harness 3200 to the patient's head 14. The securing strap assembly 3290 includes a right strap portion 3292 a left strap portion 3294 a vertical strap portion 3296 and a collector housing 3298. The collector housing 3298 is generally in the shape of an inverted T. The right strap portion 3292, left strap portion 3294, and vertical strap portion 3296 are connected to the collector housing 3298. The vertical strap portion 3296 is connected to the rear support portion 3238 generally at the bottom center portion of the rear support portion 3238. The right strap portion 3292 is connected to the right ear cover 3224 and the left strap portion 3294 is connected to left ear cover 3226. The strap portions 3292, 3294 are generally connected to the bottom portion of the respective ear covers 3224, 3226. The right and left strap portions 3292, 3294 may be adjustable to facilitate securing the head harness 3200 to the patient's head 14.

In this illustrative embodiment, the chin strap portions 3204, 3206, the halo strap portions 3212, 3214, and the head restraint strap portions 3241, 3242 are constructed from flexible but substantially non-elastic materials. The securing strap portions 3292, 3294, and 3296 are constructed from flexible elastic materials. The ear covers 3224, 3226, the rear support portion 3238, and the collector housing 3298 are constructed from generally rigid materials, such as rigid polymeric or composite materials. In some exemplary embodiments, padding may be provided on various patient contact surfaces of the head harness 3200.

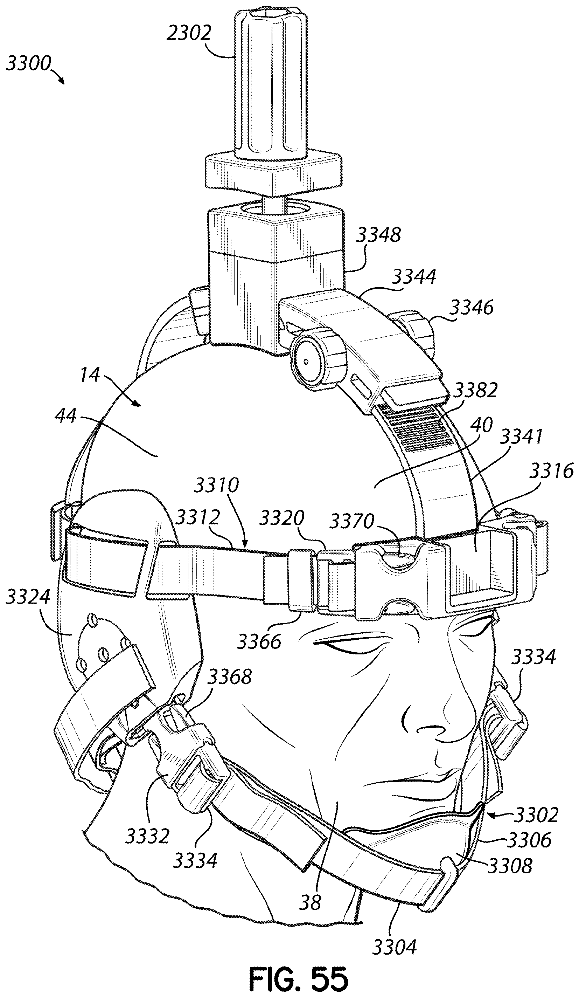

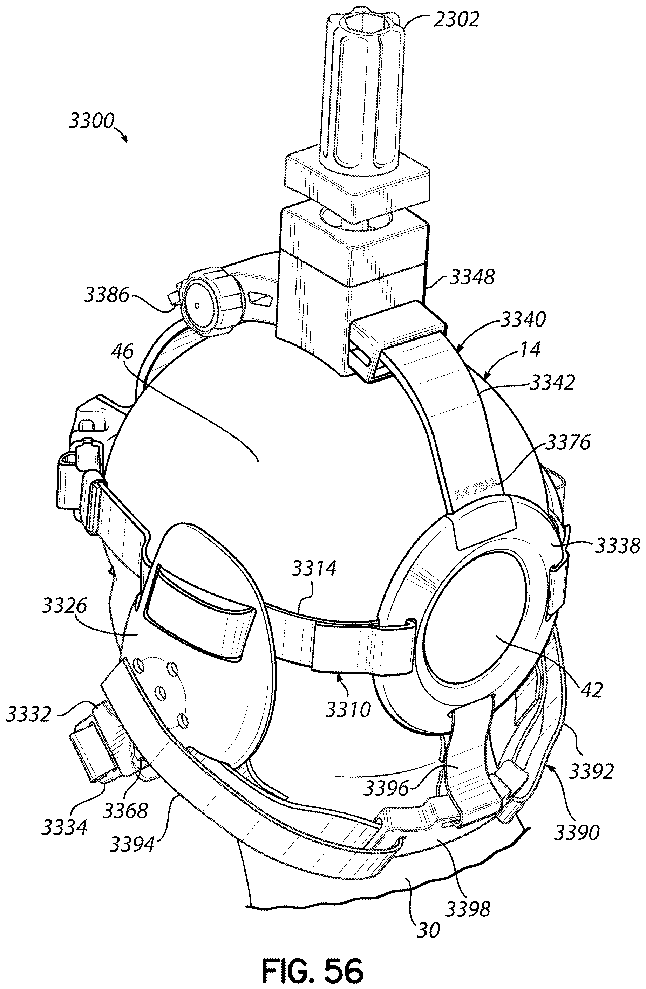

FIGS. 55 and 56 are isometric views of an illustrative head harness 3300 disposed on a patient's head 14, according to at least some aspects of the present disclosure. Generally, the head harness 3300 is similar in construction and operation to the head harnesses 200, 1200, 1500, 2200, and 3200 described above and the head harness 3300 may be substituted for other head harnesses, or any feature of the head harness 3300 may be used, in various other exemplary embodiments according to the present disclosure. Like reference numbers refer to like components.

The head harness 3300 is configured to be secured to the patient's head 14 and to be coupled (e.g., releasably) to a head restraint mechanism. Generally, the illustrative head harness 3300 is configured to engage the patient's head 14 at a chin/jaw region 38, a forehead region 40, and rear region 42. In some exemplary embodiments, the head harness 3300 may be configured to securely engage the patient's head in a non-traumatic, non-penetrating manner (e.g., no component of the head harness 3300 may penetrate the patient's skin).

The illustrative head harness 3300 includes a chin strap portion 3302 configured to engage the patient's chin/jaw region 38. The chin strap portion 3302 facilitates control of the position of the patient's chin relative to the patient's neck 30. The chin strap portion 3302 includes a right chin strap portion 3304 and a left chin strap portion 3306 that extend forward and downward generally along the patient's mandible to hold a chin pad 3308 against the patient's chin/jaw region 38. The chin strap portions 3304, 3306 may be adjustable, such as by buckle type connectors 3334.

The illustrative head harness 3300 includes one or more chin strap end connectors 3332. The right chin strap portion 3304 is connected to the chin strap end connector 3332 by a buckle type connector 3334 with an integral releasable catch 3368. The chin strap end connector 3332 and the buckle type connector 3334 are secured together by the releasable catch 3368. When actuated by a user, the releasable catch 3368 releases the buckle type connector 3334 from the chin strap end connector 3332. In some embodiments, the releasable catch 3368 may serve as an emergency release to allow the patient 10 to be quickly disconnected from the head harness 3300. For example, if the patient's airway becomes compromised, it may be desirable to quickly access the patient's mouth by detaching the chin strap portion 3302 (e.g., by actuating the releasable catch 3368 as a chin strap emergency release). In some embodiments, the releasable catch 3368 may be configured for one-hand operation. The left chin strap portion 3306 is constructed in a substantially similar manner providing for chin strap emergency release.

The illustrative head harness 3300 includes a halo portion 3310 configured to engage the patient's forehead 40 and the lateral side portions 44, 46 of the patient's head 14. The halo portion 3310 includes a right halo strap portion 3312 and a left halo strap portion 3314. The halo strap portions 3312, 3314 extend back-to-front along the patient's head 14. The halo strap portions 3312, 3314 may be adjustable, such as by adjustable buckle type connectors 3320 for adjustment of the halo strap portions 3312, 3314 to securely fit an individual patient's head 14. The halo strap portions 3312, 3314 may include one or more strap retainers 3366 to secure the loose ends of the halo strap portions 3312, 3314. At the front of the patient's head 14, the halo strap portions 3312, 3314 are coupled to a halo connector housing 3316 by the buckle type connectors 3320 with a releasable catch 3370. When actuated by a user, the releasable catch 3370 releases the buckle type connectors 3320 from the halo connector housing 3316. In some embodiments, the releasable catch 3370 may serve as an emergency release to allow the patient 10 to be quickly disconnected from the head harness 3300. In some embodiments, the releasable catch 3370 may be configured for one-hand operation.

In the illustrative head harness 3300, the halo portion 3310 is connected to the chin strap portion 3302 by a right ear cover 3324 and a left ear cover 3326. Each ear cover portion 3324, 3326 is generally shaped to cover the patient's ear, with the upper portion affixed to the respective halo strap portion 3312, 3314 and the lower portion connecting to the respective chin strap portion 3304, 3306. In this illustrative embodiment, the ear covers 3324, 3326 are substantially rigid and each halo strap portion 3312, 3314 passes through and is secured to the respective ear cover 3324, 3326. The chin strap portions 3304, 3306 are attached to the ear covers 3324, 3326. Each chin strap portion 3304, 3306 is connected to an adjustable buckle type connector 3334 which is connected to a chin strap end connector 3332 which is connected to the respective ear cover 3324, 3326.

The illustrative head harness 3300 includes a rear support portion 3338, which may be in the form of a generally annular ring that is configured to be positioned centrally on the rear region 42 of the patient's head 14. The rear support portion 3338 is coupled to each of the halo strap portions 3312, 3314, so that the patient's head 14 is substantially circumscribed by the left halo strap portion 3314, the halo connector housing 3316, the right halo strap portion 3312, and the rear support portion 3338. The rear support portion 3338 and the halo connector housing 3316 are also connected by a head restraint portion 3340 that extends centrally over the top of the patient's head 14 from front to back.

In this illustrative embodiment, the head restraint portion 3340 includes a front head restraint strap portion 3341, a rear head restraint strap portion 3342, and a head restraint element 3344 disposed thereon. The head restraint portion 3340 may include radiopaque markings 3376 which may be useful for imaging equipment calibration, for example. The head restraint portion 3340 may be configured to allow adjustment to securely fit an individual patient's head 14. In this illustrative embodiment, the head restraint element 3344 includes a head restraint strap adjustment assembly 3346 configured to allow adjustment of the head restraint strap portions 3341, 3342 to securely fit the patient's head 14. The front head restraint strap portion 3341 includes a plurality of teeth 3382 aligned on the top side of the strap portion 3341. The rear head restraint strap portion 3342 includes a plurality of teeth 3386 aligned on the bottom side of the strap portion 3342 such that the teeth 3386 are aligned opposite the teeth 3382 of the front strap portion 3341 when the strap portions 3341, 3342 are in their installed positions. The adjustment knob assembly 3346 engages the teeth 3382, 3386 of the strap portions 3341, 3342 such that when the adjustment knob assembly 3346 is rotated the strap portions 3341, 3342 will move in relation to each other to lengthen or shorten the length of the head restraint portion 3340 to securely fit the patient's head 14.

The illustrative head harness 3300 includes a connecting element, such as a harness connector housing 3348, which is configured to releasably couple the head harness 3300 to the head restraint mechanism 2300, for example.

The illustrative head harness 3300 includes a securing strap assembly 3390 located at the rear of the patient's head 14 and is intended to engage the base of the patient's skull to further secure the head harness 3300 to the patient's head 14. The securing strap assembly 3390 includes a right strap portion 3392 a left strap portion 3394 a vertical strap portion 3396 and a collector housing 3398. The collector housing 3398 is generally in the shape of an inverted T. The right strap portion 3392, left strap portion 3394, and vertical strap portion 3396 are connected to the collector housing 3398. The vertical strap portion 3396 is connected to the rear support portion 3338 generally at the bottom center portion of the rear support portion 3338. The right strap portion 3392 is connected to the right ear cover 3324 and the left strap portion 3394 is connected to left ear cover 3326. The strap portions 3392, 3394 are generally connected to the bottom portion of the respective ear cover portions 3324, 3326. The right and left strap portions 3392, 3394 may be adjustable to facilitate securing the head harness 3300 to the patient's head 14.

In this illustrative embodiment, the chin strap portions 3304, 3306, the halo strap portions 3312, 3314, and the head restraint strap portions 3341, 3342 are constructed from flexible but substantially non-elastic materials. The securing strap portions 3392, 3394, and 3396 are constructed from flexible elastic materials. The ear cover portions 3324, 3326, the rear support portion 3338, and the collector housing 3398 are constructed from generally rigid materials, such as rigid polymeric or composite materials. In some exemplary embodiments, padding may be provided on various patient contact surfaces of the head harness 3300.

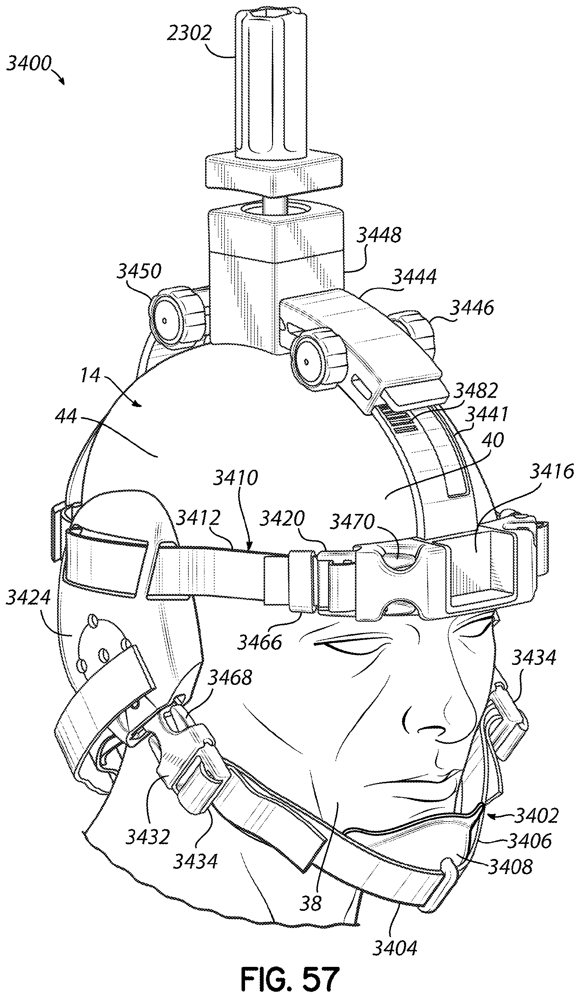

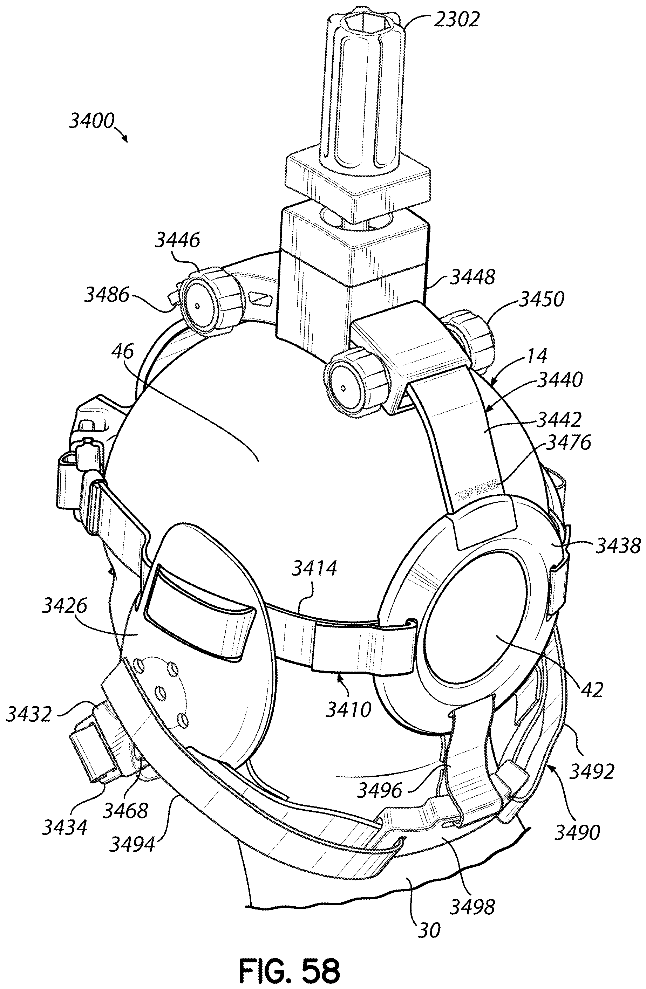

FIGS. 57 and 58 are isometric views of an illustrative head harness 3400 disposed on a patient's head 14, according to at least some aspects of the present disclosure. Generally, the head harness 3400 is similar in construction and operation to the head harnesses 200, 1200, 1500, 2200, 3200, and 3300 described above and the head harness 3400 may be substituted for other head harnesses, or any feature of the head harness 3400 may be used, in various other exemplary embodiments according to the present disclosure. Like reference numbers refer to like components.

The head harness 3400 is configured to be secured to the patient's head 14 and to be coupled (e.g., releasably) to a head restraint mechanism. Generally, the illustrative head harness 3400 is configured to engage the patient's head 14 at a chin/jaw region 38, a forehead region 40, and rear region 42. In some exemplary embodiments, the head harness 3400 may be configured to securely engage the patient's head in a non-traumatic, non-penetrating manner (e.g., no component of the head harness 3400 may penetrate the patient's skin).

The illustrative head harness 3400 includes a chin strap portion 3402 configured to engage the patient's chin/jaw region 38. The chin strap portion 3402 facilitates control of the position of the patient's chin relative to the patient's neck 30. The chin strap portion 3402 includes a right chin strap portion 3404 and a left chin strap portion 3406 that extend forward and downward generally along the patient's mandible to hold a chin pad 3408 against the patient's chin/jaw region 38. The chin strap portions 3404, 3406 may be adjustable, such as by buckle type connectors 3434.

The illustrative head harness 3400 includes one or more chin strap end connectors 3432. The right chin strap portion 3404 is connected to the chin strap end connector 3432 by a buckle type connector 3434 with an integral releasable catch 3468. The chin strap end connector 3432 and the buckle type connector 3434 are secured together by the releasable catch 3468. When actuated by a user, the releasable catch 3468 releases the buckle type connector 3434 from the chin strap end connector 3432. In some embodiments, the releasable catch 3468 may serve as an emergency release to allow the patient 10 to be quickly disconnected from the head harness 3400. For example, if the patient's airway becomes compromised, it may be desirable to quickly access the patient's mouth by detaching the chin strap portion 3402 (e.g., by actuating the releasable catch 3468 as a chin strap emergency release). In some embodiments, the releasable catch 3468 may be configured for one-hand operation. The left chin strap portion 3406 is constructed in a substantially similar manner providing for chin strap emergency release.

The illustrative head harness 3400 includes a halo portion 3410 configured to engage the patient's forehead 40 and the lateral side portions 44, 46 of the patient's head 14. The halo portion 3410 includes a right halo strap portion 3412 and a left halo strap portion 3414. The halo strap portions 3412, 3414 extend back-to-front along the patient's head 14. The halo strap portions 3412, 3414 may be adjustable, such as by adjustable buckle type connectors 3420 for adjustment of the halo strap portions 3412, 3414 to securely fit an individual patient's head 14. The halo strap portions 3412, 3414 may include one or more strap retainers 3466 to secure the loose ends of the halo strap portions 3412, 3414. At the front of the patient's head 14, the halo strap portions 3412, 3414 are coupled to a halo connector housing 3416 by the buckle type connectors 3420 with a releasable catch 3470. When actuated by a user, the releasable catch 3470 releases the buckle type connectors 4320 from the halo connector housing 3416. In some embodiments, the releasable catch 3470 may serve as an emergency release to allow the patient 10 to be quickly disconnected from the head harness 3400. In some embodiments, the releasable catch 3470 may be configured for one-hand operation.

In the illustrative head harness 3400, the halo portion 3410 is connected to the chin strap portion 3402 by a right ear cover 3424 and a left ear cover 3426. Each ear cover 3424, 3426 is generally shaped to cover the patient's ear, with the upper portion affixed to the respective halo strap portion 3412, 3414 and the lower portion connecting to the respective chin strap portion 3404, 3406. In this illustrative embodiment, the ear covers 3424, 3426 are substantially rigid and each halo strap portion 3412, 3414 passes through and is secured to the respective ear cover 3424, 3426. The chin strap portions 3404, 3406 are attached to the ear covers 3424, 3426. Each chin strap portion 3404, 3406 is connected to an adjustable buckle type connector 3434 which is connected to a chin strap end connector 3432 which is connected to the respective ear cover 3424, 3426.

The illustrative head harness 3400 includes a rear support portion 3438, which may be in the form of a generally annular ring that is configured to be positioned centrally on the rear region 42 of the patient's head 14. The rear support portion 3438 is coupled to each of the halo strap portions 3412, 3414, so that the patient's head 14 is substantially circumscribed by the left halo strap portion 3414, the halo connector housing 3416, the right halo strap portion 3412, and the rear support portion 3438. The rear support portion 3438 and the halo connector housing 3416 are also connected by a head restraint portion 3440 that extends centrally over the top of the patient's head 14 from front to back.

In this illustrative embodiment, the head restraint portion 3440 includes a front, a rear head restraint strap portion 3442, and a head restraint element 3444 disposed thereon. The head restraint portion 3440 may include radiopaque markings 3476 which may be useful for imaging equipment calibration, for example. The head restraint portion 3440 may be configured to allow adjustment to securely fit an individual patient's head 14. In this illustrative embodiment, the head restraint element 3444 includes a front head restraint strap adjustment assembly 3446 configured to allow adjustment of the front head restraint strap portion 3441. The head restraint element 3444 also includes a rear head restraint strap adjustment assembly 3450 configured to allow adjustment of the rear head restraint strap portion 3442. In this illustrative embodiment, the head restraint element 3444 may be adjusted to securely fit the patient's head 14 by using the head restraint strap adjustment knob assemblies 3446, 3450 to adjust head restraint strap portions 3441, 3442.

The illustrative head harness 3400 includes a connecting element, such as a harness connector housing 3448, which is configured to releasably couple the head harness 3400 to the head restraint mechanism 2300, for example.

The illustrative head harness 3400 includes a securing strap assembly 3490 located at the rear of the patient's head 14 and is intended to engage the base of the patient's skull to further secure the head harness 3400 to the patient's head 14. The securing strap assembly 3490 includes a right strap portion 3492 a left strap portion 3494 a vertical strap portion 3496 and a collector housing 3498. The collector housing 3498 is generally in the shape of an inverted T. The right strap portion 3492, left strap portion 3494, and vertical strap portion 3496 are connected to the collector housing 3498. The vertical strap portion 3496 is connected to the rear support portion 3438 generally at the bottom center portion of the rear support portion 3438. The right strap portion 3492 is connected to the right ear cover 3424 and the left strap portion 3494 is connected to left ear cover 3426. The strap portions 3492, 3494 are generally connected to the bottom portion of the respective ear covers 3424, 3426. The right and left strap portions 3492, 3494 may be adjustable to facilitate securing the head harness 3400 to the patient's head 14.

In this illustrative embodiment, the chin strap portions 3404, 3406, the halo strap portions 3412, 3414, and the head restraint strap portions 3441, 3442 are constructed from flexible but substantially non-elastic materials. The securing strap portions 3492, 3494, and 3496 are constructed from flexible elastic materials. The ear covers 3424, 3426, the rear support portion 3438, and the collector housing 3498 are constructed from generally rigid materials, such as rigid polymeric or composite materials. In some exemplary embodiments, padding may be provided on various patient contact surfaces of the head harness 3400.

The various features discussed herein may be used alone or in any combination within and between the various embodiments. Additional advantages and modifications will readily appear to those skilled in the art. The invention in its broader aspects is therefore not limited to the specific details, representative apparatus and methods and illustrative examples shown and described.

## Claims

1. A head stabilization system, comprising:
a head restraint mechanism (1300) configured to be operatively disposed on a patient support device, the head restraint mechanism includes a harness connector assembly (1302), the harness connector assembly (1302) includes a harness connector stop (1306) including a generally spherical ball disposed on a harness connector shaft (1308); and
a head hardness (1200) configured to engage a head of a patient, the head harness includes a head harness connecting element, the head harness connecting element includes a harness connector housing including a cavity (1250) and a slot (1252) including a generally circular opening (1255);
wherein the head restraint mechanism (1300) is configured to stabilize the head harness (1200) relative to the patient support device and is configured to selectively allow lateral flexion of a neck of the patient, lateral rotation of the head of the patient, and extension and flexion of the neck of the patient;
wherein the head harness (1200) is releasably attachable to and at least partially supported by the head restraint mechanism (1300) in that the harness connector stop (1306) is configured to be releasably received within the cavity (1250) of the harness connector housing (1248), the harness connector shaft (1308) is configured to be releasably received within the slot (1252) of the harness connector housing, and the generally spherical ball is configured to seat against the generally circular opening (1255) of the slot.

2. The system of claim 1, wherein the head harness (1200) and the head restraint mechanism (1300) are configured to stabilize the head of the patient with the patient in a generally supine position.

3. The system of claim 1, wherein the head restraint mechanism (1300) is configured to selectively provide about 30 degrees of lateral flexion of the neck of the patient, left and right, for a total of about 60 degrees of flexion of the neck of the patient.

4. The system of claim 1, wherein the head restraint mechanism (1300) is configured to selectively provide about 60 degrees of lateral rotation of the head of the patient, left and right, for a total of about 120 degrees of rotation of the head of the patient.

5. The system of claim 1, wherein the head restraint mechanism (1300) is configured to selectively provide about 25 degrees of neck extension and about 25 degrees of neck flexion, for a total of about 50 degrees of extension and flexion of the neck of the patient.

6. The system of claim 1, wherein the head harness (1200) is configured to engage a chin/jaw region, a forehead region, and a rear region of the head of the patient.

7. The system of claim 6, wherein the head harness (1200) comprises a halo portion (1210) configured to engage the forehead region, the halo portion (1210) including:
a right halo strap configured to extend front-to-back along the head of the patient;
a left halo strap configured to extend front-to back along the head of the patient; and
a halo adjustment mechanism coupled to the right halo strap and the left halo strap, the halo adjustment mechanism being configured to allow adjustment of the halo portion to fit the head of the patient.

8. The system of claim 7, wherein the head harness comprises a chin strap portion (1202) configured to engage the chin/jaw region.

9. The system of claim 8, wherein the head harness comprises
a right halo stabilizer (1224) connecting the chin strap portion (1202) to the right halo strap; and
a left halo stabilizer (1226) connecting the chin strap portion (1202) to the left halo strap.

10. The system of claim 9, wherein the right halo stabilizer (1224) comprises
a right halo stabilizer halo strap portion (1232) connected to the right halo strap; and
a right halo stabilizer chin strap portion (1234) connected to the chin strap;
wherein the right halo stabilizer halo strap portion (1232) is releasably secured to the right halo stabilizer chin strap portion.

11. The system of claim 9, wherein the left halo stabilizer (1226) comprises
a left halo stabilizer halo strap portion connected to the left halo strap; and
a left halo stabilizer chin strap portion connected to the chin strap;
wherein the left halo stabilizer halo strap portion is releasably secured to the left halo stabilizer chin strap portion.

12. The system of claim 7, wherein the head harness (1200) comprises a rear support portion (1238) configured to engage the rear region.

13. The system of claim 12, wherein the rear support portion (1238) comprises a generally annular ring configured to be positioned centrally on the rear region.

14. The system of claim 12, wherein the rear support portion(1238) is connected to the right halo strap and the left halo strap.

15. The system of claim 12, wherein the head harness (1200) comprises a head restraint portion (1240) connecting the rear support portion (1238) and the halo adjustment mechanism, the head restraint portion (1240) being configured to extend centrally front-to-back over the head of the patient.

## Patentansprüche

1. Kopfstabilisierungssystem, umfassend:
einen Kopfrückhaltemechanismus (1300), der so ausgebildet ist, dass sie betriebsmäßig an einer Patientenauflagevorrichtung angeordnet wird, wobei der Kopfrückhaltemechanismus eine Geschirrverbinderanordnung (1302) aufweist, wobei die Geschirrverbinderanordnung (1302) einen Geschirrverbinderanschlag (1306) einschließt, der eine an einem Geschirrverbinderschaft (1308) angeordnete, im Wesentlichen kugelförmige Kugel einschließt; und
ein Kopfgeschirr (1200), das dazu ausgebildet ist, mit einem Kopf eines Patienten in Eingriff zu treten, wobei das Kopfgeschirr ein Kopfgeschirrverbindungselement einschließt, wobei das Kopfgeschirrverbindungselement ein Geschirrverbindergehäuse einschließt, welches einen Hohlraum (1250) und einen Schlitz (1252) mit einer im Wesentlichen kreisförmigen Öffnung (1255) einschließt;
wobei der Kopfrückhaltemechanismus (1300) dazu ausgebildet ist, das Kopfgeschirr (1200) relativ zu der Patientenauflagevorrichtung zu stabilisieren, und dazu ausgebildet ist, wahlweise eine seitliche Beugung eines Halses des Patienten, eine seitliche Drehung des Kopfes des Patienten sowie eine Streckung und Beugung des Halses des Patienten zu ermöglichen;
wobei das Kopfgeschirr (1200) lösbar an dem Kopfrückhaltemechanismus (1300) befestigbar und zumindest teilweise von diesem getragen ist, indem
der Geschirrverbinderanschlag (1306) so ausgebildet ist, dass er lösbar in dem Hohlraum (1250) des Geschirrverbindergehäuses (1248) aufgenommen wird, der Geschirrverbinderschaft (1308) so ausgebildet ist, dass er lösbar in dem Schlitz (1252) des Geschirrverbindergehäuses aufgenommen wird, und die im Wesentlichen kugelförmige Kugel dazu ausgebildet ist, an der im Wesentlichen kreisförmigen Öffnung (1255) des Schlitzes anzuliegen.

2. System nach Anspruch 1, wobei das Kopfgeschirr (1200) und der Kopfrückhaltemechanismus (1300) dazu ausgebildet sind, den Kopf des Patienten zu stabilisieren, wenn sich der Patient im Wesentlichen in Rückenlage befindet.

3. System nach Anspruch 1, wobei der Kopfrückhaltemechanismus (1300) dazu ausgebildet ist, wahlweise etwa 30 Grad seitliche Beugung des Halses des Patienten nach links und nach rechts vorzusehen, sodass sich insgesamt etwa 60 Grad Beugung des Halses des Patienten ergeben.

4. System nach Anspruch 1, wobei der Kopfrückhaltemechanismus (1300) dazu ausgebildet ist, wahlweise etwa 60 Grad seitliche Drehung des Kopfes des Patienten nach links und nach rechts vorzusehen, sodass sich insgesamt etwa 120 Grad Drehung des Kopfes des Patienten ergeben.

5. System nach Anspruch 1, wobei der Kopfrückhaltemechanismus (1300) dazu ausgebildet ist, wahlweise etwa 25 Grad Halsstreckung und etwa 25 Grad Beugung des Halses vorzusehen, sodass sich insgesamt etwa 50 Grad Streckung und Beugung des Halses des Patienten ergeben.

6. System nach Anspruch 1, wobei das Kopfgeschirr (1200) dazu ausgebildet ist, mit einem Kinn-/Kieferbereich, einem Stirnbereich und einem hinteren Bereich des Kopfes des Patienten in Eingriff zu treten.

7. System nach Anspruch 6, wobei das Kopfgeschirr (1200) einen Halo-Abschnitt (1210) umfasst, der dazu ausgebildet ist, mit dem Stirnbereich in Eingriff zu treten, wobei der Halo-Abschnitt (1210) Folgendes einschließt:
einen rechten Halo-Gurt, der dazu ausgebildet ist, sich entlang des Kopfes des Patienten von vorne nach hinten zu erstrecken;
einen linken Halo-Gurt, der dazu ausgebildet ist, sich entlang des Kopfes des Patienten von vorne nach hinten zu erstrecken; und
einen Halo-Verstellmechanismus, der mit dem rechten Halo-Gurt und dem linken Halo-Gurt gekoppelt ist, wobei der Halo-Verstellmechanismus dazu ausgebildet ist, eine Verstellung des Halo-Abschnitts zur Anpassung an den Kopf des Patienten zu ermöglichen.

8. System nach Anspruch 7, wobei das Kopfgeschirr einen Kinngurtabschnitt (1202) umfasst, der dazu ausgebildet ist, mit dem Kinn-/Kieferbereich in Eingriff zu treten.

9. System nach Anspruch 8, wobei das Kopfgeschirr Folgendes umfasst
einen rechten Halo-Stabilisator (1224), der den Kinngurtabschnitt (1202) mit dem rechten Halo-Gurt verbindet; und
einen linken Halo-Stabilisator (1226), der den Kinngurtabschnitt (1202) mit dem linken Halo-Gurt verbindet.

10. System nach Anspruch 9, wobei der rechte Halo-Stabilisator (1224) Folgendes umfasst
einen Halo-Gurtabschnitt des rechten Halo-Stabilisators (1232), der mit dem rechten Halo-Gurt verbunden ist; und einen Kinngurtabschnitt des rechten Halo-Stabilisators (1234), der mit dem Kinngurt verbunden ist;
wobei der Halo-Gurtabschnitt des rechten Halo-Stabilisators (1232) lösbar an dem Kinngurtabschnitt des rechten Halo-Stabilisators gesichert ist.

11. System nach Anspruch 9, wobei der linke Halo-Stabilisator (1226) Folgendes umfasst
einen Halo-Gurtabschnitt des linken Halo-Stabilisators, der mit dem linken Halo-Gurt verbunden ist; und
einen Kinngurtabschnitt des linken Halo-Stabilisators, der mit dem Kinngurt verbunden ist;
wobei der Halo-Gurtabschnitt des linken Halo-Stabilisators lösbar an dem Kinngurtabschnitt des linken Halo-Stabilisators gesichert ist.

12. System nach Anspruch 7, wobei das Kopfgeschirr (1200) einen hinteren Stützabschnitt (1238) umfasst, der dazu ausgebildet ist, mit dem hinteren Bereich in Eingriff zu treten.

13. System nach Anspruch 12, wobei der hintere Stützabschnitt (1238) einen im Wesentlichen ringförmigen Ring umfasst, der dazu ausgebildet ist, mittig an dem hinteren Bereich positioniert zu werden.

14. System nach Anspruch 12, wobei der hintere Stützabschnitt (1238) mit dem rechten Halo-Gurt und dem linken Halo-Gurt verbunden ist.

15. System nach Anspruch 12, wobei das Kopfgeschirr (1200) einen Kopfrückhalteabschnitt (1240) umfasst, der den hinteren Stützabschnitt (1238) und den Halo-Verstellmechanismus verbindet, wobei der Kopfrückhalteabschnitt (1240) dazu ausgebildet ist, sich mittig von vorne nach hinten über den Kopf des Patienten zu erstrecken.

## Revendications

1. Système de stabilisation de tête, comprenant :
un mécanisme de retenue de tête (1300) conçu pour être disposé fonctionnellement sur un dispositif de support de patient, le mécanisme de retenue de tête inclut un ensemble raccord de harnais (1302), l'ensemble raccord de harnais (1302) inclut une butée de raccord de harnais (1306) incluant une bille généralement sphérique disposée sur un arbre de raccord de harnais (1308) ; et
un harnais de tête (1200) conçu pour venir en prise avec la tête d'un patient, le harnais de tête inclut un élément de raccord au harnais de tête, l'élément de raccord au harnais de tête inclut un boîtier de raccord de harnais incluant une cavité (1250) et une fente (1252) incluant une ouverture généralement circulaire (1255) ;
dans lequel le mécanisme de retenue de tête (1300) est conçu pour stabiliser le harnais de tête (1200) par rapport au dispositif de support de patient et est conçu pour permettre sélectivement la flexion latérale d'un cou du patient, la rotation latérale de la tête du patient, et l'extension et la flexion du cou du patient ;
dans lequel le harnais de tête (1200) peut être fixé de manière amovible et est au moins partiellement supporté par le mécanisme de retenue de tête (1300) en ce que
la butée de raccord de harnais (1306) est conçue pour être reçue de manière amovible dans la cavité (1250) du boîtier de raccord de harnais (1248), l'arbre de raccord de harnais (1308) est conçu pour être reçu de manière amovible dans la fente (1252) du boîtier de raccord de harnais, et la bille généralement sphérique est conçue pour être positionnée contre l'ouverture généralement circulaire (1255) de la fente.

2. Système selon la revendication 1, dans lequel le harnais de tête (1200) et le mécanisme de retenue de tête (1300) sont conçus pour stabiliser la tête du patient avec le patient en position généralement allongée sur le dos.

3. Système selon la revendication 1, dans lequel le mécanisme de retenue de tête (1300) est conçu pour fournir sélectivement environ 30 degrés de flexion latérale du cou du patient, à gauche et à droite, pour un total d'environ 60 degrés de flexion du cou du patient.

4. Système selon la revendication 1, dans lequel le mécanisme de retenue de tête (1300) est conçu pour fournir sélectivement environ 60 degrés de rotation latérale de la tête du patient, à gauche et à droite, pour un total d'environ 120 degrés de rotation de la tête du patient.

5. Système selon la revendication 1, dans lequel le mécanisme de retenue de tête (1300) est conçu pour fournir sélectivement environ 25 degrés d'extension du cou et environ 25 degrés de flexion du cou, pour un total d'environ 50 degrés d'extension et de flexion du cou du patient.

6. Système selon la revendication 1, dans lequel le harnais de tête (1200) est conçu pour venir en prise avec une région menton/mâchoire, une région front et une région arrière de la tête du patient.

7. Système selon la revendication 6, dans lequel le harnais de tête (1200) comprend une portion de halo (1210) conçue pour venir en prise avec la région frontale, la portion de halo (1210) incluant :
une sangle de halo droite conçue pour s'étendre d'avant en arrière le long de la tête du patient ;
une sangle de halo gauche conçue pour s'étendre d'avant en arrière le long de la tête du patient ; et
un mécanisme d'ajustement de halo couplé à la sangle de halo droite et à la sangle de halo gauche, le mécanisme d'ajustement de halo étant conçu pour permettre l'ajustement de la portion de halo pour s'adapter à la tête du patient.

8. Système selon la revendication 7, dans lequel le harnais de tête comprend une portion de sangle de menton (1202) conçue pour venir en prise avec la région menton/mâchoire.

9. Système selon la revendication 8, dans lequel le harnais de tête comprend un stabilisateur de halo droit (1224) raccordant la portion de sangle de menton (1202) à la sangle de halo droite ; et
un stabilisateur de halo gauche (1226) raccordant la portion de sangle de menton (1202) à la sangle de halo gauche.

10. Système selon la revendication 9, dans lequel le stabilisateur de halo droit (1224) comprend
une portion de sangle de halo de stabilisateur de halo droit (1232) raccordée à la sangle de halo droite ; et une portion de sangle de menton de stabilisateur de halo droit (1234) reliée à la sangle de menton ;
dans lequel la portion de sangle de halo de stabilisateur de halo droit (1232) est fixée de manière amovible à la portion de sangle de menton de stabilisateur de halo droit.

11. Système selon la revendication 9, dans lequel le stabilisateur de halo gauche (1226) comprend
une portion de sangle de stabilisateur de halo gauche raccordée à la sangle de halo gauche ; et
une portion de sangle de menton de stabilisateur de halo gauche raccordée à la sangle de menton ;
dans lequel la portion de sangle de halo de stabilisateur de halo gauche est fixée de manière amovible à la portion de sangle de menton de stabilisateur de halo gauche.

12. Système selon la revendication 7, dans lequel le harnais de tête (1200) comprend une portion de support arrière (1238) conçue pour venir en prise avec la région arrière.

13. Système selon la revendication 12, dans lequel la portion de support arrière (1238) comprend une bague généralement annulaire conçue pour être positionnée centralement sur la région arrière.

14. Système selon la revendication 12, dans lequel la portion de support arrière (1238) est raccordée à la sangle de halo droite et à la sangle de halo gauche.

15. Système selon la revendication 12, dans lequel le harnais de tête (1200) comprend une portion de retenue de tête (1240) raccordée à la portion de support arrière (1238) et au mécanisme de réglage de halo, la portion de retenue de tête (1240) étant conçue pour s'étendre centralement d'avant en arrière sur la tête du patient.
